# EUROPEAN PATENT APPLICATION

(11) **EP 3 656 784 A1**
(43) Date of publication of application: **27.05.2020**
(21) Application number: 18207314.8
(22) Date of filing: 20.11.2018
(51) Int. Cl.: C07K 14/415, C12N 9/12, C07K 19/00

(54) **CHIMERIC PATTERN RECOGNITION RECEPTOR KINASES**

(71) Applicant: Eberhard Karls Universität Tübingen, 72074 Tübingen (DE)
(72) Inventor: Chatelain, Philippe, 72108 Rottenburg (DE); Felix, Georg, 72076 Tübingen (DE); Weldle, Melissa, 78727 Oberndorf (DE)
(74) Representative: Kuttenkeuler, David

(57) **Abstract**

The present invention pertains to novel artificially designed plant receptors for mediating defense reactions. The present invention provides chimeric pattern recognition receptors (PRRs) which are composed of the ectodomain sequences of leucine rich repeat receptor like protein (LRR-RLP) which lacks a functional intracellular signalling domain, and a transmembrane and/or cytoplasmatic domain of a receptor like kinase (RLK) that functions as PRR. The chimeric receptors of the invention can be used to transform plant cells or plants in order to supplement and enhance the defense capabilities in strength and/or diversity in the transformed plant or plant cell. Hence, the present invention pertains to such chimeric PRR proteins, their encoding nucleic acids, methods for their construction, as well as their various uses in agriculture or other plant-based businesses.

## Description

### FIELD OF THE INVENTION

The present invention pertains to novel artificially designed plant receptors for mediating defense reactions. The present invention provides chimeric pattern recognition receptors (PRRs) which are composed of the ectodomain sequences of leucine rich repeat receptor like protein (LRR-RLP) which lacks a functional intracellular signalling domain, and a transmembrane and/or cytoplasmatic domain of a receptor like kinase (RLK) that functions as PRR. The chimeric receptors of the invention can be used to transform plant cells or plants in order to supplement and enhance the defense capabilities in strength and/or diversity in the transformed plant or plant cell. Hence, the present invention pertains to such chimeric PRR proteins, their encoding nucleic acids, methods for their construction, as well as their various uses in agriculture or other plant-based businesses.

### DESCRIPTION

Due to the constantly-increasing human population, and the declining area of land available for agriculture, it remains a major goal to improve the efficiency of agriculture and to increase the range of exploitable plants in agriculture. Conventional approaches for crop and horticultural improvements utilise classical plant breeding techniques in order to identify new plant varieties having desirable characteristics, like higher yield, resistance to pests and unfavourable weather conditions, root development, nutrient uptake and stress tolerance in general. However, such classical breeding techniques have several negative aspects, namely that these techniques are typically labour intensive and often include alteration of multiple traits. Most importantly, they suffer from the very close restrictions of interspecies crossing. This is a particular a restraint for traits like resistance genes and pattern recognition receptors that can confer resistance against herbivores and microbial pathogens. Such highly useful genes often occur in species that cannot be used in classical breeding.

Genetic engineering of plants entails the isolation and manipulation of the genetic and epige-netic material (typically in the form of DNA or RNA) and the subsequent introduction of that genetic material into a plant. Genetic engineering of plants at the industrial and laboratory scale is well established and has led to the development of plants having various improved economic, agronomic or horticultural traits. These technological advances have yielded crops that reduce food production costs through resistance to pests, herbicide, drought, and flood or generally enhance the growth and resistance of plants. Furthermore crops were engineered to produce substances such as vitamins that could improve human health. These approaches could help treat health issues in countries where people suffer from malnutrition such as vitamin A deficiency in countries where available foods do not provide the necessary nutrients for people.

Traits of particular economic interest are growth characteristics such as high yield of plant material such as fruits. Yield is normally defined as the measurable produce of economic value from a crop. This may be defined in terms of quantity and/or quality. Yield is directly dependent on several factors, for example, the number and size of the organs, plant architecture (for example, the number of branches), seed production and more. Sustainability of yield, an eminent issue, is threatened by a multitude of herbivores and microbial pathogens. Thus, traits of resistance, notably those that act as specific immunodetectors for inducing active defense responses in host plants, are highly valuable.

Cellular signalling upon environmental or developmental signals in plants and animals alike are coordinated by membrane associated proteins - receptors - which provide an extracellular binding domain to which a signal-molecule can attach, for example pathogenic structures of pests or growth factors, and intracellular signalling domains, specifically kinase domains, which can transmit the extracellular signal intra-cellular by initiating a signalling cascade. In the plant kingdom many of such membrane-bound receptor kinases were identified in the past decade, e.g. in the genomes of rice or *Arabidopsis* are about 600 to 1000 genes that encode for these proteins.

In plants leucine-rich-repeat receptor-like proteins and receptor like kinases (LRR-RLP, LRR-RLK) are key in the recognition and transmittal of exogenic as well as endogenic signals. LRR-RLKs (or simply "RLKs") are involved in developmental processes, growth, cell death, abscission fruit ripening and immune reactions upon pathogenic attacks. Individual RLPs or RLKs are involved in one or more biological processes; they bind through their extracellular portion specific signal-molecules, such as molecules that are characteristic in the event of an infection or damage caused by microbial or herbivorous attacks.

LRR-RLKs have a multi-domain architecture in which each domain provides a specific function for the receptor. The extracellular part of the receptor contains a leucine rich repeat (LRR) domain. The domain is named according to a series of repeating sequence motifs comprising about 20 to 30 amino acids with a high content of leucine. Leucine-rich repeats are frequently involved in the formation of protein-protein interactions, and not only found in plant receptors but also in a large variety of functionally unrelated proteins. Examples comprise the ribonuclease inhibitor, tropomodulin and toll-like receptors. Toll-like receptors are involved in innate immunity and developmental (Toll) processes. The LRR domain mediates the binding of the receptor to a ligand molecule, and hence can be classified as the input domain of the LRR-RLK.

On the intracellular side the LRR-RLK has a protein kinase domain, that enzymatically catalyses the transfer of a phosphate group to a substrate protein. Usually the addition of phosphate groups to intracellular substrates results in the initiation of a signal cascade that eventually induces into a cellular response, for example the induction of immune related genes in order to fight off a pathogenic attack. Thus, the kinase domain could be classified as the output domain of the LRR-RLK. Kinase and LRR domains are interconnected by the juxtamembrane and transmembrane domains, the latter spanning the cellular membrane, which allow the receptor to be membrane bound.

Although the function of many LRR-RLKs remains elusive, examples of LRR-RLKs that recognize exogenous signals are Flagellin Sensing 2 (FLS2) and Elongation Factor Tu Receptor (EFR) of *Arabidopsis.* The corresponding ligands of the receptors are flg22, a specific domain of bacterial flagellin and elfi8, the N-terminus of bacterial elongation factor Tu (EF-Tu), respectively. The EFR and FLS2 receptors function as immune receptors, also termed pattern recognition receptors (PRRs), recognising the presence of pathogens via microbe associated molecular patterns (MAMPs) and initiating a defense response in the plant cell. Other defense response related receptors include XA21 from rice, CERK1 and AtPEPRi, the latter recognizing endogenous danger signals related to wound healing processes.

A known LRR-RLK that binds endogenous signals of a plant is the brassinosteroid receptor BRI1 (brassinosteroid insensitive 1) that is an essential component of a membrane bound multi-receptor complex recognizing the steroid brassinolide. This steroid hormone in plants is important for physiological and developmental regulation. He Z and colleagues showed in a study with chimeric receptors composed of extracellular BRI1 domains and intracellular kinase domain of XA21, a rice defense response receptor, that artificial produced receptor chimeras could initiate a defense response in rice upon treatment with brassinosteroids. This study therefore shows that LRR-RLKs constitute an interesting starting point for the rational design of new receptor proteins in plants.

More novel chimeric receptors were constructed in *Arabidopsis thaliana* consisting of the complete ectodomain of EFR and the intracellular FLS2 kinase. These chimeras were shown to be sensitive towards the elf18 peptide, the natural ligand of EFR (Albert et al 2010; J Biol Chem; Vol 285, pp 19035-19042). The study shows that both LRR-RLKs EFR and FLS2 although they recognize different ligand molecules, still share all functional aspects for initiating the intracellular signal output of LRR-RLKs. A similar chimeric receptor approach was used in the elucidation of the function of wall associated kinases (WAK), which are involved in the detection of oligogalacturonides, molecular signals of cell wall damages, growth and development. Swapping of the ecto- or endo-domains of WAK and EFR resulted in a substitution of receptor function. EFR ectodomain WAKi kinase domain receptors sense elf18 but display a signal output typical for native WAK1 and *vice versa* (Brutus A et al., 2010; Proc Natl Acad Sci USA, Vol 107, pp 9452-9457).

Chimeric receptors based on the LRR-RLKs domain structure, in which the ectodomain and the intracellular kinase domain are derived from different receptors can be used for the controlled activation of cellular responses like immune response reactions in transgenic plants. A chimeric receptor that exploits the peculiarities of two receptors associated with plant defense, like FLS2, EFR or WAKi receptors, was designed with the intend to increase plant resistance to phytopathogenic organisms (WO 2010/139790).

Other that LRR-RLKs, also LRR receptor-like proteins (LRR-RLPs) which are characterized by a lack of any intracellular signalling domain (such as a kinase domain) are known to mediate various defense responses (Molecular Plant Pathology 6(1):85-97 January 2005). Their mechanism of signalling depends on a SOBIR1-type of adaptor kinase to form bimolecular receptor kinases (Gust and Felix, 2014). All families of higher plants have divergent, multi-membered arrays of LRR-RLPs and at least one gene of a SOBIR1-type adaptor kinase. However, interspecies and interfamily transfer of a LRR-RLP alone often meets compatibility problem resulting in malfunction of the transgenic LRR-RLP. Thus, also LRR-RLP with novel and agronomically valuable recognition specificities are not yet sufficiently harnessed for artificially increasing defense responses in recipient crop plants.

In view of the above, it is an object of the present invention to provide novel approaches for the rational design of artificial chimeric plant receptors usable to support plant defense mechanisms.

In a first aspect of the present invention, the above object is solved by a chimeric pattern recognition receptor (PRR) for mediating defense reactions, comprising at least an ectodomain, a transmembrane domain, and a cytoplasmatic domain, characterized in that the ectodomain is derived from Leucine Rich Repeat (LRR) Receptor Like Protein (RLP), and at least the transmembrane domain and/or the cytoplasmatic domain is derived from a membrane bound kinase.

As used herein, the term "chimeric" in context of a protein or gene sequence refers to two or more coding sequences obtained from different genes, that have been cloned together and that, after translation, act as a single polypeptide sequence. Chimeric proteins are also referred to as "hybrid proteins." As used herein, the term "chimeric protein" refers to coding sequences that are obtained from different species of organisms, as well as coding sequences that are obtained from the same species of organisms. A chimeric PRR in context of the invention is therefore a chimeric protein as defined before, but having/maintaining a biological function of a PRR. The herein disclosed chimeric PRRs consist of a sequence which purely artificial and therefore does not occur in nature.

In the context of the present invention the term "ectodomain" shall refer to the extracellular parts of a transmembrane receptor which harbour the receptor's function to bind to its ligands. Specifically, in certain embodiments of the present invention, the term "ectodomain" shall preferably refer to the LRR domain of a plant PRR.

In the context of the present invention and in all of its embodiments the term "pathogen-associated molecular patterns" abbreviated PAMP shall refer to any molecular structure derived from a plant pathogenic organism and/or any other microorganism, such as bacteria, fungi or unicellular eukaryotic organisms and/or small multi-cellular organisms.

The term "damage-associated molecular pattern", abbreviated DAMP, refers to a biomarker metabolite pattern that is characteristic of cell death (e.g., for example, apoptosis). For example, such DAMP biomarkers reflect the presence of oxidation and release biomarker including, but not limited to or intracellular nucleic acid release. Such cell death in a plant might occur due to injury for example in context of herbivorous attacks.

In the context of the present invention the term "transmembrane domain" shall refer to parts of a transmembrane receptor which form a stable fold in a cellular membrane. The term shall refer in case of single pass transmembrane receptors only to the domain which spans the cellular membrane. In case for multi pass transmembrane domains the term also denotes the region containing multiple single transmembrane domains.

In the context of the present invention the term "juxtamembrane domain" shall refer to the stretch of amino acids in a transmembrane receptor which is located on the extracellular side of the receptor, directly adjacent to the transmembrane domain. The juxtamembrane domain therefore connects the ectodomain with the transmembrane domain.

The term "derived from" as used herein in context of sequences or domains or parts of genes and proteins, shall denote that the referred protein or gene, domain, part of a protein or gene, shall be understood to refer to a use of a sequence of said protein or gene, domain, or part of a protein or gene, as a source for the chimeric PRR. As an example the expression "domain A derived from protein Z" is to be understood that the nucleic acid sequence encoding or the amino acid sequence of protein Z is used as a source for the sequence of domain A, and that the sequence of domain A is at least 50%, 60%, 70%, 80%, 85%, 90%, 95%, 98%, 99%, 100% identical to the sequence of the domain A in context of protein Z. Also "derived from" shall refer to a situation where the said protein or gene, domain, or part of a protein or gene is removed from its context, or isolated from, the entity it is derived from.

As used herein, the terms "identical" or percent "identity", when used anywhere herein in the context of two or more nucleic acid or protein/polypeptide sequences, refer to two or more sequences or sub-sequences that are the same or have (or have at least) a specified percentage of amino acid residues or nucleotides that are the same (i.e., at, or at least, about 60% identity, preferably at, or at least, 65%, 70%, 75%, 80%, 85%, 90%, 91%, 92%, 93% or 94%, identity, and more preferably at, or at least, about 95%, 96%, 97%, 98%, 99%, or higher identity over a specified region - preferably over their full length sequences - , when compared and aligned for maximum correspondence over the comparison window or designated region) as measured using a sequence comparison algorithms, or by manual alignment and visual inspection (see, e.g., NCBI web site). In a particular embodiment, the percentage identity can be determined by the Blast searches for amino acid identity, those using BLASTP 2.2.28+ with the following parameters: Matrix: BLOSUM62; Gap Penal-ties: Existence: 11, Extension: 1; Neighboring words threshold: 11; Window for multiple hits: 40.

The term "protein kinase" and "kinase" shall be understood to include enzymes that can catalyze phosphorylation (addition of phosphate/phosphoryl group) of phosphorylatable amino acids in proteins and/or peptides. These enzymes comprise, but are not limited to, protein tyrosine kinases, serine kinases and threonine kinases. A "membrane bound kinase" is a kinase protein that is located to a cellular membrane, for example the plasma membrane, but also any intracellular membranes such as organelle-membranes, endosomes, the endoplasmatic reticulum, Golgi apparatus etc. Such kinases often comprise a transmembrane domain or other membrane anchor.

Preferably the membrane bound kinase of the invention is selected from the group of leucine rich repeat (LRR) receptor-like kinases (RLK), such as a kinase selected from any of the following protein families: C-Lectin, CR4-like, CRPK1-like1, CRPKi-like2, DUF26, Extensin-like, L-Lectin, LRK10-like1, LRK10-like2, GDPDb, LRR I, LRR II, LRR III, LRR IV, LRR V, LRR VI, LRR VII, LRR VIII-1, LRR VIII-2, LRR IX, LRR X, LRR XI, LRR XII, LRR XIII, LRR XIV, LysM, PERK, RKF3-like, RLCK I, RLCK II, RLCK III, RLCK IV, TAK(RLCK V), RLCK VI, RLCK VII, RLCK VIII, RLCK IX, RLCK X, RLCK XI, RLCK XII, S-domain1, S-domain2, S-domain3, Thaumatin, URK I, or WAK-like (Plant Physiol. 2003 Jun;132(2):530-43.).

Even more preferably, the LRR-RLK is selected from any one of the following RLKs: BAK1, SERK4, SISERK1, BIR1, BIR2, SOBIR1, FER, CRK28, IOS1, PSKR1, PSY1R, ERECTA, SRF3, XA26, dsi, Bti9, SILyk13, THE1, Pi-d2, LecRK-I.9, LecRK-V.5, LecRK-VI.2, NgRLK1, LecRK1, NbLRK1, WAKL22, OsWAK1, TaRLK-R1,2,3, SNC4, LRK10, SNC2, and RLP52; and preferably wherein the LRR-RLK is SOBIR1 (The Plant Cell, Vol. 29: 618-637, April 2017).

Preferred SOBIR1 orthologs of *Arabidopsis thaliana* SOBIR1 are any one of the following (according to the July 2018 release of the Ensembl plant genome release: http://jul2018-plants.ensembl.org/index.html):

| **Species** | **Orthologue** |
|---|---|
| *Arabidopsis lyrata* | fgenesh2_kg.4_1173_AT2G31880.1 |
| *Brassica oleracea* | Bo4g177670 |
| *Brassica napus* | BnaCnng39500D |
| *Brassica napus* | BnaC03g17800D |
| *Brassica napus* | BnaA03g14760D |
| *Brassica rapa* | Bra022870 |
| *Brassica oleracea* | Bo3g026470 |
| *Brassica napus* | BnaA04g18600D |
| *Brassica rapa* | Bra021759 |
| *Brassica napus* | BnaA04g18590D |
| *Brassica napus* | BnaCnng39490D |
| *Brassica rapa* | Bra021758 |
| *Brassica oleracea* | Bo4g177660 |
| *Helianthus annuus* | HannXRQ_Chr16g0497571 |
| *Corchorus capsularis* | CCACVL1_08498 |
| *Theobroma cacao* | TCM_014517 |
| *Manihot esculenta* | MANES_14G057400 |
| *Populus trichocarpa* | POPTR_012G090500v3 |
| *Gossypium raimondii* | B456_003G108000 |
| *Vitis vinifera* | VIT_17s0000g09710 |
| *Vigna angularis* | LR48_Vigan04g070200 |
| *Glycine max* | GLYMA_04G190400 |
| *Manihot esculenta* | MANES_06G112200 |
| *Phaseolus vulgaris* | PHAVU_009G17200 |
| *Glycine max* | GLYMA_06G175100 |
| *Medicago truncatula* | MTR_3g075440 |
| *Vigna radiata* | gene7626 |
| *Helianthus annuus* | SOBIR1 (HannXRQ_Chr16g0497561) |
| *Prunus persica* | PRUPE_5G161700 |
| *Helianthus annuus* | HannXRQ_Chr09g0272651 |
| *Populus trichocarpa* | POPTR_015G086800v3 |

Therefore, the skilled person is aware that SOBIR1 orthologs usable in context of the present invention include proteins having an amino acid sequence with at least 60% identity to A. *thaliana* SOBIR1.

In other preferred embodiments the ectodomain of the chimeric PRR is not identical to, preferably at most 95%, 90%, 85%, or most preferably less than or equal to 80% identical to, an ectodomain of the LRR-Receptor-like Kinase (LRR-RLK).

Furthermore preferred is one embodiment where the ectodomain of the chimeric PRR is derived from an RLP which in its wild-type sequence does not contain a functional intracellular signalling domain, such as a functional intracellular kinase domain.

In context with the present invention, the term "wild type" in context of proteins, genes or plants, means that protein, gene or plant concerned has not been artificially genetically modified, and therefore consists of a sequence which occurs in nature and which was not subject to a targeted genetic alteration by humans. In particular such wild type proteins or genes do not consist of the sequence of a chimeric PRR of the invention.

In some embodiments it is preferred that the cytoplasmatic domain of the chimeric PRR comprises at least the functional kinase domain of said LRR-RLK. A kinase domain is functional in context of the present invention if it mediates a signal dependent or constitutive phosphorylation of a protein target by the chimeric PRR of the invention. In context of the present invention it is in particular preferred that the transmembrane domain and the cytoplasmatic domain of the chimeric PRR are derived from SOBIR1.

In some embodiments the chimeric PRR is preferred, wherein the sequences of the LRR-RLP and the LRR-RLK are derived (i) from the same species, or (ii) from different species.

In some embodiments the chimeric PRR of the invention further comprises an intracellular and/or extracellular juxtamembrane domain, wherein said intracellular and/or extracellular juxtamembrane domain is derived from said LRR-RLK. Most preferably the intracellular juxtamembrane domain is derived from said LRR-RLK.

In context of the invention it is in some embodiments preferred that the transmembrane domain of the chimeric PRR comprises at least one GxxxG (SEQ ID NO: 21) amino acid sequence motif, preferably more than one repeat of the GXXXG amino acid sequence motif, and preferably wherein the GxxxG amino acid sequence motif is located in the N-terminal half of the transmembrane domain. For example the transmembrane domain of the chimeric PRR is preferred comprising at least two, preferably three, more preferably four, consecutive GXXXG motifs, most preferably, the transmembrane domain comprises the conserved motif GXXXGXXXGXXXGXXXG (SEQ ID NO: 22), with not more than one variation of one G in said conserved motif.

As mentioned herein preferred embodiments include chimeric PRRs comprising SOBIR intracellular signalling domains, and therefore it is in some embodiments preferred that the transmembrane domain of the chimeric PRR is derived from SOBIR1, and is at least 50%, 60%, 70%, 80%, 85%, 90%, 95%, 98%, 99%, 100% identical to the transmembrane domain sequence of SOBIR1 of *Arabidopsis thaliana* (SEQ ID NO: 3), or homologs, paralogs or orthologs thereof.

Preferably the LRR-RLP from which the ectodomain of the chimeric PRR is derived is selected from a LRR-RLP involved in defense reactions such as, cellular damage, pathogen or parasite recognition, and is for example RLP1, RLP23, and any other RLPxx from *Arabidopsis thaliana,* or EIX2 (EIX2), or CURE1 from *S. lycopersicum.* Also any orthologs and homologs of these proteins shall be included in the present invention. However, only such LRR-RLP are referred to herein which do not contain a functional intracellular signalling domain. Preferably the ectodomain of the chimeric PRR of the invention is at least 50%, 60%, 70%, 80%, 85%, 90%, 95%, 98%, 99%, 100% identical to the ectodomain of the said LRR-RLP. Furthermore, it is preferable that the ectodomain of the chimeric PRR of the invention is capable of binding to a molecular pattern associated with and indicative for the presence of microbial pathogens, herbivores, parasitic plants or tissue injury, such as a pathogen- and/or damage-associated molecular patterns (PAMPs, DAMPs), for example xylanase from fungi, flagellin and "Emax" from bacteria; or patterns indicative for cell necrosis, or ethylene-inducing peptide 1-like proteins (NLPs) from bacteria, fungi or oomycetes.

A chimeric PRR according to the invention preferably consists of, or consists essentially of, a sequence that is not 90%, 95%, 98%, 99% most preferably not 100% identical to any naturally occurring (wild type) plant protein sequence (is artificial).

In further embodiments the cytoplasmatic domain of the chimeric PRR of the invention is derived from SOBIR1 of any plant species, or preferably of *Arabidopsis thaliana,* and comprises a SOBIR1 functional kinase domain having an amino acid sequence that is at least 50%, 60%, 70%, 80%, 85%, 90%, 95%, 98%, 99%, 100% identical to the kinase domain of SOBIR1 of *Arabidopsis thaliana* (SEQ ID NO: 3).

The chimeric PRR of the invention may be provided as a protein or a gene construct, therefore the term shall, if not otherwise indicated or derivable from the context, refer to both amino acid sequence of the chimeric PRR or a nucleic acid sequence encoding for the chimeric PRR protein.

Furthermore provided are in one additional aspect of the invention a nucleic acid comprising a nucleotide sequence encoding for a chimeric PRR protein according to the invention as described herein. Such a nucleic acid may be provided in context of a vector, such as an expression vector, comprising said nucleic acid. Such nucleic acids or vectors are also referred to as being "recombinant". A vector within the meaning of the present invention is a protein or a nucleic acid or a mixture thereof which is capable of being introduced or of introducing the polynucleotides comprised into a cell. It is preferred that the proteins encoded by the introduced nucleic acid are expressed within the cell upon introduction of the vector.

In a preferred embodiment, the vector of the present invention comprises recombinant vectors, plasmids, phagemids, phages, cosmids, viruses, in particular but not limited to virus-derived amplicon vectors, potato virus X based vectors, tobacco rattle virus based vectors, geminivirus-based vectors such as cabbage leaf curl virus and barley stripe mosaic virus based vectors and vectors based on satellite viruses (reviewed in Curtin, S.J., Wang, M.-B., Watson, J.M., Roffey, P., Blanchard, C.L. and Waterhouse, P.M.. (2007), chapter 12, p 291-332 in "Rice Functional Genomics; Challenges, Progress and Prospects". Upadhyaya, Narayana M. (Ed.), ISBN: 978-0-387-48903-2), virosomes, and nucleic acid coated particles, in particular gold spheres.

The term "recombinant nucleic acid molecule" refers to a polynucleotide produced by human intervention. A recombinant nucleic acid molecule can contain two or more nucleotide sequences that are linked in a manner such that the product is not found in a cell in nature. In particular, the two or more nucleotide sequences can be operatively linked and, for example, can encode a fusion polypeptide, or can comprise a nucleotide sequence and a regulatory element. A recombinant nucleic acid molecule also can be based on, but different, from a naturally occurring polynucleotide, for example, a polynucleotide having one or more nucleotide changes such that a first codon, which normally is found in the polynucleotide, is replaced with a degenerate codon that encodes the same or a conservative amino acid, or such that a sequence of interest is introduced into the polynucleotide, for example, a restriction endonuclease recognition site or a splice site, a promoter, a DNA replication initiation site, or the like.

Preferred is a recombinant vector according to the present invention, which is an expression vector, optionally comprising one or more genes, such as a gene of a chimeric PRR, to be expressed. Preferably, said expression is driven by a regulatory sequence (or sequences). A regulatory sequence can be isolated from a naturally occurring genomic DNA sequence or can be synthetic, for example, a synthetic promoter.

Regulatory sequences can be constitutively expressed regulatory sequences, which maintain gene expression at a relative level of activity (basal level), or can be regulated regulatory sequences. Constitutively expressed regulatory sequence can be expressed in any cell type, or can be tissue specific, which are expressed only in particular cell types, phase specific, which are expressed only during particular developmental or growth stages of a plant cell, or the like. A regulatory sequence such as a tissue specific or phase specific regulatory sequences or an inducible regulatory sequence useful in constructing a recombinant polynucleotide or in a practicing a method of the invention can be a regulatory sequence that generally, in nature, is found in a plant genome. However, the regulatory sequence also can be from an organism other than a plant, including, for example, from a plant virus, an animal virus, or a cell from an animal or other multicellular organism.

A preferred regulatory sequence useful for expression of polynucleotides of the invention is a promoter element. Useful promoters include, but are not limited to, constitutive, inducible, temporally regulated, developmentally regulated, spatially-regulated, chemically regulated, stress-responsive, tissue-specific, viral and synthetic promoters. Promoter sequences are known to be strong or weak. A strong promoter provides for a high level of gene expression, whereas a weak promoter provides for a very low level of gene expression. An inducible promoter is a promoter that provides for the turning on and off of gene expression in response to an exogenously added agent, or to an environmental or developmental stimulus. A bacterial promoter can be induced to varying levels of gene expression depending on the level of isothiopropyl galactoside added to the transformed bacterial cells. An isolated promoter sequence that is a strong promoter for heterologous nucleic acid is advantageous because it provides for a sufficient level of gene expression to allow for easy detection and selection of transformed cells and provides for a high level of gene expression when desired.

The choice of promoter will vary depending on the temporal and spatial requirements for expression, and also depending on the target species. In some cases, expression in multiple tissues is desirable. While in others, tissue-specific, e.g., leaf-specific, seed-specific, petal-specific, anther-specific, or pith-specific, expression is desirable. Although many promoters from dicotyledons have been shown to be operational in monocotyledons and vice versa, ideally dicotyledonous promoters are selected for expression in dicotyledons, and monocotyle-donous promoters for expression in monocotyledons. There is, however, no restriction to the origin or source of a selected promoter. It is sufficient that the promoters are operational in driving the expression of a desired nucleotide sequence in the particular cell.

Other sequences that have been found to enhance gene expression in transgenic plants include intron sequences (e. g., from Adh 1, bronze 1, actin 1, actin 2 (WO 00/760067), or the sucrose synthase intron), poly adenylation signals in the 3' prime UTR and viral leader sequences (e.g., from TMV, MCMV and AMV). For example, a number of non-translated leader sequences derived from viruses are known to enhance expression. Specifically, leader sequences from tobacco mosaic virus (TMV), maize chlorotic mottle virus (MCMV), and alfalfa mosaic virus (AMV) have been shown to be effective in enhancing expression (e.g., Gallie et al., 1987; Skuzeski et al., 1990). Other leaders known in the art include but are not limited topicornavirus leaders, for example, EMCV leader (encephalomyocarditis virus 5'-non-coding region; Elroy-Stein et al., 1989); potyvirus leaders, for example, TEV leader (tobacco etch virus); MDMV leader (maize dwarf mosaic virus); human immunoglobulin heavy chain binding protein (BiP) leader, (Macejak et al., 1991); untranslated leader from the coat protein mRNA of AMV (AMV RNA 4; Jobling et al., 1987), TMV (Gallie et al.,1989), and MCMV (Lommel et al., 1991; see also, della Cioppa et al., 1987).

For the expression of any constructs as described herein such as the chimeric PRR in a plant or plant cell, the invention preferably embodies that the described polynucleotides are operable linked to a promoter and to a polyadenylation site, wherein said promoter is characterized in that it is functional in said cell of said plant. As a promoter in this context, any sequence element is sufficient that induces transcription of the downstream sequence. The minimal requirements of promoters are very well known in the art and many of such promoters are conventionally used for gene expression in plants.

In a preferred embodiment of the invention, the transformation of a plant or plant cell with any polynucleotide encoding a chimeric PRR of the invention and as described herein, is performed by a method selected from standard procedures known in the art. Transformation of plant tissue can be achieved preferably by particle bombardment (Klein et al., "High- Velocity Microprojectiles for Delivering Nucleic Acids Into Living Cells," Nature 327:70-73 (1987)), also known as ballistic transformation of the host cell, as disclosed in U.S. Patent Nos. 4,945,050, 5,036,006, and 5,100,792, all to Sanford et al., and in Emerschad et al., "Somatic Embryogenesis and Plant Development from Immature Zygotic Embryos of Seedless Grapes (Vitis vinifera)" Plant Cell Reports 14:6-12 (1995). In particle bombardment, tungsten or gold microparticles are coated with the DNA of interest and then bombarded at the tissue using high pressure gas. In this way, it is possible to deliver foreign nucleotides into the nucleus. Biologically active particles (e.g., dried bacterial cells containing the vector and heterologous DNA) can also be propelled into plant cells. Other variations of particle bombardment, now known or hereafter developed, can also be used. Another preferred method of stably introducing the nucleic acid construct into plant cells is to infect a plant cell with *Agrobacterium tumefaciens* or *Agrobacterium rhizogenes* previously transformed with the polynucleotide construct. As described above, the Ti (or RI) plasmid of *Agrobacterium* enables the highly successful transfer of a foreign nucleic acid molecule into plant cells. A preferred variation of *Agrobacterium* transformation uses vacuum infiltration in which whole plants are used (Senior, "Uses of Plant Gene Silencing," Biotechnology and Genetic Engineering Reviews 15:79-119 (1998)). Yet another referred method of introduction is fusion of protoplasts with other entities, either mini-cells, cells, lysosomes, or other fusible lipid-surfaced bodies (Fraley et al., Proc. Natl. Acad. Sci. USA 79:1859-63 (1982),). Also preferred in a method, wherein the nucleic acid molecule is introduced into the plant cells by electroporation (Fromm et al., Proc. Natl. Acad. Sci. USA 82:5824 (1985)). In this technique, plant protoplasts are electroporated in the presence of plasmids containing the expression cassette. Electrical impulses of high field strength reversibly permeabilize biomembranes allowing the introduction of the plasmids. Electroporated plant protoplasts reform the cell wall, divide, and regenerate. Other preferred methods of transformation include chemical-mediated plant transformation, microinjection, physical abrasives, viral transduction and laser beams (Senior, "Uses of Plant Gene Silencing," Biotechnology and Genetic Engineering Reviews 15 :79- 119 (1998)). The precise method of transformation is not critical to the practice of the present invention. Any method that results in efficient transformation of the host cell of choice is appropriate for practicing the present invention.

Therefore, in another aspect of the invention a recombinant cell comprising a chimeric PRR of the invention, a nucleic acid, or a vector is provided. Such a recombinant cell is a bacterial cell or is a plant cell.

Additionally provided in context of the invention are recombinant multicellular organisms, or a part, organ, fruit or seed thereof, comprising the recombinant cell of the invention; preferably wherein the organism is a plant. Such an organism in some embodiments and aspects is not obtained by an essentially biological process for the production of plants. More preferably the organisms according to the invention are not exclusively obtained or obtainable by an essentially biological process. This may apply for any herein described embodiments or aspects relating to plants or animals.

Another aspect also pertains to a product for use in the treatment and/or protection of a plant against injury, a pathogenic infection, such as a bacterial infection, a viral infection or fungal infection, wherein the product is selected from a chimeric PRR, a nucleic acid, a vector, a recombinant cell, or a recombinant multicellular organism according to the herein disclosed invention.

Yet another aspect pertains to a method for increasing the resistance of a plant to a pathogen infection, comprising, introducing into said plant a chimeric PRR, a nucleic acid, a vector, a recombinant cell, or a recombinant multicellular organism of the invention.

A plant of the invention the in the plant a PRR according to any one of claims 1 to 17 is introduced by ectopic protein expression of said chimeric PRR in said plant.

In the herein disclosed invention the plant is a lower or higher plant, preferably including but not limited to corn (*Zea mays),* Brassica sp. (e.g., *B. napes, B. rapa, B.juncea),* alfalfa (*Medicago sativa*)*,* rye (*Secale cereale*)*,* sorghum (*Sorghum bicolor, Sorghum vulgare*)*,* sunflower (*Helianthus annuus*)*,* safflower (*Carthamus tinctorius*)*,* wheat (*Triticum* aestivum), soybean (*Glycine max*)*,* tobacco (*Nicotiana tabacum*)*,* potato (*Solanum tuberosum*)*,* peanuts (*Rachis hypogaea*)*,* cotton (*Gossypium barbadense, Gossypium hirsutum*)*,* sweet potato (*Ipomoea batatus*)*,* cassava (*Manihot esculenta*)*,* coffee (*Cofea spp.),* coconut (*Cocos nucifera*)*,* pineapple (*Ananas comosus*)*,* citrus trees (*Citrus spp.*)*,* cocoa (*Theobroma cacao*)*,* tea (*Camellia* sinensis), banana (*Musa spp.*)*,* avocado (*Perseaultilane*)*,* fig (*Ficuscasica*)*,* guava (Psidium guava), mango (Mangifera indica), olive (Olea europaea), papaya (Carica papaya), cashew (Anacardium occidentale), macadamia (Macadamia integrifolia), almond (Prunus amygdalus), sugar beets (Beta vulgaris), sugar-cane (Saccharum spp.), oats (Avena sativa), duckweed (Lemna), barley (Hordeum vulgare), tomatoes (Lycopersicon esculentum), lettuce (e. g., Lactuca sativa), green beans (Phaseolus vulgaris), lima beans (Phaseolus limensis), peas (Lathyrus spp.), and members of the genus Cucumis such as cucumber (C. sativus), cantaloupe (C. cantalupensis), and musk melon (C. melo). Ornamentals such as azalea (Rhododendron spp.), hydrangea (Macrophylla hydrangea), hibiscus (Hibiscus rosasanensis), roses (Rosa spp.), tulips (Tulipa spp.), daffodils (Narcissus spp.), petunias (Petunia hybrida), carnation (Dianthus caryophyllus), poinsettia (Euphorbia pulcherrima), and chrysanthemum (Chrysanthemum spp.), impatiens, Begonia, Pelargonium, Viola, Cyclamen, Verbena, Vinca, Tagetes, Primula, Saint Paulia, Agertum, Amaranthus, Antihirrhinum, Aquilegia, Cineraria, Clover, Cosmo, Cowpea, Dahlia, Datura, Delphinium, Gerbera, Gladiolus, Gloxinia, Hippeastrum, Mesembryanthemum, Salpiglossos, and Zinnia. Conifers that may be em-ployed in practicing the present invention include, for example, pines such as loblolly pine (Pinus taeda), slash pine (Pinus elliotii), ponderosa pine (Pinus ponderosa), lodgepole pine (Pinus contorta), and Monterey pine (Pinus radiata), Douglas-fir (Pseudotsuga menziesii); Western hemlock (Tsugaultilane); Sitka spruce (Picea glauca); redwood (Sequoia sempervirens); true firs such as silver fir (Abies amabilis) and balsam fir (Abies balsamea); and cedars such as Western red cedar (Thuja plicata), and Alaska yellow-cedar (Chamaecyparis nootkatensis.

Yet another aspect then pertains to a method for producing a transgenic plant having enhanced resistance to a pathogen infection, comprising the step of introducing into a plant or into a plant cell of said plant a chimeric PRR, a nucleic acid, a vector, a recombinant cell, or a recombinant multicellular organism of the invention. Also part of the invention is a plant, characterized in that it expresses a chimeric PRR as described herein before.

Another aspect also pertains to a screening method for damage- and/or microbe-associated molecular patterns (DAMPS, MAMPs), comprising the method steps of (i) expressing in a plant or plant cell a chimeric PRR according to the invention, (ii) contacting said plant or plant cell with a candidate compound, (iii) measuring the immune response of said plant or plant cell in comparison with a control plant or plant cell, wherein an elevated immune response of said plant or plant cell indicates that said candidate compound is a DAMP and/or MAMP.

One aspect of the invention pertains to a method for sensitizing a plant against bacterial/viral/fungal/microbial infections or herbivorous attacks, the method comprising performing in said plant a method for increasing the resistance of a plant to a pathogen infection.

In a final aspect the invention further pertains to a method for producing a chimeric PRR for mediating defense reactions, the chimeric PRR comprising at least an ectodomain, a transmembrane domain, and a cytoplasmatic domain, the method comprising a step of directly or indirectly fusing together (i) an ectodomain derived from Leucine Rich Repeat (LRR) Receptor Like Protein (RLP), to (ii) a transmembrane domain and/or a cytoplasmatic domain derived from a membrane bound kinase. Preferably the method is not an essentially biological process for the production of plants.

In context of this invention "fusing together" preferably constitutes either a step of introducing at least two donor nucleic acid sequences into one expressible nucleic acid sequence that encodes for the chimeric PRR, wherein the two donor nucleic acid sequences comprise a first donor nucleic acid sequence encoding for the ectodomain derived from the LRR-RLP, and a second donor nucleic acid encoding for the transmembrane domain and/or the cytoplasmatic domain derived from the membrane bound kinase.

The chimeric PRR is preferably one as disclosed herein above. Therefore, the above definitions and embodiments equally apply to the chimeric PRR produced by the above method and are omitted here in order to avoid unnecessary redundancy.

In some embodiments the method comprises a step of expressing said expressible nucleic acid sequence in a plant or plant cell, to obtain the chimeric PRR protein.

In order to allow for an easy isolation or purification of the chimeric PRRs of the present invention, said chimeric PRR of the invention is in preferred embodiments conjugated to a tag, for example a poly histidine tag or to any other detectable tag, like for example a fluorescent or luminescent protein, preferably to GFP. Also any other fusion protein derived from the chimeric PRR of the invention is comprised by the present invention.

Preferred is also the chimeric PRR according to the invention that when expressed in a plant cell induces, upon binding to a ligand, intracellular signalling in said plant cell, preferably yielding into an immune response or other defense or protective response of said plant cell, such as ethylene synthesis, expression of immune genes or production of reactive oxygen species (oxidative burst).

On the other hand another preferred embodiment of the invention is that the chimeric PRR when expressed in a plant cell, induces upon binding to a ligand intracellular signalling, preferably yielding into plant growth, plant cell death or a promotion of plant development or any other plant cellular response besides pathogen-specific responses.

In context of the invention an expressible sequence preferably relates to a genetic expression construct which allows for the expression of said nucleotide sequences in a plant, plant cell or plant tissue. It is specifically preferred that said construct is usable for stably or transiently transforming a plant, a plant cell or plant tissue with said construct.

For the expression of any constructs or vectors as described herein in a plant, plant tissue or plant cell, the invention preferably embodies that the described polynucleotides are operable linked to a promoter and to a polyadenylation site, wherein said promoter is characterized in that it is functional in said cell of said plant. As a promoter in this context, any sequence element is sufficient that induces transcription of the downstream sequence. The minimal requirements of promoters are very well known in the art and many of such promoters are conventionally used for gene expression in plants.

Also preferred in the context of the above method is to determine the binding of said chimeric PRR to said candidate ligand molecule by binding assays, such as described in Albert et al. 2010, or via competition assays.

Furthermore provided is a use of a chimeric PRR as described herein before, in a plant or plant cell or plant tissue to stimulate and/or promote plant growth, development or cell death.

Particularly preferred is the method as described herein, wherein said expression constructs allows for a plant cell type-specific or plant tissue-specific expression of said chimeric PRR. In this way it is possible to specifically flag certain plant cells of choice which then, by using the above method for isolation can be isolated/enriched and/or purified. In this respect it is preferred that said expression construct allows for a constitutive expression or a inducible expression after the transformation of the construct.

The matrix of choice in the above methods is selected from the group comprising tissue culture plates and dishes, beads or membranes.

All embodiments and aspects of the herein described invention may be combined with each other in a way plausible and technical feasible for the skilled artisan.

The present invention will now be further described in the following examples with reference to the accompanying figures and sequences, nevertheless, without being limited thereto. For the purposes of the present invention, all references as cited herein are incorporated by reference in their entireties. In the Figures and Sequences:

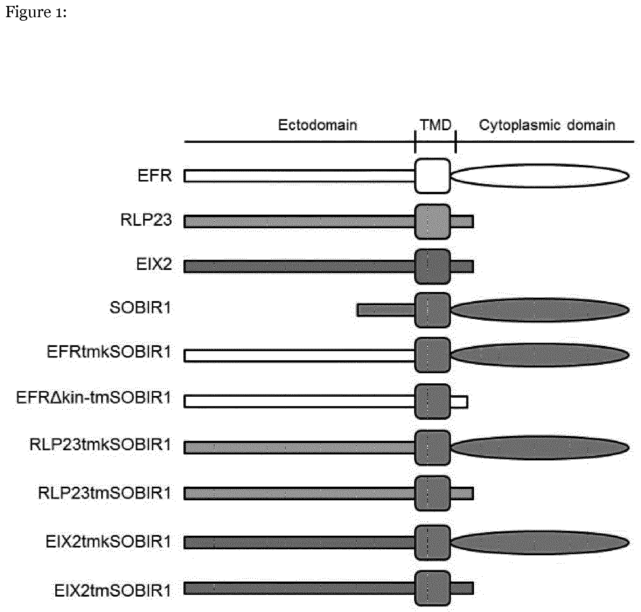

**Fig 1** depicts constructs used for turning a LRR-RLP into a LRR-RLK; to turn RLP23 or EIX2 into a RLK-like, the inventors added the TMD and the kinase of SOBIR1. To ensure that the resulting RLP23tmkSOBIR1 and EIX2tmkSOBIR1 behaves like a RLK, the inventors built RLP23tmSOBIR1 and EIX2tmSOBIR1 as control. The inventors also built EFRtmkSOBIR and EFRΔkin-tmSOBIR1 as controls.

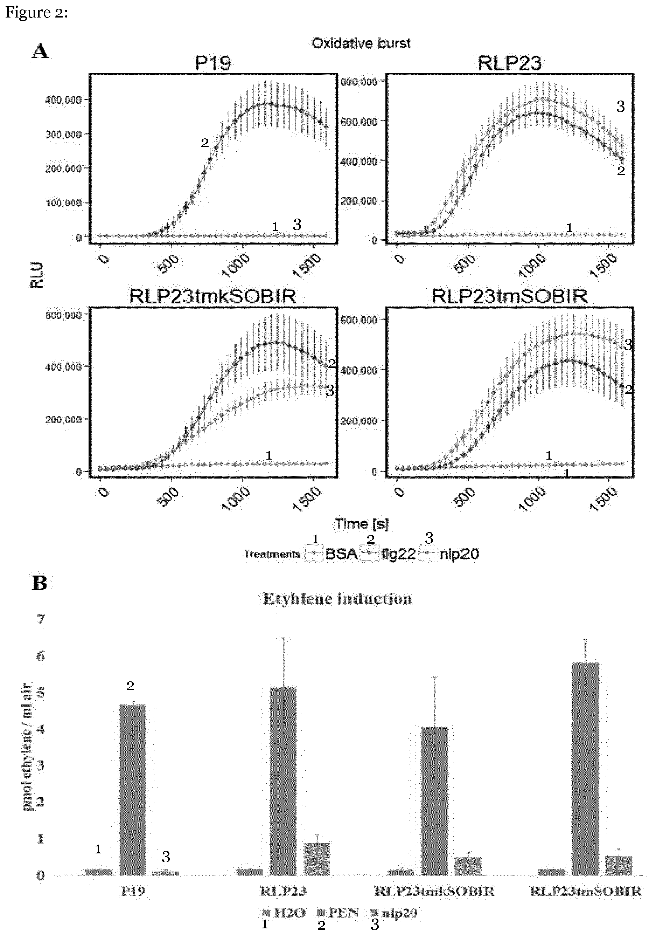

**Fig. 2** depicts RLP23 constructs containing the TMD of SOBIR1 can propagate nlp20 signaling; (A) Oxidative burst, treatments were flg22 100 nM (2, positive control), BSA (1, negative control) or nlp20 100 nM (3). Data show mean ± SD in luminol-dependent light emission (RLU, relative light units) of at least 4 replicates. (B) Ethylene induction in transformed leaf pieces treated for 3 h in the presence of 100 nM nlp20 (3), 4.5 µg Penicillium extract (2, positive control) or water (1, negative control). Values show mean ± SD in pmol ethylene per ml of air of 3 replicates.

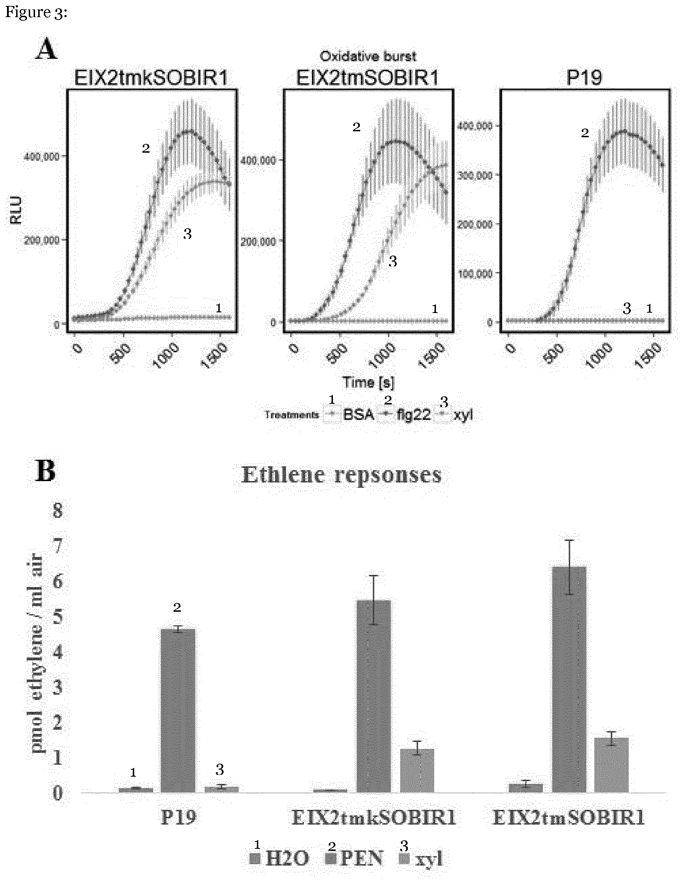

**Fig. 3** depicts EIX2 constructs containing the TMD of SOBIR1 can propagate xylanase signaling; (A) Oxidative burst, treatments were flg22 100 nM (2, positive control), BSA (1, negative control) or xylanase 1 mg/ml (3). Data show mean ± SD in luminol-dependent light emission (RLU, relative light units) of at least 4 replicates. (B) Ethylene induction in transformed leaf pieces treated for 3 h in the presence of 1 mg xylanase (3), 4.5 µg Penicillium extract (2, positive control) or water (1, negative control). Values show mean ± SD in pmol ethylene per ml of air of 3 replicates.

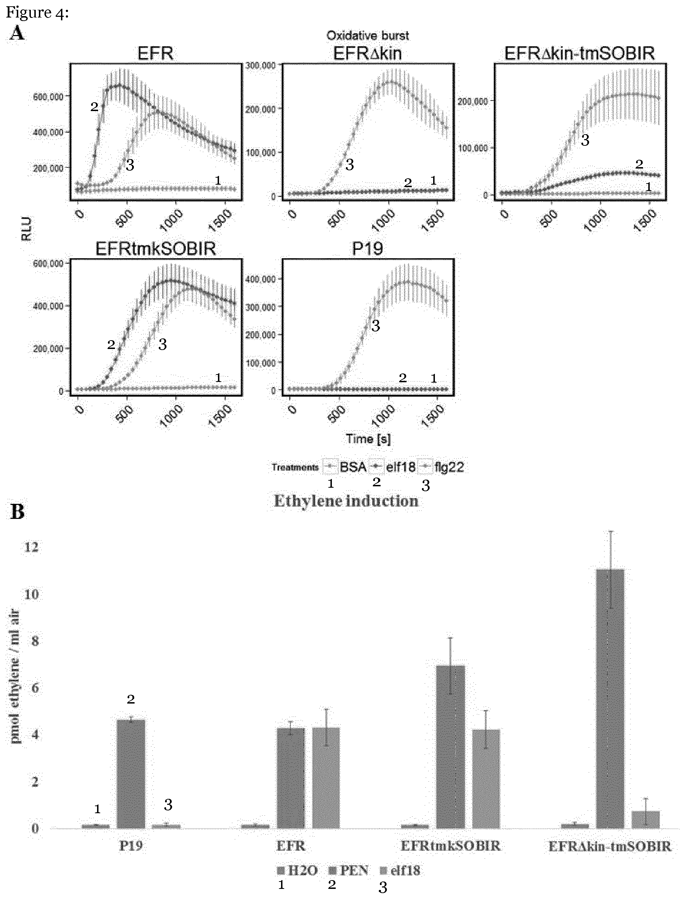

**Fig. 4** depicts EFR constructs containing the TMD of SOBIR1 can propagate elf18 signaling (A) Oxidative burst, treatments were flg22 100 nM (3, positive control), BSA (1, negative control) or elf18 100 nM (2). Data show mean ± SD in luminol-dependent light emission (RLU, relative light units) of at least 4 replicates. (B) Ethylene induction in transformed leaf pieces treated for 3 h in the presence of 100 nM elf18 (3), 4.5 µg Penicillium extract (2, positive control) or water (1, negative control). Values show mean ± SD in pmol ethylene per ml of air of 3 replicates.

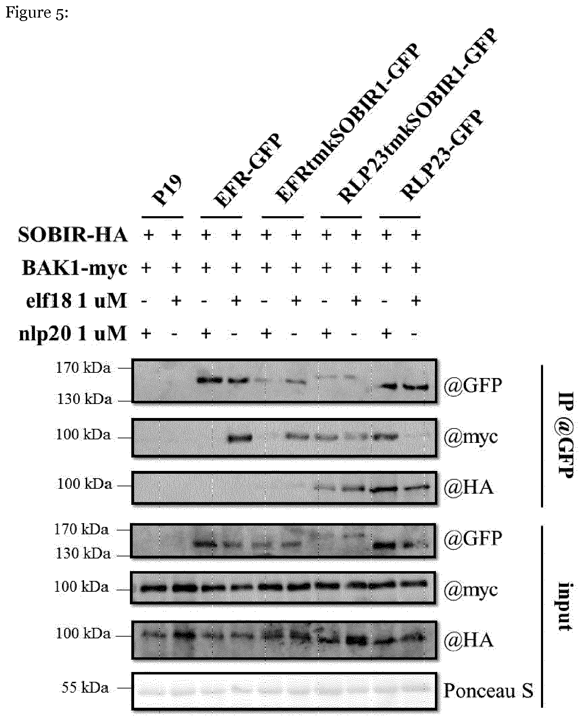

**Fig. 5** depicts co-immunoprecipitation of RLP23tmkSOBIR1 with SOBIR1 and BAKi; GFP-tagged receptors, SOBIR1-HA and BAK1-myc were expressed in N. benthamiana leaves for 72 h, were purified and tested for complex formation upon ligand-perception. Peptide was infiltrated at 1 µM for 3 min prior to harvest.

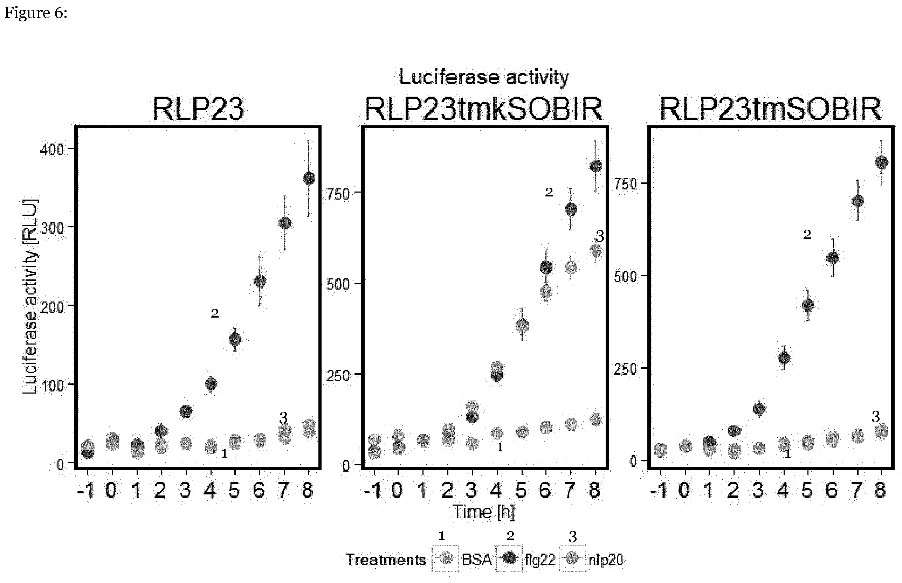

**Fig. 6** depicts RLP23tmkSOBIR can induce FRK1 in A. thaliana sobin1-12 mutant; Induction of the reporter gene pFRK1::luc in protoplasts of A. thaliana sobir1-12 mutant complemented with RLP23, RLP23tmkSOBIR or RLP23tmSOBIR and treated with BSA (1), flg22 (2) or with nlp20 (3).

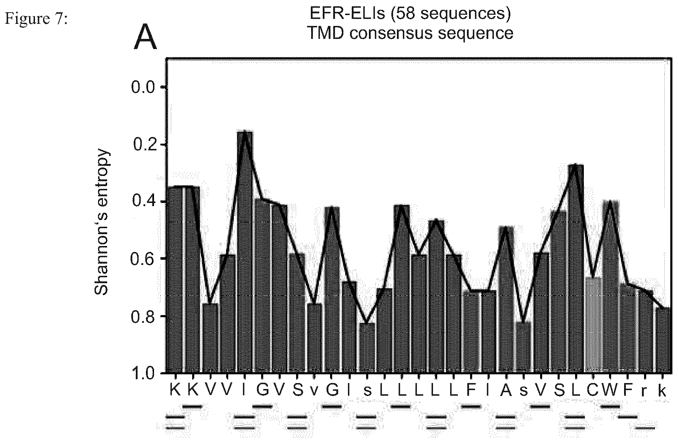

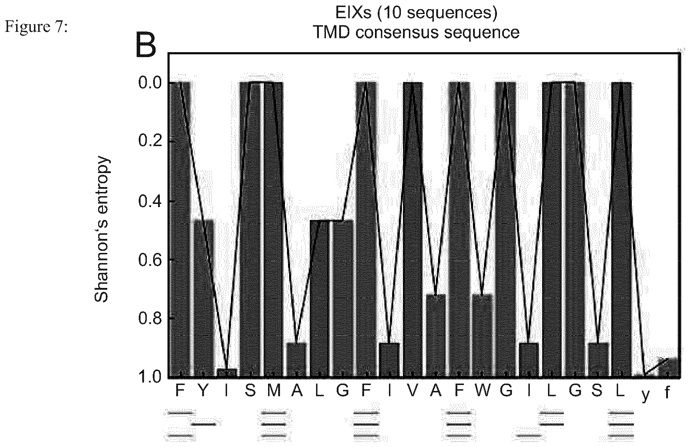

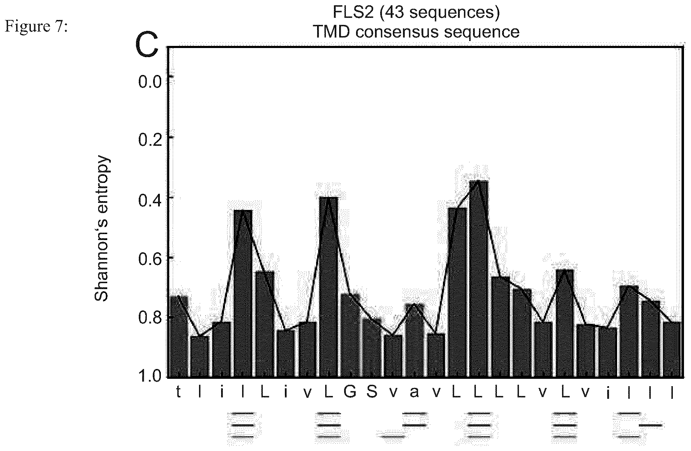

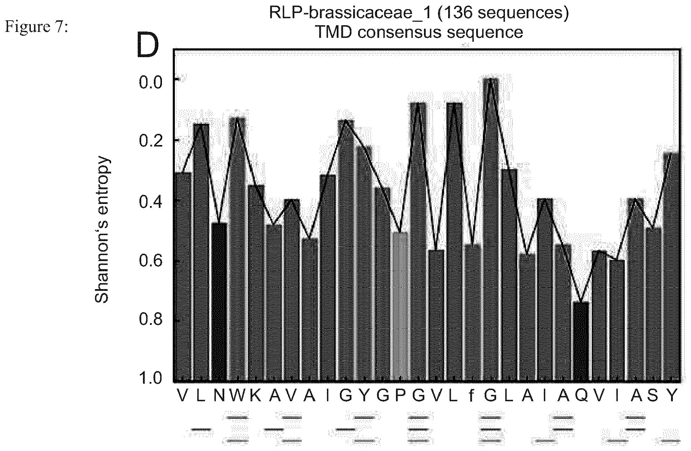

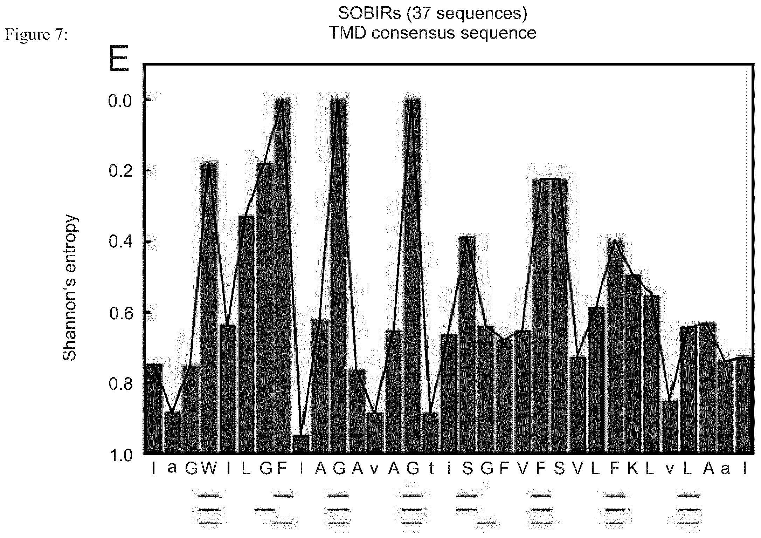

**Fig. 7** depicts entropy analysis of TMD consensus sequences; the entropy of each position in canonical TMD sequences reveals the conservation momentum of the α-helix for LRR-RLKs and LRR-RLPs. (A) EFR and EFR-likes (ELIs, 58 sequences), (B) EIX2 (10 sequences), (C) FLS2 (43 sequences), (D) RLP23 and homologs from Brassicaceae (136 sequences), (E) SOBIR1 (37 sequences). Entropy of 0.0 means high conservation (no variation), entropy of 1.0 means low conservation (high variation). Red for charged residues (R, K), green for polar residues (N, Q), salmon for proline (P), orange for small side chain residues (G, S, T, A), blue for hydrophobic residues (I, L, V) and yellow for hydrophobic residues with an aromatic ring (F, Y, W). The best three models for α-helix momentum are indicated below the consensus sequence and show entropy fits.

And in the sequences:
**SEQ ID NO: 1: EFR** 
MKLSFSLVFNALTLLLQVCIFAQARFSNETDMQALLEFKSQVSENNKREVLA
SWNHSSPFCNWIGVTCGRRRERVISLNLGGFKLTGVISPSIGNLSFLRLLNLA
DNSFGSTIPQKVGRLFRLQYLNMSYNLLEGRIPSSLSNCSRLSTVDLSSNHLG
HGVPSELGSLSKLAILDLSKNNLTGNFPASLGNLTSLQKLDFAYNQMRGEIPD
EVARLTQMVFFQIALNSFSGGFPPALYNISSLESLSLADNSFSGNLRADFGYLL
PNLRRLLLGTNQFTGAIPKTLANISSLERFDISSNYLSGSIPLSFGKLRNLWWL
GIRNNSLGNNSSSGLEFIGAVANCTQLEYLDVGYNRLGGELPASIANLSTTLTS
LFLGQNLISGTIPHDIGNLVSLQELSLETNMLSGELPVSFGKLLNLQVVDLYS
NAISGEIPSYFGNMTRLQKLHLNSNSFHGRIPQSLGRCRYLLDLWMDTNRLN
GTIPQEILQIPSLAYIDLSNNFLTGHFPEEVGKLELLVGLGASYNKLSGKMPQA
IGGCLSMEFLFMQGNSFDGAIPDISRLVSLKNVDFSNNNLSGRIPRYLASLPSL
RNLNLSMNKFEGRVPTTGVFRNATAVSVFGNTNICGGVREMQLKPCIVQASP
RKRKPLSVRKKVVSGICIGIASLLLIIIVASLCWFMKRKKKNNASDGNPSDSTT
LGMFHEKVSYEELHSATSRFSSTNLIGSGNFGNVFKGLLGPENKLVAVKVLN
LLKHGATKSFMAECETFKGIRHRNLVKLITVCSSLDSEGNDFRALVYEFMPK
GSLDMWLQLEDLERVNDHSRSLTPAEKLNIAIDVASALEYLHVHCHDPVAH
CDIKPSNILLDDDLTAHVSDFGLAQLLYKYDRESFLNQFSSAGVRGTIGYAAP
EYGMGGQPSIQGDVYSFGILLLEMFSGKKPTDESFAGDYNLHSYTKSILSGCT
SSGGSNAIDEGLRLVLQVGIKCSEEYPRDRMRTDEAVRELISIRSKFFSSKTTI
TESPRDAPQSSPQEWMLNTDMHTM
**SEQ ID NO: 2** b**RLP23** 
MSKALLHLHFLSLFLLCCVCHSSIFTLNFHFTGIVACRPHQIQAFTKFTNEFD
TRGCNNSDTFNGVWCDNSTGAVAVLQLRKCLSGTLKSNSSLFGFHQLRYVD
LQNNNLTSSSLPSGFGNLKRLEGLFLSSNGFLGQVPSSFSNLTMLAQLDLSYN
KLTGSFPLVRGLRKLIVLDLSYNHFSGTLNPNSSLFELHQLRYLNLAFNNFSS
SLPSKFGNLHRLENLILSSNGFSGQVPSTISNLTRLTKLYLDQNKLTSSFPLVQ
NLTNLYELDLSYNKFFGVIPSSLLTLPFLAHLALRENNLAGSVEVSNSSTSSRL
EIMYLGSNHFEGQILEPISKLINLKHLDLSFLNTSYPIDLKLFSSLKSLRSLDLS
GNSISSASLSSDSYIPLTLEMLTLRHCDINEFPNILKTLKELVYIDISNNRMKG
KIPEWLWSLPLLQSVTLGNNYFTGFQGSAEILVNSSVLLLYLDSNNFEGALPD
LPLSIKGFGVASNSFTSEIPLSICNRSSLAAIDLSYNNFTGPIPPCLRNLELVYLR
NNNLEGSIPDALCDGASLRTLDVSHNRLTGKLPRSFVNCSSLKFLSVINNRIE
 
DTFPFWLKALPNLQVLTLRSNRFYGPISPPHQGPLGFPELRIFEISDNKFTGSL
PPNYFVNWKASSRTMNQDGGLYMVYEEKLFDEGGYGYTDALDLQYKGLHM
EQAKALTSYAAIDFSGNRLEGQIPESIGLLKALIAVNISNNAFTGHIPLSMANL
ENLESLDMSRNQLSGTIPNGLGSISFLAYINVSHNQLTGEIPQGTQITGQSKSS
FEGNAGLCGLPLKESCFGTGAPPMYHQKQEDKEEEEEEEEEEEEVLNGRAV
AIGYGSGLLLGLAIAQVIASYKPEWLVKIIGLNKRRKR
**SEQ ID NO: 3 SOBIR1** 
MAVPTGSANLFLRPLILAVLSFLLLSSFVSSVEWLDIDSSDLKALQVIETELGV
NSQRSSASDVNPCGRRGVFCERRHSATTGEYVLRVTRLVYRSRSLTGTISPVI
GMLSELKELTLSNNQLVNAVPVDILSCKQLEVLDLRKNRFSGQIPGNFSSLSR
LRILDLSSNKLSGNLNFLKNLRNLENLSVANNLFSGKIPEQIVSFHNLRFFDF
SGNRYLEGPAPVMSSIKLQTSPHQTRHILAETPTSSPTNKPNNSTTSKAPKGA
PKPGKLKKKKKSKKKKVAAWILGFVVGAIGGTISGFVFSVLFKLIIQAIRGSE
KPPGPSIFSPLIKKAEDLAFLENEEALASLEIIGRGGCGEVFKAELPGSNGKIIA
VKKVIQPPKDADELTDEDSKFLNKKMRQIRSEINTVGHIRHRNLLPLLAHVS
RPECHYLVYEYMEKGSLQDILTDVQAGNQELMWPARHKIALGIAAGLEYLH
MDHNPRIIHRDLKPANVLLDDDMEARISDFGLAKAMPDAVTHITTSHVAGT
VGYIAPEFYQTHKFTDKCDIYSFGVILGILVIGKLPSDEFFQHTDEMSLIKWM
RNIITSENPSLAIDPKLMDQGFDEQMLLVLKIACYCTLDDPKQRPNSKDVRT
MLSQIKH
**SEQ ID NO: 4 EIX2** 
MGKRTNPRHFLVTWSLLLLETAFGLTSREVNKTLCIEKERGALLEFKRGLND
DFGRLSTWGDEEECCNWKGIECDKRTGHVIVLDLHSEVTCPGHACFAPILTG
KVSPSLLELEYLNFLDLSVNGFENSEIPRFIGSLKRLEYLNLSSSDFSGEIPAQF
QNLTSLRILDLGNNNLIVKDLVWLSHLSSLEFLRLGGNDFQARNWFREITKV
PSLKELDLSVCGLSKFVPSPADVANSSLISLSVLHLCCNEFSTSSEYSWLFNFST
SLTSIDLSHNQLSRQIDDRFGSLMYLEHLNLANNFGAEGGVPSSFGNLTRLH
YLDMSNTQTYQWLPELFLRLSGSRKSLEVLGLNDNSLFGSIVNVPRFSSLKKL
YLQKNMLNGFFMERVGQVSSLEYLDLSDNQMRGPLPDLALFPSLRELHLGS
NQFQGRIPQGIGKLSQLRIFDVSSNRLEGLPESMGQLSNLERFDASYNVLKGT
ITESHFSNLSSLVDLDLSFNLLSLNTRFDWVPPFQLQFIRLPSCNMGPSFPKW
LQTQNNYTLLDISLANISDMLPSWFSNLPPELKILNLSNNHISGRVSEFIVSKQ
DYMIIDLSSNNFSGHLPLVPANIQIFYLHKNHFSGSISSICRNTIGAATSIDLSR
NQFSGEVPDCWMNMSNLAVLNLAYNNFSGKVPQSLGSLTNLEALYIRQNSF
RGMLPSFSQCQLLQILDIGGNKLTGRIPAWIGTDLLQLRILSLRSNKFDGSIPS
LICQLQFLQILDLSENGLSGKIPQCLNNFTILRQENGSGESMDFKVRYDYIPGS
YLYIGDLLIQWKNQESEYKNALLYLKIIDLSSNKLVGGIPKEIAEMRGLRSLNL
SRNDLNGTVVEGIGQMKLLESLDLSRNQLSGMIPQGLSNLTFLSVLDLSNNH
 
LSGRIPSSTQLQSFDRSSYSGNAQLCGPPLEECPGYAPPIDRGSNTNPQEHDD
DDEFSSLEFYVSMVLGFFVTFWGILGCLIVNRSWRNAYFTFLTDMKSWLHM
TSRVCFARLKGKLRN
**SEQ ID NO: 5 RLP23tmSOBIR1** 
MSKALLHLHFLSLFLLCCVCHSSIFTLNFHFTGIVACRPHQIQAFTKFTNEFD
TRGCNNSDTFNGVWCDNSTGAVAVLQLRKCLSGTLKSNSSLFGFHQLRYVD
LQNNNLTSSSLPSGFGNLKRLEGLFLSSNGFLGQVPSSFSNLTMLAQLDLSYN
KLTGSFPLVRGLRKLIVLDLSYNHFSGTLNPNSSLFELHQLRYLNLAFNNFSS
SLPSKFGNLHRLENLILSSNGFSGQVPSTISNLTRLTKLYLDQNKLTSSFPLVQ
NLTNLYELDLSYNKFFGVIPSSLLTLPFLAHLALRENNLAGSVEVSNSSTSSRL
EIMYLGSNHFEGQILEPISKLINLKHLDLSFLNTSYPIDLKLFSSLKSLRSLDLS
GNSISSASLSSDSYIPLTLEMLTLRHCDINEFPNILKTLKELVYIDISNNRMKG
KIPEWLWSLPLLQSVTLGNNYFTGFQGSAEILVNSSVLLLYLDSNNFEGALPD
LPLSIKGFGVASNSFTSEIPLSICNRSSLAAIDLSYNNFTGPIPPCLRNLELVYLR
NNNLEGSIPDALCDGASLRTLDVSHNRLTGKLPRSFVNCSSLKFLSVINNRIE
DTFPFWLKALPNLQVLTLRSNRFYGPISPPHQGPLGFPELRIFEISDNKFTGSL
PPNYFVNWKASSRTMNQDGGLYMVYEEKLFDEGGYGYTDALDLQYKGLHM
EQAKALTSYAAIDFSGNRLEGQIPESIGLLKALIAVNISNNAFTGHIPLSMANL
ENLESLDMSRNQLSGTIPNGLGSISFLAYINVSHNQLTGEIPQGTQITGQSKSS
FEGNAGLCGLPLKESCFGTGAPPMYHQKQEDKEEEEEEEEEEEEVAAWILGF
VVGAIGGTISGFVFSVLFKPEWLVKIIGLNKRRKR
**SEQ ID NO: 6 RLP2**3**tmkSOBIR1** 
MSKALLHLHFLSLFLLCCVCHSSIFTLNFHFTGIVACRPHQIQAFTKFTNEFD
TRGCNNSDTFNGVWCDNSTGAVAVLQLRKCLSGTLKSNSSLFGFHQLRYVD
LQNNNLTSSSLPSGFGNLKRLEGLFLSSNGFLGQVPSSFSNLTMLAQLDLSYN
KLTGSFPLVRGLRKLIVLDLSYNHFSGTLNPNSSLFELHQLRYLNLAFNNFSS
SLPSKFGNLHRLENLILSSNGFSGQVPSTISNLTRLTKLYLDQNKLTSSFPLVQ
NLTNLYELDLSYNKFFGVIPSSLLTLPFLAHLALRENNLAGSVEVSNSSTSSRL
EIMYLGSNHFEGQILEPISKLINLKHLDLSFLNTSYPIDLKLFSSLKSLRSLDLS
GNSISSASLSSDSYIPLTLEMLTLRHCDINEFPNILKTLKELVYIDISNNRMKG
KIPEWLWSLPLLQSVTLGNNYFTGFQGSAEILVNSSVLLLYLDSNNFEGALPD
LPLSIKGFGVASNSFTSEIPLSICNRSSLAAIDLSYNNFTGPIPPCLRNLELVYLR
NNNLEGSIPDALCDGASLRTLDVSHNRLTGKLPRSFVNCSSLKFLSVINNRIE
DTFPFWLKALPNLQVLTLRSNRFYGPISPPHQGPLGFPELRIFEISDNKFTGSL
PPNYFVNWKASSRTMNQDGGLYMVYEEKLFDEGGYGYTDALDLQYKGLHM
EQAKALTSYAAIDFSGNRLEGQIPESIGLLKALIAVNISNNAFTGHIPLSMANL
ENLESLDMSRNQLSGTIPNGLGSISFLAYINVSHNQLTGEIPQGTQITGQSKSS
FEGNAGLCGLPLKESCFGTGAPPMYHQKQEDKEEEEEEEEEEEEVAAWILGF
 
VVGAIGGTISGFVFSVLFKLIIQAIRGSEKPPGPSIFSPLIKKAEDLAFLENEEAL
ASLEIIGRGGCGEVFKAELPGSNGKIIAVKKVIQPPKDADELTDEDSKFLNKK
MRQIRSEINTVGHIRHRNLLPLLAHVSRPECHYLVYEYMEKGSLQDILTDVQ
AGNQELMWPARHKIALGIAAGLEYLHMDHNPRIIHRDLKPANVLLDDDME
ARISDFGLAKAMPDAVTHITTSHVAGTVGYIAPEFYQTHKFTDKCDIYSFGVIL
GILVIGKLPSDEFFQHTDEMSLIKWMRNIITSENPSLAIDPKLMDQGFDEQM
LLVLKIACYCTLDDPKQRPNSKDVRTMLSQIKH
**SEQ ID NO: 7 EIX2tmSOBIRi** 
MGKRTNPRHFLVTWSLLLLETAFGLTSREVNKTLCIEKERGALLEFKRGLND
DFGRLSTWGDEEECCNWKGIECDKRTGHVIVLDLHSEVTCPGHACFAPILTG
KVSPSLLELEYLNFLDLSVNGFENSEIPRFIGSLKRLEYLNLSSSDFSGEIPAQF
QNLTSLRILDLGNNNLIVKDLVWLSHLSSLEFLRLGGNDFQARNWFREITKV
PSLKELDLSVCGLSKFVPSPADVANSSLISLSVLHLCCNEFSTSSEYSWLFNFST
SLTSIDLSHNQLSRQIDDRFGSLMYLEHLNLANNFGAEGGVPSSFGNLTRLH
YLDMSNTQTYQWLPELFLRLSGSRKSLEVLGLNDNSLFGSIVNVPRFSSLKKL
YLQKNMLNGFFMERVGQVSSLEYLDLSDNQMRGPLPDLALFPSLRELHLGS
NQFQGRIPQGIGKLSQLRIFDVSSNRLEGLPESMGQLSNLERFDASYNVLKGT
ITESHFSNLSSLVDLDLSFNLLSLNTRFDWVPPFQLQFIRLPSCNMGPSFPKW
LQTQNNYTLLDISLANISDMLPSWFSNLPPELKILNLSNNHISGRVSEFIVSKQ
DYMIIDLSSNNFSGHLPLVPANIQIFYLHKNHFSGSISSICRNTIGAATSIDLSR
NQFSGEVPDCWMNMSNLAVLNLAYNNFSGKVPQSLGSLTNLEALYIRQNSF
RGMLPSFSQCQLLQILDIGGNKLTGRIPAWIGTDLLQLRILSLRSNKFDGSIPS
LICQLQFLQILDLSENGLSGKIPQCLNNFTILRQENGSGESMDFKVRYDYIPGS
YLYIGDLLIQWKNQESEYKNALLYLKIIDLSSNKLVGGIPKEIAEMRGLRSLNL
SRNDLNGTVVEGIGQMKLLESLDLSRNQLSGMIPQGLSNLTFLSVLDLSNNH
LSGRIPSSTQLQSFDRSSYSGNAQLCGPPLEECPGYAPPIDRGSNTNPQEHDD
DDEFSSLEVAAWILGFVVGAIGGTISGFVFSVLFRSWRNAYFTFLTDMKSWL
HMTSRVCFARLKGKLRN
**SEQ ID NO: 8 EIX2tmkSOBIRi** 
MGKRTNPRHFLVTWSLLLLETAFGLTSREVNKTLCIEKERGALLEFKRGLND
DFGRLSTWGDEEECCNWKGIECDKRTGHVIVLDLHSEVTCPGHACFAPILTG
KVSPSLLELEYLNFLDLSVNGFENSEIPRFIGSLKRLEYLNLSSSDFSGEIPAQF
QNLTSLRILDLGNNNLIVKDLVWLSHLSSLEFLRLGGNDFQARNWFREITKV
PSLKELDLSVCGLSKFVPSPADVANSSLISLSVLHLCCNEFSTSSEYSWLFNFST
SLTSIDLSHNQLSRQIDDRFGSLMYLEHLNLANNFGAEGGVPSSFGNLTRLH
YLDMSNTQTYQWLPELFLRLSGSRKSLEVLGLNDNSLFGSIVNVPRFSSLKKL
YLQKNMLNGFFMERVGQVSSLEYLDLSDNQMRGPLPDLALFPSLRELHLGS
NQFQGRIPQGIGKLSQLRIFDVSSNRLEGLPESMGQLSNLERFDASYNVLKGT
 
ITESHFSNLSSLVDLDLSFNLLSLNTRFDWVPPFQLQFIRLPSCNMGPSFPKW
LQTQNNYTLLDISLANISDMLPSWFSNLPPELKILNLSNNHISGRVSEFIVSKQ
DYMIIDLSSNNFSGHLPLVPANIQIFYLHKNHFSGSISSICRNTIGAATSIDLSR
NQFSGEVPDCWMNMSNLAVLNLAYNNFSGKVPQSLGSLTNLEALYIRQNSF
RGMLPSFSQCQLLQILDIGGNKLTGRIPAWIGTDLLQLRILSLRSNKFDGSIPS
LICQLQFLQILDLSENGLSGKIPQCLNNFTILRQENGSGESMDFKVRYDYIPGS
YLYIGDLLIQWKNQESEYKNALLYLKIIDLSSNKLVGGIPKEIAEMRGLRSLNL
SRNDLNGTVVEGIGQMKLLESLDLSRNQLSGMIPQGLSNLTFLSVLDLSNNH
LSGRIPSSTQLQSFDRSSYSGNAQLCGPPLEECPGYAPPIDRGSNTNPQEHDD
DDEFSSLEVAAWILGFVVGAIGGTISGFVFSVLFKLIIQAIRGSEKPPGPSIFSPL
IKKAEDLAFLENEEALASLEIIGRGGCGEVFKAELPGSNGKIIAVKKVIQPPKD
ADELTDEDSKFLNKKMRQIRSEINTVGHIRHRNLLPLLAHVSRPECHYLVYE
YMEKGSLQDILTDVQAGNQELMWPARHKIALGIAAGLEYLHMDHNPRIIHR
DLKPANVLLDDDMEARISDFGLAKAMPDAVTHITTSHVAGTVGYIAPEFYQT
HKFTDKCDIYSFGVILGILVIGKLPSDEFFQHTDEMSLIKWMRNIITSENPSLA
IDPKLMDQGFDEQMLLVLKIACYCTLDDPKQRPNSKDVRTMLSQIKH
**SEQ ID NO: 9 EFRtmkSOBIRi** 
MKLSFSLVFNALTLLLQVCIFAQARFSNETDMQALLEFKSQVSENNKREVLA
SWNHSSPFCNWIGVTCGRRRERVISLNLGGFKLTGVISPSIGNLSFLRLLNLA
DNSFGSTIPQKVGRLFRLQYLNMSYNLLEGRIPSSLSNCSRLSTVDLSSNHLG
HGVPSELGSLSKLAILDLSKNNLTGNFPASLGNLTSLQKLDFAYNQMRGEIPD
EVARLTQMVFFQIALNSFSGGFPPALYNISSLESLSLADNSFSGNLRADFGYLL
PNLRRLLLGTNQFTGAIPKTLANISSLERFDISSNYLSGSIPLSFGKLRNLWWL
GIRNNSLGNNSSSGLEFIGAVANCTQLEYLDVGYNRLGGELPASIANLSTTLTS
LFLGQNLISGTIPHDIGNLVSLQELSLETNMLSGELPVSFGKLLNLQVVDLYS
NAISGEIPSYFGNMTRLQKLHLNSNSFHGRIPQSLGRCRYLLDLWMDTNRLN
GTIPQEILQIPSLAYIDLSNNFLTGHFPEEVGKLELLVGLGASYNKLSGKMPQA
IGGCLSMEFLFMQGNSFDGAIPDISRLVSLKNVDFSNNNLSGRIPRYLASLPSL
RNLNLSMNKFEGRVPTTGVFRNATAVSVFGNTNICGGVREMQLKPCIVQASP
RKRKPLSVRKKVAAWILGFVVGAIGGTISGFVFSVLFKLIIQAIRGSEKPPGPSI
FSPLIKKAEDLAFLENEEALASLEIIGRGGCGEVFKAELPGSNGKIIAVKKVIQP
PKDADELTDEDSKFLNKKMRQIRSEINTVGHIRHRNLLPLLAHVSRPECHYL
VYEYMEKGSLQDILTDVQAGNQELMWPARHKIALGIAAGLEYLHMDHNPRI
IHRDLKPANVLLDDDMEARISDFGLAKAMPDAVTHITTSHVAGTVGYIAPEF
YQTHKFTDKCDIYSFGVILGILVIGKLPSDEFFQHTDEMSLIKWMRNIITSENP
SLAIDPKLMDQGFDEQMLLVLKIACYCTLDDPKQRPNSKDVRTMLSQIKH
**SEQ ID NO: 10 EFRΔkin-tmSOBIR1** MKLSFSLVFNALTLLLQVCIFAQARFSNETDMQALLEFKSQVSENNKREVLA 
SWNHSSPFCNWIGVTCGRRRERVISLNLGGFKLTGVISPSIGNLSFLRLLNLA
DNSFGSTIPQKVGRLFRLQYLNMSYNLLEGRIPSSLSNCSRLSTVDLSSNHLG
HGVPSELGSLSKLAILDLSKNNLTGNFPASLGNLTSLQKLDFAYNQMRGEIPD
EVARLTQMVFFQIALNSFSGGFPPALYNISSLESLSLADNSFSGNLRADFGYLL
PNLRRLLLGTNQFTGAIPKTLANISSLERFDISSNYLSGSIPLSFGKLRNLWWL
GIRNNSLGNNSSSGLEFIGAVANCTQLEYLDVGYNRLGGELPASIANLSTTLTS
LFLGQNLISGTIPHDIGNLVSLQELSLETNMLSGELPVSFGKLLNLQVVDLYS
NAISGEIPSYFGNMTRLQKLHLNSNSFHGRIPQSLGRCRYLLDLWMDTNRLN
GTIPQEILQIPSLAYIDLSNNFLTGHFPEEVGKLELLVGLGASYNKLSGKMPQA
IGGCLSMEFLFMQGNSFDGAIPDISRLVSLKNVDFSNNNLSGRIPRYLASLPSL
RNLNLSMNKFEGRVPTTGVFRNATAVSVFGNTNICGGVREMQLKPCIVQASP
RKRKPLSVRKKVAAWILGFVVGAIGGTISGFVFSVLFMKRKKKNNASDGNPS
DSTTLGMFHEKVSYEELHSA
SEQ ID NO: 11 to 20 depict nucleic acid sequences encoding the corresponding amino acid sequences of SEQ ID NO: 1 to 10, in that order (see table 1).

### EXAMPLES

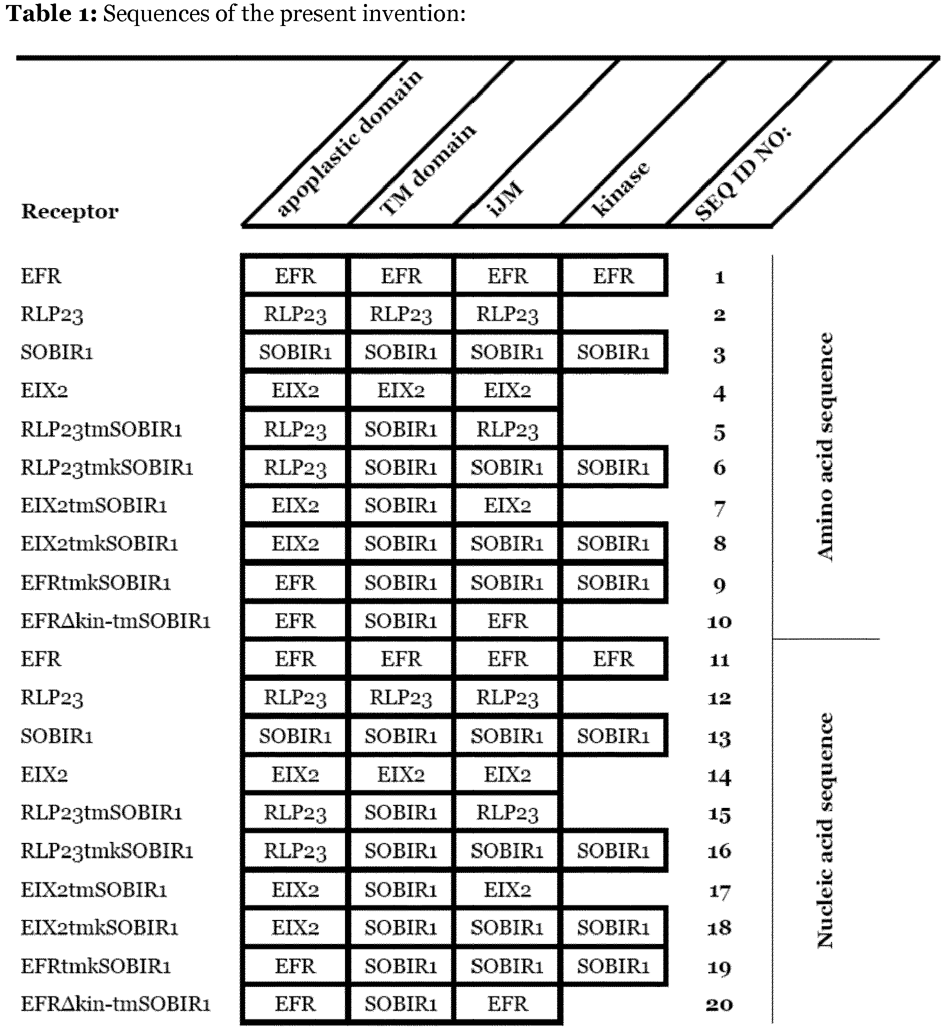

### Molecular differences between LRR-RLKs and LRR-RLPs: turning a RLP in a RLK-like

To identify the molecular characteristics of RLKs and based on the previous results, the inventors generated chimeric receptors containing the ectodomain of RLP23 and either the TMD and kinase of SOBIR1 (RLP23tmkSOBIR1) or the TMD of SOBIR1 and the cytoplasmic C-tail of RLP23 (RLP23tmSOBIR1, Fig. 1) to assess what molecular features are important for RLK-like functionality. As control, the inventors built similar construct based on EIX2 and EFRΔkin. The inventors transiently expressed these constructs in *N. benthamiana,* a RLP23-, EIX2- and EFR-free background, and could observe for all constructs a gain of perception to nlp20, xylanase and elf18, respectively (Fig. 2-4). The constitutive interaction with SOBIR1 and the ligand-dependent interaction with BAK1 for RLP23tmkSOBIR1 and EFRtmkSOBIR1 was subsequently shown in a co-immunoprecipitation assay from transformed *N. benthamiana* material (Fig. 5), thus suggesting a tripartite kinase complex. Finally, to determine whether these constructs truly behave like RLKs, the inventors measured the induction of the FRK1 reporter gene in protoplasts of *A. thaliana* sobir1-12 mutants transiently expressing either RLP23tmkSOBIR1 or RLP23tmSOBIR1. As depicted in Figure 6, RLP23tmkSOBIR1, but not RLP23tmSOBIR1, could induce FRK1 expression, thus showing that the former does behave like a RLK.

### Analysis of TMD sequences using Shannon's entropy

To study the evolution of LRR receptors, the inventors first had to establish a suitable dataset. For that, AtEFR (UniProt ID: CoLGT6) was BLASTed against all plant reference proteomes available on UniProt (www.uniprot.org). A CLANS map was generated using the best 1000 hits from every proteome. The 1000 hits per proteome mark was chosen to keep the following computing steps to a reasonable time. A pairwise comparison was launched using the global alignment STRETCHER from EMBOSS to generate the CLANS map. Length-adjusted scores were then used as cutoff in the CLANS map, as p-values were giving a suboptimal discriminating power within this dataset. For instance, when running a BLASTp search on NCBI for AtEFR against all Viridiplantae (taxonomy ID: 33090, max target output: 5000), the typical results would show over 2500 results having an E-value of 0.0, while their Max scores vary from 2099 to 546, giving a long range of differences which are not reflected in E-values. Nota bene, the analysis was performed on sequences available on UniProt, which in some cases may contain sequencing errors or sequences that might have been mislabelled. The CLANS map ran at different cutoff values to identify the optimal cutoff: enough to separate clusters that they can be individually identified, but not so much that it becomes tedious work to decipher the map. For the rest of the analysis, the length-adjusted HSP score cutoff of 2.0 was chosen as indicative of cluster evolution. After manually annotating the map by accessing UniProt Reference Proteome annotations and checking back on the NR database for annotations not present in the Reference Proteomes, sequences of the main clusters were extracted on separate FASTA files and the phylogenetic tree of each cluster was established. Sequences from each cluster were aligned using Clustal Omega (v. 1.2.1) and alignments were refined using MUSCLE (v. 3.8.31). TMD were identified with PolyPhobius (phobi-us.sbc.su.se/poly.html).

Based on the above results, the inventors extracted from a CLANS map the sequences of SOBIR1, EFR, RLP23, FLS2 and EIX2 and their respective homologs from other plant species and aligned their TMD sequences. The inventors then analyzed the variation of the canonical TMD sequences and calculated the entropy for each position, using Shannon's equation (Shannon, 1948). After that, the inventors plotted the resulting consensus sequences, with uppercase symbols representing the amino acids that are highly conserved and lowercase letters representing positions where there is some variation among the canonical sequences. The inventors fitted the entropy of each position to a hypothetical α-helix momentum. The angle in a straight α-helix is of 3.63 residues per turn, but natural variations can present angles of 3.5 (7 residues over 2 complete turns, depicted as 7/2), 3.6 (18/5), 3.66 (11/3) or 3.75 (15/4) (Lupas and Bassler, 2017). The inventors fitted the entropies of this TMD canonical sequences on the best helical momentum, as based on the conservation of each position of the TMD. As depicted on Fig. 7, the TMD sequences of EIXs, RLP23s and SOBIRis contain a highly conserved GxxxG motif, whereas the TMD of EFRs or FLS2s do not present such a motif. Moreover, the TMD of LRR-RLPs and SOBIR1 are richer in aromatic residues which are located opposite, before or after to the GxxxG motif. Such residues are in contrast not present in such proportions in the TMDs of LRR-RLKs.

Furthermore, the GxxxG motif in the TMD of SOBIR1 locates in the first half of the sequence, whereas this motif appears in the middle and final sections of the TMD from LRR-RLPs, thus suggesting that other residues are playing a role and might act as key-hole actors to ensure the specificity of interaction.

### Methods:

### Chemicals and solvents

Chemicals and solvents were ordered from Sigma-Aldrich (St. Louis, USA), Carl Roth (Karlsruhe), Merck (Darmstadt), VWR (Radnor, USA), Duchefa (Haarlem, NL) and Fluka (Buchs, CH).

### Peptides and elicitors

Flg22, nlp20 and elf18 were synthetized and provided by different suppliers. Stock solutions of peptides were prepared in H2O and diluted in a solution medium with 1 mg/ml BSA and 100 mM NaCl.

### Bacterial strains

XL1 blue *Escherichia coli* were used for vector production in bacteria (maxi- and mini-prep). TOP10 *E. coli* were used for pENTR TOPO cloning. *E. coli* were grown in liquid LB medium at 37°C and 200 rpm in a shaker or on LB-agarose plates at 37°C in stationary incubators. *Agrobacterium tumefaciens* GV3101 were used for plant transformation and transient expression in *Nicotiana benthamiana. A. tumefaciens* were transformed by electroporation and positive colonies were picked after 48h growth on LB-agarose plates at 30°C in stationary incubators. They were then grown overnight at 30°C and 250 rpm in a shaker in liquid LB medium.

### Plant material

Seedlings of *Arabidopsis thaliana* were grown at 22°C under a 8:16 h light:dark photoperiod at 70 µE/m2/s and 40-65% relative humidity in controlled environment (four weeks covered with a lid in a Percival growth chamber, followed by two weeks without lid in a Mobylux Grow Bank, CLF Plant-Climatics, Emersacker). Leaves of 4-6 weeks old *A*. *thaliana* sobin1-12 mutant plants were used for protoplasts isolation and transformation. To study protein expression and functionality in a different plant background, 4-5 weeks old *Nicotiana benthamiana* were used for bioassays as well as for interaction studies. They were grown in the greenhouse at 22°C 16:8 h light:dark photoperiod.

### Agrobacterium-mediated transformation of Nicotiana benthamiana

*Nicotiana benthamiana* is commonly used for transient expression assays (e.g. Albert *et al.,* 2015). Transformation was mediated by *Agrobacterium tumefaciens* (strain GV3101) carrying plasmids encoding the gene of interest. After overnight incubation of transformed bacteria in LB medium (at 30°C, 250 rpm), bacteria were collected by centrifugation (4000 *g,* 8 min) and resuspended to an OD of 1 in 10 mM MgCl2 with 150 uM acetosyringone. After incubation at room-temperature for 90 min, bacteria were diluted to an OD of 0.4, mixed 1:1 with *A. tumefaciens* (C58C1) carrying the P19 suppressor of silencing (Voinnet *et al.,* 2003) and pressure-infiltrated in 4-5 week old *N. benthamiana* leaves. Leaves were cut in pieces for bioassays 24h after infiltration, or harvested 36h after infiltration and directly frozen in liquid nitrogen for protein expression analysis.

### Protoplasts isolation and pFRK1::Luc assay

Transient expression in mesophyll protoplasts from *Arabidopsis thaliana* sobin1-12 mutant leaves was performed as described in (Yoo *et al.,* 2007). Briefly, aliquots of 20 000 protoplasts were cotransformed with 5 µg plasmid with the reporter construct pFRK1::luciferase and 5 µg plasmid with the receptor construct to be tested. Protoplasts were resuspended in W5 solution with 0.2 mM luciferin. Aliquots were incubated overnight (max 12h) in a 96-well plate before treatment with the respective peptides (BSA-NaCl as control, elfi8, pepi, BL, csp22, CLV3, flg22). Luciferase activity was monitored via light emission using a luminometer (Mithras LB 940).

### Oxidative burst

Analysis of oxidative burst was performed on leaf pieces (see Ethylene production, above) of transiently transformed *N. benthamiana.* Leaf pieces were placed in individual wells of a 96-well plate with a 90:10 µl H2O:mastermix solution (1 ml H2O, 20 µl luminol, 3 µl peroxidase). After a 5-10 min pre-run to test for the background of ROS production, leaf pieces were induced with either the peptide to be tested, BSA/NaCl (neg. control) or flg22 100 nM (positive control) as previously described (Albert et al., 2010).

### PCRs. electrophoresis and DNA sequencing

Primers were designed with CLC Workbench 7, with an optimal length of 20-22 nt, ideally with 60% GC content and a melting temperature between 55-63°C. The forward primers had an additional "CACC" overhang for directional pENTR TOPO cloning. Chimeric receptors were created by binding two separate PCR products with overlapping ends as previously described (Albert *et al.,* 2010*c*; Mueller *et al.,* 2012*a*). Truncated constructs were generated using reverse primers at the desired target (no stop codon). Chimeric receptors were built with normal external primers and overlapping chimeric internal primers, which had half of each sequence and were 40-50 nt long. All primers were ordered from Sigma-Aldrich.

Gel electrophoresis were all performed with 1% agarose-TAE gels and band purification was done with the GeneJET Gel Extraction kit from ThermoFisher Scientific. DNA sequencing was performed by GATC Biotech (Konstanz).

### Vectors

After using pENTR/TEV/D-TOPO or pENTR/D-TOPO as entry vectors (pENTR directional TOPO kits, Invitrogen), constructs were exported to EcoRI-digested pK7FWG2 expression vectors (Spectinomycin resistance in bacteria), containing the cauliflower mosaic virus 35S overexpressing promoter and a C-terminal GFP tag. Alternatively, pGWB14 was used for HA-tagged constructs and pGWB17 for Myc-tagged constructs (both vectors have Hygromycin and Kanamycin resistances in bacteria).

### Protein biochemistry

Protein separation from crude extract or immune-enriched fractions was achieved with 8% SDS-PAGE gels, unless indicated otherwise. The inventorsstern blotting to nitrocellulose membrane (GE Healthcare) was performed using semi-dry western blotting technology from BioRad. Immunoprecipitation (IP) and co-IP was based on magnetic GFP-Trap MA beads (unless indicated otherwise) from Chromotek, and performed as described in Jehle et al., (2013). Crude extract blotting used 30 mg of grinded leaf material resuspended in 2 volumes [w/v] of loading buffer mixed with β-mercaptoethanol. The following antibodies were used: anti-GFP produced in rabbit (Torrey Pines Biolabs Inc.), anti-Myc produced in rabbit (Sigma-Aldrich) and anti-HA produced in mouse (Sigma-Aldrich).

## Claims

1. A chimeric pattern recognition receptor (PRR) for mediating defense reactions, comprising at least an ectodomain, a transmembrane domain, and a cytoplasmatic domain, **characterized in that** the ectodomain is derived from Leucine Rich Repeat (LRR) Receptor Like Protein (RLP), and at least the transmembrane domain and/or the cytoplasmatic domain is derived from a membrane bound kinase.

2. The chimeric PRR according to claim 1, wherein the membrane bound kinase is selected from the group of leucine rich repeat (LRR) receptor-like kinases (RLK), such as a kinase selected from any of the following protein families: C-Lectin, CR4-like, CRPK1-like1, CRPKi-like2, DUF26, Extensin-like, L-Lectin, LRK10-like1, LRK10-like2, GDPDb, LRR I, LRR II, LRR III, LRR IV, LRR V, LRR VI, LRR VII, LRR VIII-1, LRR VIII-2, LRR IX, LRR X, LRR XI, LRR XII, LRR XIII, LRR XIV, LysM, PERK, RKF3-like, RLCK I, RLCK II, RLCK III, RLCK IV, TAK(RLCK V), RLCK VI, RLCK VII, RLCK VIII, RLCK IX, RLCK X, RLCK XI, RLCK XII, S-domain1, S-domain2, S-domain3, Thaumatin, URK I, or WAK-like.

3. The chimeric PRR according to claim 1 or 2, wherein the LRR-RLK is selected from any one of the following RLKs: BAK1, SERK4, SISERK1, BIR1, BIR2, SOBIR1, FER, CRK28, IOS1, PSKR1, PSY1R, ERECTA, SRF3, XA26, dsi, Bti9, SILyk13, THE1, Pi-d2, LecRK-I.9, LecRK-V.5, LecRK-VI.2, NgRLK1, LecRK1, NbLRK1, WAKL22, OsWAK1, TaRLK-R1,2,3, SNC4, LRK10, SNC2, and RLP52; and preferably wherein the LRR-RLK is SOBIR1.

4. The chimeric PRR according to any one of claims 1 to 3, wherein the transmembrane domain and the cytoplasmatic domain are derived from the LRR-RLK, such as and preferably SOBIR1.

5. The chimeric PRR according to any one of claims 1 to 4, wherein the ectodomain is capable of binding to a molecular pattern associated with and indicative for the presence of microbial pathogens, herbivores, parasitic plants or tissue injury, such as a pathogen- and/or damage-associated molecular patterns (PAMPs, DAMPs), for example xylanase from gungi, flagellin and Emax from bacteria; or patterns indicative for cell necrosis, or ethylene-inducing peptide 1-like proteins (NLPs) from bacteria, fungi or oomycetes.

6. A nucleic acid comprising a nucleotide sequence encoding for a chimeric PRR protein according to any one of claims 1 to 5.

7. A vector, such as an expression vector, comprising a nucleic acid according to claim 6.

8. A recombinant cell comprising a chimeric PRR according to any one of claims 1 to 5, a nucleic acid according to claim 6, or a vector according to claim 7

9. A recombinant multicellular organism, or parts, organs, fruits or seeds thereof, comprising the chimeric PRR protein according to any one of claims 1 to 5, preferably wherein the organism is a plant.

10. A use of a product in the treatment and/or protection of a plant against injury, a pathogenic infection, such as a bacterial infection, a viral infection or fungal infection, wherein the product is selected from a chimeric PRR according to any one of claims 1 to 5, a nucleic acid according to claim 6, a vector according to claim 7, a recombinant cell according to claim 8, or a recombinant multicellular organism according to claim 9.

11. A method for increasing the resistance of a plant to a pathogen infection, comprising, introducing into said plant a chimeric PRR according to any one of claims 1 to 5, a nucleic acid according to claim 6, a vector according to claim 7, a recombinant cell according to claim 8, or a recombinant multicellular organism according to claim 9.

12. A method for producing a transgenic plant having enhanced resistance to a pathogen infection, comprising the step of introducing into a plant or into a plant cell of said plant a chimeric PRR according to any one of claims 1 to 5, a nucleic acid according to claim 6, a vector according to claim 7, a recombinant cell according to claim 8, or a recombinant multicellular organism according to claim 9.

13. A plant, **characterized in that** it expresses a chimeric PRR according to any one of claims 1 to 5.

14. A screening method for damage- and/or microbe-associated molecular patterns (DAMPS, MAMPs), comprising the method steps of (i) expressing in a plant or plant cell a chimeric PRR according to any one of claims 1 to 5, (ii) contacting said plant or plant cell with a candidate compound, (iii) measuring the immune response of said plant or plant cell in comparison with a control plant or plant cell, wherein an elevated immune response of said plant or plant cell indicates that said candidate compound is a DAMP and/or MAMP.

15. A method for sensitizing a plant against bacterial/viral/fungal/microbial infections or herbivorous attacks, the method comprising performing in said plant a method according to claim 12.

16. A method for producing a chimeric PRR for mediating defense reactions, the chimeric PRR comprising at least an ectodomain, a transmembrane domain, and a cytoplasmatic domain, the method comprising a step of directly or indirectly fusing together (i) an ectodomain derived from Leucine Rich Repeat (LRR) Receptor Like Protein (RLP), to (ii) a transmembrane domain and/or a cytoplasmatic domain derived from a membrane bound kinase, wherein prefereably the chimeric PRR is the chimeric PRR according to any one of claims 1 to 5.


SEQUENCE LISTING

<110>  Eberhard Karls Universität Tübingen

<120>  CHIMERIC PATTERN RECOGNITION RECEPTOR KINASES

<130>  U30862EP

<160>  22

<170>  PatentIn version 3.5

<210>  1
<211>  1031
<212>  PRT
<213>  Arabidopsis thaliana

<400>  1

Met Lys Leu Ser Phe Ser Leu Val Phe Asn Ala Leu Thr Leu Leu Leu
1               5                   10                  15

Gln Val Cys Ile Phe Ala Gln Ala Arg Phe Ser Asn Glu Thr Asp Met
            20                  25                  30

Gln Ala Leu Leu Glu Phe Lys Ser Gln Val Ser Glu Asn Asn Lys Arg
        35                  40                  45

Glu Val Leu Ala Ser Trp Asn His Ser Ser Pro Phe Cys Asn Trp Ile
    50                  55                  60

Gly Val Thr Cys Gly Arg Arg Arg Glu Arg Val Ile Ser Leu Asn Leu
65                  70                  75                  80

Gly Gly Phe Lys Leu Thr Gly Val Ile Ser Pro Ser Ile Gly Asn Leu
                85                  90                  95

Ser Phe Leu Arg Leu Leu Asn Leu Ala Asp Asn Ser Phe Gly Ser Thr
            100                 105                 110

Ile Pro Gln Lys Val Gly Arg Leu Phe Arg Leu Gln Tyr Leu Asn Met
        115                 120                 125

Ser Tyr Asn Leu Leu Glu Gly Arg Ile Pro Ser Ser Leu Ser Asn Cys
    130                 135                 140

Ser Arg Leu Ser Thr Val Asp Leu Ser Ser Asn His Leu Gly His Gly
145                 150                 155                 160

Val Pro Ser Glu Leu Gly Ser Leu Ser Lys Leu Ala Ile Leu Asp Leu
                165                 170                 175

Ser Lys Asn Asn Leu Thr Gly Asn Phe Pro Ala Ser Leu Gly Asn Leu
        180                 185                 190

Thr Ser Leu Gln Lys Leu Asp Phe Ala Tyr Asn Gln Met Arg Gly Glu
    195                 200                 205

Ile Pro Asp Glu Val Ala Arg Leu Thr Gln Met Val Phe Phe Gln Ile
    210                 215                 220

Ala Leu Asn Ser Phe Ser Gly Gly Phe Pro Pro Ala Leu Tyr Asn Ile
225                 230                 235                 240

Ser Ser Leu Glu Ser Leu Ser Leu Ala Asp Asn Ser Phe Ser Gly Asn
                245                 250                 255

Leu Arg Ala Asp Phe Gly Tyr Leu Leu Pro Asn Leu Arg Arg Leu Leu
            260                 265                 270

Leu Gly Thr Asn Gln Phe Thr Gly Ala Ile Pro Lys Thr Leu Ala Asn
        275                 280                 285

Ile Ser Ser Leu Glu Arg Phe Asp Ile Ser Ser Asn Tyr Leu Ser Gly
    290                 295                 300

Ser Ile Pro Leu Ser Phe Gly Lys Leu Arg Asn Leu Trp Trp Leu Gly
305                 310                 315                 320

Ile Arg Asn Asn Ser Leu Gly Asn Asn Ser Ser Ser Gly Leu Glu Phe
                325                 330                 335

Ile Gly Ala Val Ala Asn Cys Thr Gln Leu Glu Tyr Leu Asp Val Gly
            340                 345                 350

Tyr Asn Arg Leu Gly Gly Glu Leu Pro Ala Ser Ile Ala Asn Leu Ser
        355                 360                 365

Thr Thr Leu Thr Ser Leu Phe Leu Gly Gln Asn Leu Ile Ser Gly Thr
    370                 375                 380

Ile Pro His Asp Ile Gly Asn Leu Val Ser Leu Gln Glu Leu Ser Leu
385                 390                 395                 400

Glu Thr Asn Met Leu Ser Gly Glu Leu Pro Val Ser Phe Gly Lys Leu
                405                 410                 415

Leu Asn Leu Gln Val Val Asp Leu Tyr Ser Asn Ala Ile Ser Gly Glu
            420                 425                 430

Ile Pro Ser Tyr Phe Gly Asn Met Thr Arg Leu Gln Lys Leu His Leu
        435                 440                 445

Asn Ser Asn Ser Phe His Gly Arg Ile Pro Gln Ser Leu Gly Arg Cys
    450                 455                 460

Arg Tyr Leu Leu Asp Leu Trp Met Asp Thr Asn Arg Leu Asn Gly Thr
465                 470                 475                 480

Ile Pro Gln Glu Ile Leu Gln Ile Pro Ser Leu Ala Tyr Ile Asp Leu
                485                 490                 495

Ser Asn Asn Phe Leu Thr Gly His Phe Pro Glu Glu Val Gly Lys Leu
            500                 505                 510

Glu Leu Leu Val Gly Leu Gly Ala Ser Tyr Asn Lys Leu Ser Gly Lys
        515                 520                 525

Met Pro Gln Ala Ile Gly Gly Cys Leu Ser Met Glu Phe Leu Phe Met
    530                 535                 540

Gln Gly Asn Ser Phe Asp Gly Ala Ile Pro Asp Ile Ser Arg Leu Val
545                 550                 555                 560

Ser Leu Lys Asn Val Asp Phe Ser Asn Asn Asn Leu Ser Gly Arg Ile
                565                 570                 575

Pro Arg Tyr Leu Ala Ser Leu Pro Ser Leu Arg Asn Leu Asn Leu Ser
            580                 585                 590

Met Asn Lys Phe Glu Gly Arg Val Pro Thr Thr Gly Val Phe Arg Asn
        595                 600                 605

Ala Thr Ala Val Ser Val Phe Gly Asn Thr Asn Ile Cys Gly Gly Val
    610                 615                 620

Arg Glu Met Gln Leu Lys Pro Cys Ile Val Gln Ala Ser Pro Arg Lys
625                 630                 635                 640

Arg Lys Pro Leu Ser Val Arg Lys Lys Val Val Ser Gly Ile Cys Ile
                645                 650                 655

Gly Ile Ala Ser Leu Leu Leu Ile Ile Ile Val Ala Ser Leu Cys Trp
            660                 665                 670

Phe Met Lys Arg Lys Lys Lys Asn Asn Ala Ser Asp Gly Asn Pro Ser
        675                 680                 685

Asp Ser Thr Thr Leu Gly Met Phe His Glu Lys Val Ser Tyr Glu Glu
    690                 695                 700

Leu His Ser Ala Thr Ser Arg Phe Ser Ser Thr Asn Leu Ile Gly Ser
705                 710                 715                 720

Gly Asn Phe Gly Asn Val Phe Lys Gly Leu Leu Gly Pro Glu Asn Lys
                725                 730                 735

Leu Val Ala Val Lys Val Leu Asn Leu Leu Lys His Gly Ala Thr Lys
            740                 745                 750

Ser Phe Met Ala Glu Cys Glu Thr Phe Lys Gly Ile Arg His Arg Asn
        755                 760                 765

Leu Val Lys Leu Ile Thr Val Cys Ser Ser Leu Asp Ser Glu Gly Asn
    770                 775                 780

Asp Phe Arg Ala Leu Val Tyr Glu Phe Met Pro Lys Gly Ser Leu Asp
785                 790                 795                 800

Met Trp Leu Gln Leu Glu Asp Leu Glu Arg Val Asn Asp His Ser Arg
                805                 810                 815

Ser Leu Thr Pro Ala Glu Lys Leu Asn Ile Ala Ile Asp Val Ala Ser
            820                 825                 830

Ala Leu Glu Tyr Leu His Val His Cys His Asp Pro Val Ala His Cys
        835                 840                 845

Asp Ile Lys Pro Ser Asn Ile Leu Leu Asp Asp Asp Leu Thr Ala His
    850                 855                 860

Val Ser Asp Phe Gly Leu Ala Gln Leu Leu Tyr Lys Tyr Asp Arg Glu
865                 870                 875                 880

Ser Phe Leu Asn Gln Phe Ser Ser Ala Gly Val Arg Gly Thr Ile Gly
                885                 890                 895

Tyr Ala Ala Pro Glu Tyr Gly Met Gly Gly Gln Pro Ser Ile Gln Gly
            900                 905                 910

Asp Val Tyr Ser Phe Gly Ile Leu Leu Leu Glu Met Phe Ser Gly Lys
        915                 920                 925

Lys Pro Thr Asp Glu Ser Phe Ala Gly Asp Tyr Asn Leu His Ser Tyr

930                 935                 940

Thr Lys Ser Ile Leu Ser Gly Cys Thr Ser Ser Gly Gly Ser Asn Ala
945                 950                 955                 960

Ile Asp Glu Gly Leu Arg Leu Val Leu Gln Val Gly Ile Lys Cys Ser
                965                 970                 975

Glu Glu Tyr Pro Arg Asp Arg Met Arg Thr Asp Glu Ala Val Arg Glu
            980                 985                 990

Leu Ile Ser Ile Arg Ser Lys Phe  Phe Ser Ser Lys Thr  Thr Ile Thr
        995                 1000                1005

Glu Ser  Pro Arg Asp Ala Pro  Gln Ser Ser Pro Gln  Glu Trp Met
    1010                1015                1020

Leu Asn  Thr Asp Met His Thr  Met
    1025                1030

<210>  2
<211>  890
<212>  PRT
<213>  Arabidopsis thaliana

<400>  2

Met Ser Lys Ala Leu Leu His Leu His Phe Leu Ser Leu Phe Leu Leu
1               5                   10                  15

Cys Cys Val Cys His Ser Ser Ile Phe Thr Leu Asn Phe His Phe Thr
            20                  25                  30

Gly Ile Val Ala Cys Arg Pro His Gln Ile Gln Ala Phe Thr Lys Phe
        35                  40                  45

Thr Asn Glu Phe Asp Thr Arg Gly Cys Asn Asn Ser Asp Thr Phe Asn
    50                  55                  60

Gly Val Trp Cys Asp Asn Ser Thr Gly Ala Val Ala Val Leu Gln Leu
65                  70                  75                  80

Arg Lys Cys Leu Ser Gly Thr Leu Lys Ser Asn Ser Ser Leu Phe Gly
                85                  90                  95

Phe His Gln Leu Arg Tyr Val Asp Leu Gln Asn Asn Asn Leu Thr Ser
            100                 105                 110

Ser Ser Leu Pro Ser Gly Phe Gly Asn Leu Lys Arg Leu Glu Gly Leu

115                 120                 125

Phe Leu Ser Ser Asn Gly Phe Leu Gly Gln Val Pro Ser Ser Phe Ser
    130                 135                 140

Asn Leu Thr Met Leu Ala Gln Leu Asp Leu Ser Tyr Asn Lys Leu Thr
145                 150                 155                 160

Gly Ser Phe Pro Leu Val Arg Gly Leu Arg Lys Leu Ile Val Leu Asp
                165                 170                 175

Leu Ser Tyr Asn His Phe Ser Gly Thr Leu Asn Pro Asn Ser Ser Leu
            180                 185                 190

Phe Glu Leu His Gln Leu Arg Tyr Leu Asn Leu Ala Phe Asn Asn Phe
        195                 200                 205

Ser Ser Ser Leu Pro Ser Lys Phe Gly Asn Leu His Arg Leu Glu Asn
    210                 215                 220

Leu Ile Leu Ser Ser Asn Gly Phe Ser Gly Gln Val Pro Ser Thr Ile
225                 230                 235                 240

Ser Asn Leu Thr Arg Leu Thr Lys Leu Tyr Leu Asp Gln Asn Lys Leu
                245                 250                 255

Thr Ser Ser Phe Pro Leu Val Gln Asn Leu Thr Asn Leu Tyr Glu Leu
            260                 265                 270

Asp Leu Ser Tyr Asn Lys Phe Phe Gly Val Ile Pro Ser Ser Leu Leu
        275                 280                 285

Thr Leu Pro Phe Leu Ala His Leu Ala Leu Arg Glu Asn Asn Leu Ala
    290                 295                 300

Gly Ser Val Glu Val Ser Asn Ser Ser Thr Ser Ser Arg Leu Glu Ile
305                 310                 315                 320

Met Tyr Leu Gly Ser Asn His Phe Glu Gly Gln Ile Leu Glu Pro Ile
                325                 330                 335

Ser Lys Leu Ile Asn Leu Lys His Leu Asp Leu Ser Phe Leu Asn Thr
            340                 345                 350

Ser Tyr Pro Ile Asp Leu Lys Leu Phe Ser Ser Leu Lys Ser Leu Arg
        355                 360                 365

Ser Leu Asp Leu Ser Gly Asn Ser Ile Ser Ser Ala Ser Leu Ser Ser
    370                 375                 380

Asp Ser Tyr Ile Pro Leu Thr Leu Glu Met Leu Thr Leu Arg His Cys
385                 390                 395                 400

Asp Ile Asn Glu Phe Pro Asn Ile Leu Lys Thr Leu Lys Glu Leu Val
                405                 410                 415

Tyr Ile Asp Ile Ser Asn Asn Arg Met Lys Gly Lys Ile Pro Glu Trp
            420                 425                 430

Leu Trp Ser Leu Pro Leu Leu Gln Ser Val Thr Leu Gly Asn Asn Tyr
        435                 440                 445

Phe Thr Gly Phe Gln Gly Ser Ala Glu Ile Leu Val Asn Ser Ser Val
    450                 455                 460

Leu Leu Leu Tyr Leu Asp Ser Asn Asn Phe Glu Gly Ala Leu Pro Asp
465                 470                 475                 480

Leu Pro Leu Ser Ile Lys Gly Phe Gly Val Ala Ser Asn Ser Phe Thr
                485                 490                 495

Ser Glu Ile Pro Leu Ser Ile Cys Asn Arg Ser Ser Leu Ala Ala Ile
            500                 505                 510

Asp Leu Ser Tyr Asn Asn Phe Thr Gly Pro Ile Pro Pro Cys Leu Arg
        515                 520                 525

Asn Leu Glu Leu Val Tyr Leu Arg Asn Asn Asn Leu Glu Gly Ser Ile
    530                 535                 540

Pro Asp Ala Leu Cys Asp Gly Ala Ser Leu Arg Thr Leu Asp Val Ser
545                 550                 555                 560

His Asn Arg Leu Thr Gly Lys Leu Pro Arg Ser Phe Val Asn Cys Ser
                565                 570                 575

Ser Leu Lys Phe Leu Ser Val Ile Asn Asn Arg Ile Glu Asp Thr Phe
            580                 585                 590

Pro Phe Trp Leu Lys Ala Leu Pro Asn Leu Gln Val Leu Thr Leu Arg
        595                 600                 605

Ser Asn Arg Phe Tyr Gly Pro Ile Ser Pro Pro His Gln Gly Pro Leu
    610                 615                 620

Gly Phe Pro Glu Leu Arg Ile Phe Glu Ile Ser Asp Asn Lys Phe Thr
625                 630                 635                 640

Gly Ser Leu Pro Pro Asn Tyr Phe Val Asn Trp Lys Ala Ser Ser Arg
                645                 650                 655

Thr Met Asn Gln Asp Gly Gly Leu Tyr Met Val Tyr Glu Glu Lys Leu
            660                 665                 670

Phe Asp Glu Gly Gly Tyr Gly Tyr Thr Asp Ala Leu Asp Leu Gln Tyr
        675                 680                 685

Lys Gly Leu His Met Glu Gln Ala Lys Ala Leu Thr Ser Tyr Ala Ala
    690                 695                 700

Ile Asp Phe Ser Gly Asn Arg Leu Glu Gly Gln Ile Pro Glu Ser Ile
705                 710                 715                 720

Gly Leu Leu Lys Ala Leu Ile Ala Val Asn Ile Ser Asn Asn Ala Phe
                725                 730                 735

Thr Gly His Ile Pro Leu Ser Met Ala Asn Leu Glu Asn Leu Glu Ser
            740                 745                 750

Leu Asp Met Ser Arg Asn Gln Leu Ser Gly Thr Ile Pro Asn Gly Leu
        755                 760                 765

Gly Ser Ile Ser Phe Leu Ala Tyr Ile Asn Val Ser His Asn Gln Leu
    770                 775                 780

Thr Gly Glu Ile Pro Gln Gly Thr Gln Ile Thr Gly Gln Ser Lys Ser
785                 790                 795                 800

Ser Phe Glu Gly Asn Ala Gly Leu Cys Gly Leu Pro Leu Lys Glu Ser
                805                 810                 815

Cys Phe Gly Thr Gly Ala Pro Pro Met Tyr His Gln Lys Gln Glu Asp
            820                 825                 830

Lys Glu Glu Glu Glu Glu Glu Glu Glu Glu Glu Glu Glu Val Leu Asn
        835                 840                 845

Gly Arg Ala Val Ala Ile Gly Tyr Gly Ser Gly Leu Leu Leu Gly Leu
    850                 855                 860

Ala Ile Ala Gln Val Ile Ala Ser Tyr Lys Pro Glu Trp Leu Val Lys
865                 870                 875                 880

Ile Ile Gly Leu Asn Lys Arg Arg Lys Arg
                885                 890

<210>  3
<211>  641
<212>  PRT
<213>  Arabidopsis thaliana

<400>  3

Met Ala Val Pro Thr Gly Ser Ala Asn Leu Phe Leu Arg Pro Leu Ile
1               5                   10                  15

Leu Ala Val Leu Ser Phe Leu Leu Leu Ser Ser Phe Val Ser Ser Val
            20                  25                  30

Glu Trp Leu Asp Ile Asp Ser Ser Asp Leu Lys Ala Leu Gln Val Ile
        35                  40                  45

Glu Thr Glu Leu Gly Val Asn Ser Gln Arg Ser Ser Ala Ser Asp Val
    50                  55                  60

Asn Pro Cys Gly Arg Arg Gly Val Phe Cys Glu Arg Arg His Ser Ala
65                  70                  75                  80

Thr Thr Gly Glu Tyr Val Leu Arg Val Thr Arg Leu Val Tyr Arg Ser
                85                  90                  95

Arg Ser Leu Thr Gly Thr Ile Ser Pro Val Ile Gly Met Leu Ser Glu
            100                 105                 110

Leu Lys Glu Leu Thr Leu Ser Asn Asn Gln Leu Val Asn Ala Val Pro
        115                 120                 125

Val Asp Ile Leu Ser Cys Lys Gln Leu Glu Val Leu Asp Leu Arg Lys
    130                 135                 140

Asn Arg Phe Ser Gly Gln Ile Pro Gly Asn Phe Ser Ser Leu Ser Arg
145                 150                 155                 160

Leu Arg Ile Leu Asp Leu Ser Ser Asn Lys Leu Ser Gly Asn Leu Asn
                165                 170                 175

Phe Leu Lys Asn Leu Arg Asn Leu Glu Asn Leu Ser Val Ala Asn Asn
            180                 185                 190

Leu Phe Ser Gly Lys Ile Pro Glu Gln Ile Val Ser Phe His Asn Leu
        195                 200                 205

Arg Phe Phe Asp Phe Ser Gly Asn Arg Tyr Leu Glu Gly Pro Ala Pro
    210                 215                 220

Val Met Ser Ser Ile Lys Leu Gln Thr Ser Pro His Gln Thr Arg His
225                 230                 235                 240

Ile Leu Ala Glu Thr Pro Thr Ser Ser Pro Thr Asn Lys Pro Asn Asn
                245                 250                 255

Ser Thr Thr Ser Lys Ala Pro Lys Gly Ala Pro Lys Pro Gly Lys Leu
            260                 265                 270

Lys Lys Lys Lys Lys Lys Ser Lys Lys Lys Lys Val Ala Ala Trp Ile
        275                 280                 285

Leu Gly Phe Val Val Gly Ala Ile Gly Gly Thr Ile Ser Gly Phe Val
    290                 295                 300

Phe Ser Val Leu Phe Lys Leu Ile Ile Gln Ala Ile Arg Gly Ser Glu
305                 310                 315                 320

Lys Pro Pro Gly Pro Ser Ile Phe Ser Pro Leu Ile Lys Lys Ala Glu
                325                 330                 335

Asp Leu Ala Phe Leu Glu Asn Glu Glu Ala Leu Ala Ser Leu Glu Ile
            340                 345                 350

Ile Gly Arg Gly Gly Cys Gly Glu Val Phe Lys Ala Glu Leu Pro Gly
        355                 360                 365

Ser Asn Gly Lys Ile Ile Ala Val Lys Lys Val Ile Gln Pro Pro Lys
    370                 375                 380

Asp Ala Asp Glu Leu Thr Asp Glu Asp Ser Lys Phe Leu Asn Lys Lys
385                 390                 395                 400

Met Arg Gln Ile Arg Ser Glu Ile Asn Thr Val Gly His Ile Arg His
                405                 410                 415

Arg Asn Leu Leu Pro Leu Leu Ala His Val Ser Arg Pro Glu Cys His
            420                 425                 430

Tyr Leu Val Tyr Glu Tyr Met Glu Lys Gly Ser Leu Gln Asp Ile Leu
        435                 440                 445

Thr Asp Val Gln Ala Gly Asn Gln Glu Leu Met Trp Pro Ala Arg His

450                 455                 460

Lys Ile Ala Leu Gly Ile Ala Ala Gly Leu Glu Tyr Leu His Met Asp
465                 470                 475                 480

His Asn Pro Arg Ile Ile His Arg Asp Leu Lys Pro Ala Asn Val Leu
                485                 490                 495

Leu Asp Asp Asp Met Glu Ala Arg Ile Ser Asp Phe Gly Leu Ala Lys
            500                 505                 510

Ala Met Pro Asp Ala Val Thr His Ile Thr Thr Ser His Val Ala Gly
        515                 520                 525

Thr Val Gly Tyr Ile Ala Pro Glu Phe Tyr Gln Thr His Lys Phe Thr
    530                 535                 540

Asp Lys Cys Asp Ile Tyr Ser Phe Gly Val Ile Leu Gly Ile Leu Val
545                 550                 555                 560

Ile Gly Lys Leu Pro Ser Asp Glu Phe Phe Gln His Thr Asp Glu Met
                565                 570                 575

Ser Leu Ile Lys Trp Met Arg Asn Ile Ile Thr Ser Glu Asn Pro Ser
            580                 585                 590

Leu Ala Ile Asp Pro Lys Leu Met Asp Gln Gly Phe Asp Glu Gln Met
        595                 600                 605

Leu Leu Val Leu Lys Ile Ala Cys Tyr Cys Thr Leu Asp Asp Pro Lys
    610                 615                 620

Gln Arg Pro Asn Ser Lys Asp Val Arg Thr Met Leu Ser Gln Ile Lys
625                 630                 635                 640

His

<210>  4
<211>  1021
<212>  PRT
<213>  Arabidopsis thaliana

<400>  4

Met Gly Lys Arg Thr Asn Pro Arg His Phe Leu Val Thr Trp Ser Leu
1               5                   10                  15

Leu Leu Leu Glu Thr Ala Phe Gly Leu Thr Ser Arg Glu Val Asn Lys

20                  25                  30

Thr Leu Cys Ile Glu Lys Glu Arg Gly Ala Leu Leu Glu Phe Lys Arg
        35                  40                  45

Gly Leu Asn Asp Asp Phe Gly Arg Leu Ser Thr Trp Gly Asp Glu Glu
    50                  55                  60

Glu Cys Cys Asn Trp Lys Gly Ile Glu Cys Asp Lys Arg Thr Gly His
65                  70                  75                  80

Val Ile Val Leu Asp Leu His Ser Glu Val Thr Cys Pro Gly His Ala
                85                  90                  95

Cys Phe Ala Pro Ile Leu Thr Gly Lys Val Ser Pro Ser Leu Leu Glu
            100                 105                 110

Leu Glu Tyr Leu Asn Phe Leu Asp Leu Ser Val Asn Gly Phe Glu Asn
        115                 120                 125

Ser Glu Ile Pro Arg Phe Ile Gly Ser Leu Lys Arg Leu Glu Tyr Leu
    130                 135                 140

Asn Leu Ser Ser Ser Asp Phe Ser Gly Glu Ile Pro Ala Gln Phe Gln
145                 150                 155                 160

Asn Leu Thr Ser Leu Arg Ile Leu Asp Leu Gly Asn Asn Asn Leu Ile
                165                 170                 175

Val Lys Asp Leu Val Trp Leu Ser His Leu Ser Ser Leu Glu Phe Leu
            180                 185                 190

Arg Leu Gly Gly Asn Asp Phe Gln Ala Arg Asn Trp Phe Arg Glu Ile
        195                 200                 205

Thr Lys Val Pro Ser Leu Lys Glu Leu Asp Leu Ser Val Cys Gly Leu
    210                 215                 220

Ser Lys Phe Val Pro Ser Pro Ala Asp Val Ala Asn Ser Ser Leu Ile
225                 230                 235                 240

Ser Leu Ser Val Leu His Leu Cys Cys Asn Glu Phe Ser Thr Ser Ser
                245                 250                 255

Glu Tyr Ser Trp Leu Phe Asn Phe Ser Thr Ser Leu Thr Ser Ile Asp
            260                 265                 270

Leu Ser His Asn Gln Leu Ser Arg Gln Ile Asp Asp Arg Phe Gly Ser
        275                 280                 285

Leu Met Tyr Leu Glu His Leu Asn Leu Ala Asn Asn Phe Gly Ala Glu
    290                 295                 300

Gly Gly Val Pro Ser Ser Phe Gly Asn Leu Thr Arg Leu His Tyr Leu
305                 310                 315                 320

Asp Met Ser Asn Thr Gln Thr Tyr Gln Trp Leu Pro Glu Leu Phe Leu
                325                 330                 335

Arg Leu Ser Gly Ser Arg Lys Ser Leu Glu Val Leu Gly Leu Asn Asp
            340                 345                 350

Asn Ser Leu Phe Gly Ser Ile Val Asn Val Pro Arg Phe Ser Ser Leu
        355                 360                 365

Lys Lys Leu Tyr Leu Gln Lys Asn Met Leu Asn Gly Phe Phe Met Glu
    370                 375                 380

Arg Val Gly Gln Val Ser Ser Leu Glu Tyr Leu Asp Leu Ser Asp Asn
385                 390                 395                 400

Gln Met Arg Gly Pro Leu Pro Asp Leu Ala Leu Phe Pro Ser Leu Arg
                405                 410                 415

Glu Leu His Leu Gly Ser Asn Gln Phe Gln Gly Arg Ile Pro Gln Gly
            420                 425                 430

Ile Gly Lys Leu Ser Gln Leu Arg Ile Phe Asp Val Ser Ser Asn Arg
        435                 440                 445

Leu Glu Gly Leu Pro Glu Ser Met Gly Gln Leu Ser Asn Leu Glu Arg
    450                 455                 460

Phe Asp Ala Ser Tyr Asn Val Leu Lys Gly Thr Ile Thr Glu Ser His
465                 470                 475                 480

Phe Ser Asn Leu Ser Ser Leu Val Asp Leu Asp Leu Ser Phe Asn Leu
                485                 490                 495

Leu Ser Leu Asn Thr Arg Phe Asp Trp Val Pro Pro Phe Gln Leu Gln
            500                 505                 510

Phe Ile Arg Leu Pro Ser Cys Asn Met Gly Pro Ser Phe Pro Lys Trp
        515                 520                 525

Leu Gln Thr Gln Asn Asn Tyr Thr Leu Leu Asp Ile Ser Leu Ala Asn
    530                 535                 540

Ile Ser Asp Met Leu Pro Ser Trp Phe Ser Asn Leu Pro Pro Glu Leu
545                 550                 555                 560

Lys Ile Leu Asn Leu Ser Asn Asn His Ile Ser Gly Arg Val Ser Glu
                565                 570                 575

Phe Ile Val Ser Lys Gln Asp Tyr Met Ile Ile Asp Leu Ser Ser Asn
            580                 585                 590

Asn Phe Ser Gly His Leu Pro Leu Val Pro Ala Asn Ile Gln Ile Phe
        595                 600                 605

Tyr Leu His Lys Asn His Phe Ser Gly Ser Ile Ser Ser Ile Cys Arg
    610                 615                 620

Asn Thr Ile Gly Ala Ala Thr Ser Ile Asp Leu Ser Arg Asn Gln Phe
625                 630                 635                 640

Ser Gly Glu Val Pro Asp Cys Trp Met Asn Met Ser Asn Leu Ala Val
                645                 650                 655

Leu Asn Leu Ala Tyr Asn Asn Phe Ser Gly Lys Val Pro Gln Ser Leu
            660                 665                 670

Gly Ser Leu Thr Asn Leu Glu Ala Leu Tyr Ile Arg Gln Asn Ser Phe
        675                 680                 685

Arg Gly Met Leu Pro Ser Phe Ser Gln Cys Gln Leu Leu Gln Ile Leu
    690                 695                 700

Asp Ile Gly Gly Asn Lys Leu Thr Gly Arg Ile Pro Ala Trp Ile Gly
705                 710                 715                 720

Thr Asp Leu Leu Gln Leu Arg Ile Leu Ser Leu Arg Ser Asn Lys Phe
                725                 730                 735

Asp Gly Ser Ile Pro Ser Leu Ile Cys Gln Leu Gln Phe Leu Gln Ile
            740                 745                 750

Leu Asp Leu Ser Glu Asn Gly Leu Ser Gly Lys Ile Pro Gln Cys Leu
        755                 760                 765

Asn Asn Phe Thr Ile Leu Arg Gln Glu Asn Gly Ser Gly Glu Ser Met
    770                 775                 780

Asp Phe Lys Val Arg Tyr Asp Tyr Ile Pro Gly Ser Tyr Leu Tyr Ile
785                 790                 795                 800

Gly Asp Leu Leu Ile Gln Trp Lys Asn Gln Glu Ser Glu Tyr Lys Asn
                805                 810                 815

Ala Leu Leu Tyr Leu Lys Ile Ile Asp Leu Ser Ser Asn Lys Leu Val
            820                 825                 830

Gly Gly Ile Pro Lys Glu Ile Ala Glu Met Arg Gly Leu Arg Ser Leu
        835                 840                 845

Asn Leu Ser Arg Asn Asp Leu Asn Gly Thr Val Val Glu Gly Ile Gly
    850                 855                 860

Gln Met Lys Leu Leu Glu Ser Leu Asp Leu Ser Arg Asn Gln Leu Ser
865                 870                 875                 880

Gly Met Ile Pro Gln Gly Leu Ser Asn Leu Thr Phe Leu Ser Val Leu
                885                 890                 895

Asp Leu Ser Asn Asn His Leu Ser Gly Arg Ile Pro Ser Ser Thr Gln
            900                 905                 910

Leu Gln Ser Phe Asp Arg Ser Ser Tyr Ser Gly Asn Ala Gln Leu Cys
        915                 920                 925

Gly Pro Pro Leu Glu Glu Cys Pro Gly Tyr Ala Pro Pro Ile Asp Arg
    930                 935                 940

Gly Ser Asn Thr Asn Pro Gln Glu His Asp Asp Asp Asp Glu Phe Ser
945                 950                 955                 960

Ser Leu Glu Phe Tyr Val Ser Met Val Leu Gly Phe Phe Val Thr Phe
                965                 970                 975

Trp Gly Ile Leu Gly Cys Leu Ile Val Asn Arg Ser Trp Arg Asn Ala
            980                 985                 990

Tyr Phe Thr Phe Leu Thr Asp Met  Lys Ser Trp Leu His  Met Thr Ser
        995                 1000                1005

Arg Val  Cys Phe Ala Arg Leu  Lys Gly Lys Leu Arg  Asn
    1010                1015                1020

<210>  5

<211>  888
<212>  PRT
<213>  Artificial

<220>
<223>  RLP23tmSOBIR1

<400>  5

Met Ser Lys Ala Leu Leu His Leu His Phe Leu Ser Leu Phe Leu Leu
1               5                   10                  15

Cys Cys Val Cys His Ser Ser Ile Phe Thr Leu Asn Phe His Phe Thr
            20                  25                  30

Gly Ile Val Ala Cys Arg Pro His Gln Ile Gln Ala Phe Thr Lys Phe
        35                  40                  45

Thr Asn Glu Phe Asp Thr Arg Gly Cys Asn Asn Ser Asp Thr Phe Asn
    50                  55                  60

Gly Val Trp Cys Asp Asn Ser Thr Gly Ala Val Ala Val Leu Gln Leu
65                  70                  75                  80

Arg Lys Cys Leu Ser Gly Thr Leu Lys Ser Asn Ser Ser Leu Phe Gly
                85                  90                  95

Phe His Gln Leu Arg Tyr Val Asp Leu Gln Asn Asn Asn Leu Thr Ser
            100                 105                 110

Ser Ser Leu Pro Ser Gly Phe Gly Asn Leu Lys Arg Leu Glu Gly Leu
        115                 120                 125

Phe Leu Ser Ser Asn Gly Phe Leu Gly Gln Val Pro Ser Ser Phe Ser
    130                 135                 140

Asn Leu Thr Met Leu Ala Gln Leu Asp Leu Ser Tyr Asn Lys Leu Thr
145                 150                 155                 160

Gly Ser Phe Pro Leu Val Arg Gly Leu Arg Lys Leu Ile Val Leu Asp
                165                 170                 175

Leu Ser Tyr Asn His Phe Ser Gly Thr Leu Asn Pro Asn Ser Ser Leu
            180                 185                 190

Phe Glu Leu His Gln Leu Arg Tyr Leu Asn Leu Ala Phe Asn Asn Phe
        195                 200                 205

Ser Ser Ser Leu Pro Ser Lys Phe Gly Asn Leu His Arg Leu Glu Asn
    210                 215                 220

Leu Ile Leu Ser Ser Asn Gly Phe Ser Gly Gln Val Pro Ser Thr Ile
225                 230                 235                 240

Ser Asn Leu Thr Arg Leu Thr Lys Leu Tyr Leu Asp Gln Asn Lys Leu
                245                 250                 255

Thr Ser Ser Phe Pro Leu Val Gln Asn Leu Thr Asn Leu Tyr Glu Leu
            260                 265                 270

Asp Leu Ser Tyr Asn Lys Phe Phe Gly Val Ile Pro Ser Ser Leu Leu
        275                 280                 285

Thr Leu Pro Phe Leu Ala His Leu Ala Leu Arg Glu Asn Asn Leu Ala
    290                 295                 300

Gly Ser Val Glu Val Ser Asn Ser Ser Thr Ser Ser Arg Leu Glu Ile
305                 310                 315                 320

Met Tyr Leu Gly Ser Asn His Phe Glu Gly Gln Ile Leu Glu Pro Ile
                325                 330                 335

Ser Lys Leu Ile Asn Leu Lys His Leu Asp Leu Ser Phe Leu Asn Thr
            340                 345                 350

Ser Tyr Pro Ile Asp Leu Lys Leu Phe Ser Ser Leu Lys Ser Leu Arg
        355                 360                 365

Ser Leu Asp Leu Ser Gly Asn Ser Ile Ser Ser Ala Ser Leu Ser Ser
    370                 375                 380

Asp Ser Tyr Ile Pro Leu Thr Leu Glu Met Leu Thr Leu Arg His Cys
385                 390                 395                 400

Asp Ile Asn Glu Phe Pro Asn Ile Leu Lys Thr Leu Lys Glu Leu Val
                405                 410                 415

Tyr Ile Asp Ile Ser Asn Asn Arg Met Lys Gly Lys Ile Pro Glu Trp
            420                 425                 430

Leu Trp Ser Leu Pro Leu Leu Gln Ser Val Thr Leu Gly Asn Asn Tyr
        435                 440                 445

Phe Thr Gly Phe Gln Gly Ser Ala Glu Ile Leu Val Asn Ser Ser Val
    450                 455                 460

Leu Leu Leu Tyr Leu Asp Ser Asn Asn Phe Glu Gly Ala Leu Pro Asp

465                 470                 475                 480

Leu Pro Leu Ser Ile Lys Gly Phe Gly Val Ala Ser Asn Ser Phe Thr
                485                 490                 495

Ser Glu Ile Pro Leu Ser Ile Cys Asn Arg Ser Ser Leu Ala Ala Ile
            500                 505                 510

Asp Leu Ser Tyr Asn Asn Phe Thr Gly Pro Ile Pro Pro Cys Leu Arg
        515                 520                 525

Asn Leu Glu Leu Val Tyr Leu Arg Asn Asn Asn Leu Glu Gly Ser Ile
    530                 535                 540

Pro Asp Ala Leu Cys Asp Gly Ala Ser Leu Arg Thr Leu Asp Val Ser
545                 550                 555                 560

His Asn Arg Leu Thr Gly Lys Leu Pro Arg Ser Phe Val Asn Cys Ser
                565                 570                 575

Ser Leu Lys Phe Leu Ser Val Ile Asn Asn Arg Ile Glu Asp Thr Phe
            580                 585                 590

Pro Phe Trp Leu Lys Ala Leu Pro Asn Leu Gln Val Leu Thr Leu Arg
        595                 600                 605

Ser Asn Arg Phe Tyr Gly Pro Ile Ser Pro Pro His Gln Gly Pro Leu
    610                 615                 620

Gly Phe Pro Glu Leu Arg Ile Phe Glu Ile Ser Asp Asn Lys Phe Thr
625                 630                 635                 640

Gly Ser Leu Pro Pro Asn Tyr Phe Val Asn Trp Lys Ala Ser Ser Arg
                645                 650                 655

Thr Met Asn Gln Asp Gly Gly Leu Tyr Met Val Tyr Glu Glu Lys Leu
            660                 665                 670

Phe Asp Glu Gly Gly Tyr Gly Tyr Thr Asp Ala Leu Asp Leu Gln Tyr
        675                 680                 685

Lys Gly Leu His Met Glu Gln Ala Lys Ala Leu Thr Ser Tyr Ala Ala
    690                 695                 700

Ile Asp Phe Ser Gly Asn Arg Leu Glu Gly Gln Ile Pro Glu Ser Ile
705                 710                 715                 720

Gly Leu Leu Lys Ala Leu Ile Ala Val Asn Ile Ser Asn Asn Ala Phe
                725                 730                 735

Thr Gly His Ile Pro Leu Ser Met Ala Asn Leu Glu Asn Leu Glu Ser
            740                 745                 750

Leu Asp Met Ser Arg Asn Gln Leu Ser Gly Thr Ile Pro Asn Gly Leu
        755                 760                 765

Gly Ser Ile Ser Phe Leu Ala Tyr Ile Asn Val Ser His Asn Gln Leu
    770                 775                 780

Thr Gly Glu Ile Pro Gln Gly Thr Gln Ile Thr Gly Gln Ser Lys Ser
785                 790                 795                 800

Ser Phe Glu Gly Asn Ala Gly Leu Cys Gly Leu Pro Leu Lys Glu Ser
                805                 810                 815

Cys Phe Gly Thr Gly Ala Pro Pro Met Tyr His Gln Lys Gln Glu Asp
            820                 825                 830

Lys Glu Glu Glu Glu Glu Glu Glu Glu Glu Glu Glu Glu Val Ala Ala
        835                 840                 845

Trp Ile Leu Gly Phe Val Val Gly Ala Ile Gly Gly Thr Ile Ser Gly
    850                 855                 860

Phe Val Phe Ser Val Leu Phe Lys Pro Glu Trp Leu Val Lys Ile Ile
865                 870                 875                 880

Gly Leu Asn Lys Arg Arg Lys Arg
                885

<210>  6
<211>  1203
<212>  PRT
<213>  Artificial

<220>
<223>  RLP23tmkSOBIR1

<400>  6

Met Ser Lys Ala Leu Leu His Leu His Phe Leu Ser Leu Phe Leu Leu
1               5                   10                  15

Cys Cys Val Cys His Ser Ser Ile Phe Thr Leu Asn Phe His Phe Thr
            20                  25                  30

Gly Ile Val Ala Cys Arg Pro His Gln Ile Gln Ala Phe Thr Lys Phe

        35                  40                  45

Thr Asn Glu Phe Asp Thr Arg Gly Cys Asn Asn Ser Asp Thr Phe Asn
    50                  55                  60

Gly Val Trp Cys Asp Asn Ser Thr Gly Ala Val Ala Val Leu Gln Leu
65                  70                  75                  80

Arg Lys Cys Leu Ser Gly Thr Leu Lys Ser Asn Ser Ser Leu Phe Gly
                85                  90                  95

Phe His Gln Leu Arg Tyr Val Asp Leu Gln Asn Asn Asn Leu Thr Ser
            100                 105                 110

Ser Ser Leu Pro Ser Gly Phe Gly Asn Leu Lys Arg Leu Glu Gly Leu
        115                 120                 125

Phe Leu Ser Ser Asn Gly Phe Leu Gly Gln Val Pro Ser Ser Phe Ser
    130                 135                 140

Asn Leu Thr Met Leu Ala Gln Leu Asp Leu Ser Tyr Asn Lys Leu Thr
145                 150                 155                 160

Gly Ser Phe Pro Leu Val Arg Gly Leu Arg Lys Leu Ile Val Leu Asp
                165                 170                 175

Leu Ser Tyr Asn His Phe Ser Gly Thr Leu Asn Pro Asn Ser Ser Leu
            180                 185                 190

Phe Glu Leu His Gln Leu Arg Tyr Leu Asn Leu Ala Phe Asn Asn Phe
        195                 200                 205

Ser Ser Ser Leu Pro Ser Lys Phe Gly Asn Leu His Arg Leu Glu Asn
    210                 215                 220

Leu Ile Leu Ser Ser Asn Gly Phe Ser Gly Gln Val Pro Ser Thr Ile
225                 230                 235                 240

Ser Asn Leu Thr Arg Leu Thr Lys Leu Tyr Leu Asp Gln Asn Lys Leu
                245                 250                 255

Thr Ser Ser Phe Pro Leu Val Gln Asn Leu Thr Asn Leu Tyr Glu Leu
            260                 265                 270

Asp Leu Ser Tyr Asn Lys Phe Phe Gly Val Ile Pro Ser Ser Leu Leu
        275                 280                 285

Thr Leu Pro Phe Leu Ala His Leu Ala Leu Arg Glu Asn Asn Leu Ala
    290                 295                 300

Gly Ser Val Glu Val Ser Asn Ser Ser Thr Ser Ser Arg Leu Glu Ile
305                 310                 315                 320

Met Tyr Leu Gly Ser Asn His Phe Glu Gly Gln Ile Leu Glu Pro Ile
                325                 330                 335

Ser Lys Leu Ile Asn Leu Lys His Leu Asp Leu Ser Phe Leu Asn Thr
            340                 345                 350

Ser Tyr Pro Ile Asp Leu Lys Leu Phe Ser Ser Leu Lys Ser Leu Arg
        355                 360                 365

Ser Leu Asp Leu Ser Gly Asn Ser Ile Ser Ser Ala Ser Leu Ser Ser
    370                 375                 380

Asp Ser Tyr Ile Pro Leu Thr Leu Glu Met Leu Thr Leu Arg His Cys
385                 390                 395                 400

Asp Ile Asn Glu Phe Pro Asn Ile Leu Lys Thr Leu Lys Glu Leu Val
                405                 410                 415

Tyr Ile Asp Ile Ser Asn Asn Arg Met Lys Gly Lys Ile Pro Glu Trp
            420                 425                 430

Leu Trp Ser Leu Pro Leu Leu Gln Ser Val Thr Leu Gly Asn Asn Tyr
        435                 440                 445

Phe Thr Gly Phe Gln Gly Ser Ala Glu Ile Leu Val Asn Ser Ser Val
    450                 455                 460

Leu Leu Leu Tyr Leu Asp Ser Asn Asn Phe Glu Gly Ala Leu Pro Asp
465                 470                 475                 480

Leu Pro Leu Ser Ile Lys Gly Phe Gly Val Ala Ser Asn Ser Phe Thr
                485                 490                 495

Ser Glu Ile Pro Leu Ser Ile Cys Asn Arg Ser Ser Leu Ala Ala Ile
            500                 505                 510

Asp Leu Ser Tyr Asn Asn Phe Thr Gly Pro Ile Pro Pro Cys Leu Arg
        515                 520                 525

Asn Leu Glu Leu Val Tyr Leu Arg Asn Asn Asn Leu Glu Gly Ser Ile
    530                 535                 540

Pro Asp Ala Leu Cys Asp Gly Ala Ser Leu Arg Thr Leu Asp Val Ser
545                 550                 555                 560

His Asn Arg Leu Thr Gly Lys Leu Pro Arg Ser Phe Val Asn Cys Ser
                565                 570                 575

Ser Leu Lys Phe Leu Ser Val Ile Asn Asn Arg Ile Glu Asp Thr Phe
            580                 585                 590

Pro Phe Trp Leu Lys Ala Leu Pro Asn Leu Gln Val Leu Thr Leu Arg
        595                 600                 605

Ser Asn Arg Phe Tyr Gly Pro Ile Ser Pro Pro His Gln Gly Pro Leu
    610                 615                 620

Gly Phe Pro Glu Leu Arg Ile Phe Glu Ile Ser Asp Asn Lys Phe Thr
625                 630                 635                 640

Gly Ser Leu Pro Pro Asn Tyr Phe Val Asn Trp Lys Ala Ser Ser Arg
                645                 650                 655

Thr Met Asn Gln Asp Gly Gly Leu Tyr Met Val Tyr Glu Glu Lys Leu
            660                 665                 670

Phe Asp Glu Gly Gly Tyr Gly Tyr Thr Asp Ala Leu Asp Leu Gln Tyr
        675                 680                 685

Lys Gly Leu His Met Glu Gln Ala Lys Ala Leu Thr Ser Tyr Ala Ala
    690                 695                 700

Ile Asp Phe Ser Gly Asn Arg Leu Glu Gly Gln Ile Pro Glu Ser Ile
705                 710                 715                 720

Gly Leu Leu Lys Ala Leu Ile Ala Val Asn Ile Ser Asn Asn Ala Phe
                725                 730                 735

Thr Gly His Ile Pro Leu Ser Met Ala Asn Leu Glu Asn Leu Glu Ser
            740                 745                 750

Leu Asp Met Ser Arg Asn Gln Leu Ser Gly Thr Ile Pro Asn Gly Leu
        755                 760                 765

Gly Ser Ile Ser Phe Leu Ala Tyr Ile Asn Val Ser His Asn Gln Leu
    770                 775                 780

Thr Gly Glu Ile Pro Gln Gly Thr Gln Ile Thr Gly Gln Ser Lys Ser
785                 790                 795                 800

Ser Phe Glu Gly Asn Ala Gly Leu Cys Gly Leu Pro Leu Lys Glu Ser
                805                 810                 815

Cys Phe Gly Thr Gly Ala Pro Pro Met Tyr His Gln Lys Gln Glu Asp
            820                 825                 830

Lys Glu Glu Glu Glu Glu Glu Glu Glu Glu Glu Glu Glu Val Ala Ala
        835                 840                 845

Trp Ile Leu Gly Phe Val Val Gly Ala Ile Gly Gly Thr Ile Ser Gly
    850                 855                 860

Phe Val Phe Ser Val Leu Phe Lys Leu Ile Ile Gln Ala Ile Arg Gly
865                 870                 875                 880

Ser Glu Lys Pro Pro Gly Pro Ser Ile Phe Ser Pro Leu Ile Lys Lys
                885                 890                 895

Ala Glu Asp Leu Ala Phe Leu Glu Asn Glu Glu Ala Leu Ala Ser Leu
            900                 905                 910

Glu Ile Ile Gly Arg Gly Gly Cys Gly Glu Val Phe Lys Ala Glu Leu
        915                 920                 925

Pro Gly Ser Asn Gly Lys Ile Ile Ala Val Lys Lys Val Ile Gln Pro
    930                 935                 940

Pro Lys Asp Ala Asp Glu Leu Thr Asp Glu Asp Ser Lys Phe Leu Asn
945                 950                 955                 960

Lys Lys Met Arg Gln Ile Arg Ser Glu Ile Asn Thr Val Gly His Ile
                965                 970                 975

Arg His Arg Asn Leu Leu Pro Leu Leu Ala His Val Ser Arg Pro Glu
            980                 985                 990

Cys His Tyr Leu Val Tyr Glu Tyr  Met Glu Lys Gly Ser  Leu Gln Asp
        995                 1000                1005

Ile Leu  Thr Asp Val Gln Ala  Gly Asn Gln Glu Leu  Met Trp Pro
    1010                1015                1020

Ala Arg  His Lys Ile Ala Leu  Gly Ile Ala Ala Gly  Leu Glu Tyr
    1025                1030                1035

Leu His  Met Asp His Asn Pro  Arg Ile Ile His Arg  Asp Leu Lys

1040                1045                1050

Pro Ala Asn Val Leu Leu Asp Asp Asp Met Glu Ala Arg Ile Ser
    1055                1060                1065

Asp Phe Gly Leu Ala Lys Ala Met Pro Asp Ala Val Thr His Ile
    1070                1075                1080

Thr Thr Ser His Val Ala Gly Thr Val Gly Tyr Ile Ala Pro Glu
    1085                1090                1095

Phe Tyr Gln Thr His Lys Phe Thr Asp Lys Cys Asp Ile Tyr Ser
    1100                1105                1110

Phe Gly Val Ile Leu Gly Ile Leu Val Ile Gly Lys Leu Pro Ser
    1115                1120                1125

Asp Glu Phe Phe Gln His Thr Asp Glu Met Ser Leu Ile Lys Trp
    1130                1135                1140

Met Arg Asn Ile Ile Thr Ser Glu Asn Pro Ser Leu Ala Ile Asp
    1145                1150                1155

Pro Lys Leu Met Asp Gln Gly Phe Asp Glu Gln Met Leu Leu Val
    1160                1165                1170

Leu Lys Ile Ala Cys Tyr Cys Thr Leu Asp Asp Pro Lys Gln Arg
    1175                1180                1185

Pro Asn Ser Lys Asp Val Arg Thr Met Leu Ser Gln Ile Lys His
    1190                1195                1200

<210>  7
<211>  1024
<212>  PRT
<213>  Artificial

<220>
<223>  EIX2tmSOBIR1

<400>  7

Met Gly Lys Arg Thr Asn Pro Arg His Phe Leu Val Thr Trp Ser Leu
1               5                   10                  15

Leu Leu Leu Glu Thr Ala Phe Gly Leu Thr Ser Arg Glu Val Asn Lys
            20                  25                  30

Thr Leu Cys Ile Glu Lys Glu Arg Gly Ala Leu Leu Glu Phe Lys Arg
        35                  40                  45

Gly Leu Asn Asp Asp Phe Gly Arg Leu Ser Thr Trp Gly Asp Glu Glu
    50                  55                  60

Glu Cys Cys Asn Trp Lys Gly Ile Glu Cys Asp Lys Arg Thr Gly His
65                  70                  75                  80

Val Ile Val Leu Asp Leu His Ser Glu Val Thr Cys Pro Gly His Ala
                85                  90                  95

Cys Phe Ala Pro Ile Leu Thr Gly Lys Val Ser Pro Ser Leu Leu Glu
            100                 105                 110

Leu Glu Tyr Leu Asn Phe Leu Asp Leu Ser Val Asn Gly Phe Glu Asn
        115                 120                 125

Ser Glu Ile Pro Arg Phe Ile Gly Ser Leu Lys Arg Leu Glu Tyr Leu
    130                 135                 140

Asn Leu Ser Ser Ser Asp Phe Ser Gly Glu Ile Pro Ala Gln Phe Gln
145                 150                 155                 160

Asn Leu Thr Ser Leu Arg Ile Leu Asp Leu Gly Asn Asn Asn Leu Ile
                165                 170                 175

Val Lys Asp Leu Val Trp Leu Ser His Leu Ser Ser Leu Glu Phe Leu
            180                 185                 190

Arg Leu Gly Gly Asn Asp Phe Gln Ala Arg Asn Trp Phe Arg Glu Ile
        195                 200                 205

Thr Lys Val Pro Ser Leu Lys Glu Leu Asp Leu Ser Val Cys Gly Leu
    210                 215                 220

Ser Lys Phe Val Pro Ser Pro Ala Asp Val Ala Asn Ser Ser Leu Ile
225                 230                 235                 240

Ser Leu Ser Val Leu His Leu Cys Cys Asn Glu Phe Ser Thr Ser Ser
                245                 250                 255

Glu Tyr Ser Trp Leu Phe Asn Phe Ser Thr Ser Leu Thr Ser Ile Asp
            260                 265                 270

Leu Ser His Asn Gln Leu Ser Arg Gln Ile Asp Asp Arg Phe Gly Ser
        275                 280                 285

Leu Met Tyr Leu Glu His Leu Asn Leu Ala Asn Asn Phe Gly Ala Glu

290                 295                 300

Gly Gly Val Pro Ser Ser Phe Gly Asn Leu Thr Arg Leu His Tyr Leu
305                 310                 315                 320

Asp Met Ser Asn Thr Gln Thr Tyr Gln Trp Leu Pro Glu Leu Phe Leu
                325                 330                 335

Arg Leu Ser Gly Ser Arg Lys Ser Leu Glu Val Leu Gly Leu Asn Asp
            340                 345                 350

Asn Ser Leu Phe Gly Ser Ile Val Asn Val Pro Arg Phe Ser Ser Leu
        355                 360                 365

Lys Lys Leu Tyr Leu Gln Lys Asn Met Leu Asn Gly Phe Phe Met Glu
    370                 375                 380

Arg Val Gly Gln Val Ser Ser Leu Glu Tyr Leu Asp Leu Ser Asp Asn
385                 390                 395                 400

Gln Met Arg Gly Pro Leu Pro Asp Leu Ala Leu Phe Pro Ser Leu Arg
                405                 410                 415

Glu Leu His Leu Gly Ser Asn Gln Phe Gln Gly Arg Ile Pro Gln Gly
            420                 425                 430

Ile Gly Lys Leu Ser Gln Leu Arg Ile Phe Asp Val Ser Ser Asn Arg
        435                 440                 445

Leu Glu Gly Leu Pro Glu Ser Met Gly Gln Leu Ser Asn Leu Glu Arg
    450                 455                 460

Phe Asp Ala Ser Tyr Asn Val Leu Lys Gly Thr Ile Thr Glu Ser His
465                 470                 475                 480

Phe Ser Asn Leu Ser Ser Leu Val Asp Leu Asp Leu Ser Phe Asn Leu
                485                 490                 495

Leu Ser Leu Asn Thr Arg Phe Asp Trp Val Pro Pro Phe Gln Leu Gln
            500                 505                 510

Phe Ile Arg Leu Pro Ser Cys Asn Met Gly Pro Ser Phe Pro Lys Trp
        515                 520                 525

Leu Gln Thr Gln Asn Asn Tyr Thr Leu Leu Asp Ile Ser Leu Ala Asn
    530                 535                 540

Ile Ser Asp Met Leu Pro Ser Trp Phe Ser Asn Leu Pro Pro Glu Leu
545                 550                 555                 560

Lys Ile Leu Asn Leu Ser Asn Asn His Ile Ser Gly Arg Val Ser Glu
                565                 570                 575

Phe Ile Val Ser Lys Gln Asp Tyr Met Ile Ile Asp Leu Ser Ser Asn
            580                 585                 590

Asn Phe Ser Gly His Leu Pro Leu Val Pro Ala Asn Ile Gln Ile Phe
        595                 600                 605

Tyr Leu His Lys Asn His Phe Ser Gly Ser Ile Ser Ser Ile Cys Arg
    610                 615                 620

Asn Thr Ile Gly Ala Ala Thr Ser Ile Asp Leu Ser Arg Asn Gln Phe
625                 630                 635                 640

Ser Gly Glu Val Pro Asp Cys Trp Met Asn Met Ser Asn Leu Ala Val
                645                 650                 655

Leu Asn Leu Ala Tyr Asn Asn Phe Ser Gly Lys Val Pro Gln Ser Leu
            660                 665                 670

Gly Ser Leu Thr Asn Leu Glu Ala Leu Tyr Ile Arg Gln Asn Ser Phe
        675                 680                 685

Arg Gly Met Leu Pro Ser Phe Ser Gln Cys Gln Leu Leu Gln Ile Leu
    690                 695                 700

Asp Ile Gly Gly Asn Lys Leu Thr Gly Arg Ile Pro Ala Trp Ile Gly
705                 710                 715                 720

Thr Asp Leu Leu Gln Leu Arg Ile Leu Ser Leu Arg Ser Asn Lys Phe
                725                 730                 735

Asp Gly Ser Ile Pro Ser Leu Ile Cys Gln Leu Gln Phe Leu Gln Ile
            740                 745                 750

Leu Asp Leu Ser Glu Asn Gly Leu Ser Gly Lys Ile Pro Gln Cys Leu
        755                 760                 765

Asn Asn Phe Thr Ile Leu Arg Gln Glu Asn Gly Ser Gly Glu Ser Met
    770                 775                 780

Asp Phe Lys Val Arg Tyr Asp Tyr Ile Pro Gly Ser Tyr Leu Tyr Ile
785                 790                 795                 800

Gly Asp Leu Leu Ile Gln Trp Lys Asn Gln Glu Ser Glu Tyr Lys Asn
                805                 810                 815

Ala Leu Leu Tyr Leu Lys Ile Ile Asp Leu Ser Ser Asn Lys Leu Val
            820                 825                 830

Gly Gly Ile Pro Lys Glu Ile Ala Glu Met Arg Gly Leu Arg Ser Leu
        835                 840                 845

Asn Leu Ser Arg Asn Asp Leu Asn Gly Thr Val Val Glu Gly Ile Gly
    850                 855                 860

Gln Met Lys Leu Leu Glu Ser Leu Asp Leu Ser Arg Asn Gln Leu Ser
865                 870                 875                 880

Gly Met Ile Pro Gln Gly Leu Ser Asn Leu Thr Phe Leu Ser Val Leu
                885                 890                 895

Asp Leu Ser Asn Asn His Leu Ser Gly Arg Ile Pro Ser Ser Thr Gln
            900                 905                 910

Leu Gln Ser Phe Asp Arg Ser Ser Tyr Ser Gly Asn Ala Gln Leu Cys
        915                 920                 925

Gly Pro Pro Leu Glu Glu Cys Pro Gly Tyr Ala Pro Pro Ile Asp Arg
    930                 935                 940

Gly Ser Asn Thr Asn Pro Gln Glu His Asp Asp Asp Asp Glu Phe Ser
945                 950                 955                 960

Ser Leu Glu Val Ala Ala Trp Ile Leu Gly Phe Val Val Gly Ala Ile
                965                 970                 975

Gly Gly Thr Ile Ser Gly Phe Val Phe Ser Val Leu Phe Arg Ser Trp
            980                 985                 990

Arg Asn Ala Tyr Phe Thr Phe Leu  Thr Asp Met Lys Ser  Trp Leu His
        995                 1000                1005

Met Thr  Ser Arg Val Cys Phe  Ala Arg Leu Lys Gly  Lys Leu Arg
    1010                1015                1020

Asn

<210>  8
<211>  1321

<212>  PRT
<213>  Artificial

<220>
<223>  EIX2tmkSOBIR1

<400>  8

Met Gly Lys Arg Thr Asn Pro Arg His Phe Leu Val Thr Trp Ser Leu
1               5                   10                  15

Leu Leu Leu Glu Thr Ala Phe Gly Leu Thr Ser Arg Glu Val Asn Lys
            20                  25                  30

Thr Leu Cys Ile Glu Lys Glu Arg Gly Ala Leu Leu Glu Phe Lys Arg
        35                  40                  45

Gly Leu Asn Asp Asp Phe Gly Arg Leu Ser Thr Trp Gly Asp Glu Glu
    50                  55                  60

Glu Cys Cys Asn Trp Lys Gly Ile Glu Cys Asp Lys Arg Thr Gly His
65                  70                  75                  80

Val Ile Val Leu Asp Leu His Ser Glu Val Thr Cys Pro Gly His Ala
                85                  90                  95

Cys Phe Ala Pro Ile Leu Thr Gly Lys Val Ser Pro Ser Leu Leu Glu
            100                 105                 110

Leu Glu Tyr Leu Asn Phe Leu Asp Leu Ser Val Asn Gly Phe Glu Asn
        115                 120                 125

Ser Glu Ile Pro Arg Phe Ile Gly Ser Leu Lys Arg Leu Glu Tyr Leu
    130                 135                 140

Asn Leu Ser Ser Ser Asp Phe Ser Gly Glu Ile Pro Ala Gln Phe Gln
145                 150                 155                 160

Asn Leu Thr Ser Leu Arg Ile Leu Asp Leu Gly Asn Asn Asn Leu Ile
                165                 170                 175

Val Lys Asp Leu Val Trp Leu Ser His Leu Ser Ser Leu Glu Phe Leu
            180                 185                 190

Arg Leu Gly Gly Asn Asp Phe Gln Ala Arg Asn Trp Phe Arg Glu Ile
        195                 200                 205

Thr Lys Val Pro Ser Leu Lys Glu Leu Asp Leu Ser Val Cys Gly Leu
    210                 215                 220

Ser Lys Phe Val Pro Ser Pro Ala Asp Val Ala Asn Ser Ser Leu Ile
225                 230                 235                 240

Ser Leu Ser Val Leu His Leu Cys Cys Asn Glu Phe Ser Thr Ser Ser
                245                 250                 255

Glu Tyr Ser Trp Leu Phe Asn Phe Ser Thr Ser Leu Thr Ser Ile Asp
            260                 265                 270

Leu Ser His Asn Gln Leu Ser Arg Gln Ile Asp Asp Arg Phe Gly Ser
        275                 280                 285

Leu Met Tyr Leu Glu His Leu Asn Leu Ala Asn Asn Phe Gly Ala Glu
    290                 295                 300

Gly Gly Val Pro Ser Ser Phe Gly Asn Leu Thr Arg Leu His Tyr Leu
305                 310                 315                 320

Asp Met Ser Asn Thr Gln Thr Tyr Gln Trp Leu Pro Glu Leu Phe Leu
                325                 330                 335

Arg Leu Ser Gly Ser Arg Lys Ser Leu Glu Val Leu Gly Leu Asn Asp
            340                 345                 350

Asn Ser Leu Phe Gly Ser Ile Val Asn Val Pro Arg Phe Ser Ser Leu
        355                 360                 365

Lys Lys Leu Tyr Leu Gln Lys Asn Met Leu Asn Gly Phe Phe Met Glu
    370                 375                 380

Arg Val Gly Gln Val Ser Ser Leu Glu Tyr Leu Asp Leu Ser Asp Asn
385                 390                 395                 400

Gln Met Arg Gly Pro Leu Pro Asp Leu Ala Leu Phe Pro Ser Leu Arg
                405                 410                 415

Glu Leu His Leu Gly Ser Asn Gln Phe Gln Gly Arg Ile Pro Gln Gly
            420                 425                 430

Ile Gly Lys Leu Ser Gln Leu Arg Ile Phe Asp Val Ser Ser Asn Arg
        435                 440                 445

Leu Glu Gly Leu Pro Glu Ser Met Gly Gln Leu Ser Asn Leu Glu Arg
    450                 455                 460

Phe Asp Ala Ser Tyr Asn Val Leu Lys Gly Thr Ile Thr Glu Ser His
465                 470                 475                 480

Phe Ser Asn Leu Ser Ser Leu Val Asp Leu Asp Leu Ser Phe Asn Leu
                485                 490                 495

Leu Ser Leu Asn Thr Arg Phe Asp Trp Val Pro Pro Phe Gln Leu Gln
            500                 505                 510

Phe Ile Arg Leu Pro Ser Cys Asn Met Gly Pro Ser Phe Pro Lys Trp
        515                 520                 525

Leu Gln Thr Gln Asn Asn Tyr Thr Leu Leu Asp Ile Ser Leu Ala Asn
    530                 535                 540

Ile Ser Asp Met Leu Pro Ser Trp Phe Ser Asn Leu Pro Pro Glu Leu
545                 550                 555                 560

Lys Ile Leu Asn Leu Ser Asn Asn His Ile Ser Gly Arg Val Ser Glu
                565                 570                 575

Phe Ile Val Ser Lys Gln Asp Tyr Met Ile Ile Asp Leu Ser Ser Asn
            580                 585                 590

Asn Phe Ser Gly His Leu Pro Leu Val Pro Ala Asn Ile Gln Ile Phe
        595                 600                 605

Tyr Leu His Lys Asn His Phe Ser Gly Ser Ile Ser Ser Ile Cys Arg
    610                 615                 620

Asn Thr Ile Gly Ala Ala Thr Ser Ile Asp Leu Ser Arg Asn Gln Phe
625                 630                 635                 640

Ser Gly Glu Val Pro Asp Cys Trp Met Asn Met Ser Asn Leu Ala Val
                645                 650                 655

Leu Asn Leu Ala Tyr Asn Asn Phe Ser Gly Lys Val Pro Gln Ser Leu
            660                 665                 670

Gly Ser Leu Thr Asn Leu Glu Ala Leu Tyr Ile Arg Gln Asn Ser Phe
        675                 680                 685

Arg Gly Met Leu Pro Ser Phe Ser Gln Cys Gln Leu Leu Gln Ile Leu
    690                 695                 700

Asp Ile Gly Gly Asn Lys Leu Thr Gly Arg Ile Pro Ala Trp Ile Gly
705                 710                 715                 720

Thr Asp Leu Leu Gln Leu Arg Ile Leu Ser Leu Arg Ser Asn Lys Phe

                725                 730                 735

Asp Gly Ser Ile Pro Ser Leu Ile Cys Gln Leu Gln Phe Leu Gln Ile
            740                 745                 750

Leu Asp Leu Ser Glu Asn Gly Leu Ser Gly Lys Ile Pro Gln Cys Leu
        755                 760                 765

Asn Asn Phe Thr Ile Leu Arg Gln Glu Asn Gly Ser Gly Glu Ser Met
    770                 775                 780

Asp Phe Lys Val Arg Tyr Asp Tyr Ile Pro Gly Ser Tyr Leu Tyr Ile
785                 790                 795                 800

Gly Asp Leu Leu Ile Gln Trp Lys Asn Gln Glu Ser Glu Tyr Lys Asn
                805                 810                 815

Ala Leu Leu Tyr Leu Lys Ile Ile Asp Leu Ser Ser Asn Lys Leu Val
            820                 825                 830

Gly Gly Ile Pro Lys Glu Ile Ala Glu Met Arg Gly Leu Arg Ser Leu
        835                 840                 845

Asn Leu Ser Arg Asn Asp Leu Asn Gly Thr Val Val Glu Gly Ile Gly
    850                 855                 860

Gln Met Lys Leu Leu Glu Ser Leu Asp Leu Ser Arg Asn Gln Leu Ser
865                 870                 875                 880

Gly Met Ile Pro Gln Gly Leu Ser Asn Leu Thr Phe Leu Ser Val Leu
                885                 890                 895

Asp Leu Ser Asn Asn His Leu Ser Gly Arg Ile Pro Ser Ser Thr Gln
            900                 905                 910

Leu Gln Ser Phe Asp Arg Ser Ser Tyr Ser Gly Asn Ala Gln Leu Cys
        915                 920                 925

Gly Pro Pro Leu Glu Glu Cys Pro Gly Tyr Ala Pro Pro Ile Asp Arg
    930                 935                 940

Gly Ser Asn Thr Asn Pro Gln Glu His Asp Asp Asp Asp Glu Phe Ser
945                 950                 955                 960

Ser Leu Glu Val Ala Ala Trp Ile Leu Gly Phe Val Val Gly Ala Ile
                965                 970                 975

Gly Gly Thr Ile Ser Gly Phe Val Phe Ser Val Leu Phe Lys Leu Ile
            980                 985                 990

Ile Gln Ala Ile Arg Gly Ser Glu Lys Pro Pro Gly Pro Ser Ile Phe
        995                 1000                1005

Ser Pro Leu Ile Lys Lys Ala Glu Asp Leu Ala Phe Leu Glu Asn
    1010                1015                1020

Glu Glu Ala Leu Ala Ser Leu Glu Ile Ile Gly Arg Gly Gly Cys
    1025                1030                1035

Gly Glu Val Phe Lys Ala Glu Leu Pro Gly Ser Asn Gly Lys Ile
    1040                1045                1050

Ile Ala Val Lys Lys Val Ile Gln Pro Pro Lys Asp Ala Asp Glu
    1055                1060                1065

Leu Thr Asp Glu Asp Ser Lys Phe Leu Asn Lys Lys Met Arg Gln
    1070                1075                1080

Ile Arg Ser Glu Ile Asn Thr Val Gly His Ile Arg His Arg Asn
    1085                1090                1095

Leu Leu Pro Leu Leu Ala His Val Ser Arg Pro Glu Cys His Tyr
    1100                1105                1110

Leu Val Tyr Glu Tyr Met Glu Lys Gly Ser Leu Gln Asp Ile Leu
    1115                1120                1125

Thr Asp Val Gln Ala Gly Asn Gln Glu Leu Met Trp Pro Ala Arg
    1130                1135                1140

His Lys Ile Ala Leu Gly Ile Ala Ala Gly Leu Glu Tyr Leu His
    1145                1150                1155

Met Asp His Asn Pro Arg Ile Ile His Arg Asp Leu Lys Pro Ala
    1160                1165                1170

Asn Val Leu Leu Asp Asp Asp Met Glu Ala Arg Ile Ser Asp Phe
    1175                1180                1185

Gly Leu Ala Lys Ala Met Pro Asp Ala Val Thr His Ile Thr Thr
    1190                1195                1200

Ser His Val Ala Gly Thr Val Gly Tyr Ile Ala Pro Glu Phe Tyr
    1205                1210                1215

Gln Thr His Lys Phe Thr Asp Lys Cys Asp Ile Tyr Ser Phe Gly
    1220                1225                1230

Val Ile Leu Gly Ile Leu Val Ile Gly Lys Leu Pro Ser Asp Glu
    1235                1240                1245

Phe Phe Gln His Thr Asp Glu Met Ser Leu Ile Lys Trp Met Arg
    1250                1255                1260

Asn Ile Ile Thr Ser Glu Asn Pro Ser Leu Ala Ile Asp Pro Lys
    1265                1270                1275

Leu Met Asp Gln Gly Phe Asp Glu Gln Met Leu Leu Val Leu Lys
    1280                1285                1290

Ile Ala Cys Tyr Cys Thr Leu Asp Asp Pro Lys Gln Arg Pro Asn
    1295                1300                1305

Ser Lys Asp Val Arg Thr Met Leu Ser Gln Ile Lys His
    1310                1315                1320

<210> 9
<211> 1007
<212> PRT
<213> Artificial

<220>
<223> EFRtmkSOBIR1

<400> 9

Met Lys Leu Ser Phe Ser Leu Val Phe Asn Ala Leu Thr Leu Leu Leu
1               5                   10                  15

Gln Val Cys Ile Phe Ala Gln Ala Arg Phe Ser Asn Glu Thr Asp Met
            20                  25                  30

Gln Ala Leu Leu Glu Phe Lys Ser Gln Val Ser Glu Asn Asn Lys Arg
        35                  40                  45

Glu Val Leu Ala Ser Trp Asn His Ser Ser Pro Phe Cys Asn Trp Ile
    50                  55                  60

Gly Val Thr Cys Gly Arg Arg Arg Glu Arg Val Ile Ser Leu Asn Leu
65                  70                  75                  80

Gly Gly Phe Lys Leu Thr Gly Val Ile Ser Pro Ser Ile Gly Asn Leu
                85                  90                  95

Ser Phe Leu Arg Leu Leu Asn Leu Ala Asp Asn Ser Phe Gly Ser Thr
            100                 105                 110

Ile Pro Gln Lys Val Gly Arg Leu Phe Arg Leu Gln Tyr Leu Asn Met
        115                 120                 125

Ser Tyr Asn Leu Leu Glu Gly Arg Ile Pro Ser Ser Leu Ser Asn Cys
    130                 135                 140

Ser Arg Leu Ser Thr Val Asp Leu Ser Ser Asn His Leu Gly His Gly
145                 150                 155                 160

Val Pro Ser Glu Leu Gly Ser Leu Ser Lys Leu Ala Ile Leu Asp Leu
                165                 170                 175

Ser Lys Asn Asn Leu Thr Gly Asn Phe Pro Ala Ser Leu Gly Asn Leu
            180                 185                 190

Thr Ser Leu Gln Lys Leu Asp Phe Ala Tyr Asn Gln Met Arg Gly Glu
        195                 200                 205

Ile Pro Asp Glu Val Ala Arg Leu Thr Gln Met Val Phe Phe Gln Ile
    210                 215                 220

Ala Leu Asn Ser Phe Ser Gly Gly Phe Pro Pro Ala Leu Tyr Asn Ile
225                 230                 235                 240

Ser Ser Leu Glu Ser Leu Ser Leu Ala Asp Asn Ser Phe Ser Gly Asn
                245                 250                 255

Leu Arg Ala Asp Phe Gly Tyr Leu Leu Pro Asn Leu Arg Arg Leu Leu
            260                 265                 270

Leu Gly Thr Asn Gln Phe Thr Gly Ala Ile Pro Lys Thr Leu Ala Asn
        275                 280                 285

Ile Ser Ser Leu Glu Arg Phe Asp Ile Ser Ser Asn Tyr Leu Ser Gly
    290                 295                 300

Ser Ile Pro Leu Ser Phe Gly Lys Leu Arg Asn Leu Trp Trp Leu Gly
305                 310                 315                 320

Ile Arg Asn Asn Ser Leu Gly Asn Asn Ser Ser Ser Gly Leu Glu Phe
                325                 330                 335

Ile Gly Ala Val Ala Asn Cys Thr Gln Leu Glu Tyr Leu Asp Val Gly
            340                 345                 350

Tyr Asn Arg Leu Gly Gly Glu Leu Pro Ala Ser Ile Ala Asn Leu Ser
        355                 360                 365

Thr Thr Leu Thr Ser Leu Phe Leu Gly Gln Asn Leu Ile Ser Gly Thr
    370                 375                 380

Ile Pro His Asp Ile Gly Asn Leu Val Ser Leu Gln Glu Leu Ser Leu
385                 390                 395                 400

Glu Thr Asn Met Leu Ser Gly Glu Leu Pro Val Ser Phe Gly Lys Leu
                405                 410                 415

Leu Asn Leu Gln Val Val Asp Leu Tyr Ser Asn Ala Ile Ser Gly Glu
            420                 425                 430

Ile Pro Ser Tyr Phe Gly Asn Met Thr Arg Leu Gln Lys Leu His Leu
        435                 440                 445

Asn Ser Asn Ser Phe His Gly Arg Ile Pro Gln Ser Leu Gly Arg Cys
    450                 455                 460

Arg Tyr Leu Leu Asp Leu Trp Met Asp Thr Asn Arg Leu Asn Gly Thr
465                 470                 475                 480

Ile Pro Gln Glu Ile Leu Gln Ile Pro Ser Leu Ala Tyr Ile Asp Leu
                485                 490                 495

Ser Asn Asn Phe Leu Thr Gly His Phe Pro Glu Glu Val Gly Lys Leu
            500                 505                 510

Glu Leu Leu Val Gly Leu Gly Ala Ser Tyr Asn Lys Leu Ser Gly Lys
        515                 520                 525

Met Pro Gln Ala Ile Gly Gly Cys Leu Ser Met Glu Phe Leu Phe Met
    530                 535                 540

Gln Gly Asn Ser Phe Asp Gly Ala Ile Pro Asp Ile Ser Arg Leu Val
545                 550                 555                 560

Ser Leu Lys Asn Val Asp Phe Ser Asn Asn Asn Leu Ser Gly Arg Ile
                565                 570                 575

Pro Arg Tyr Leu Ala Ser Leu Pro Ser Leu Arg Asn Leu Asn Leu Ser
            580                 585                 590

Met Asn Lys Phe Glu Gly Arg Val Pro Thr Thr Gly Val Phe Arg Asn
        595                 600                 605

Ala Thr Ala Val Ser Val Phe Gly Asn Thr Asn Ile Cys Gly Gly Val
    610                 615                 620

Arg Glu Met Gln Leu Lys Pro Cys Ile Val Gln Ala Ser Pro Arg Lys
625                 630                 635                 640

Arg Lys Pro Leu Ser Val Arg Lys Lys Val Ala Ala Trp Ile Leu Gly
                645                 650                 655

Phe Val Val Gly Ala Ile Gly Gly Thr Ile Ser Gly Phe Val Phe Ser
            660                 665                 670

Val Leu Phe Lys Leu Ile Ile Gln Ala Ile Arg Gly Ser Glu Lys Pro
        675                 680                 685

Pro Gly Pro Ser Ile Phe Ser Pro Leu Ile Lys Lys Ala Glu Asp Leu
    690                 695                 700

Ala Phe Leu Glu Asn Glu Glu Ala Leu Ala Ser Leu Glu Ile Ile Gly
705                 710                 715                 720

Arg Gly Gly Cys Gly Glu Val Phe Lys Ala Glu Leu Pro Gly Ser Asn
                725                 730                 735

Gly Lys Ile Ile Ala Val Lys Lys Val Ile Gln Pro Pro Lys Asp Ala
            740                 745                 750

Asp Glu Leu Thr Asp Glu Asp Ser Lys Phe Leu Asn Lys Lys Met Arg
        755                 760                 765

Gln Ile Arg Ser Glu Ile Asn Thr Val Gly His Ile Arg His Arg Asn
    770                 775                 780

Leu Leu Pro Leu Leu Ala His Val Ser Arg Pro Glu Cys His Tyr Leu
785                 790                 795                 800

Val Tyr Glu Tyr Met Glu Lys Gly Ser Leu Gln Asp Ile Leu Thr Asp
                805                 810                 815

Val Gln Ala Gly Asn Gln Glu Leu Met Trp Pro Ala Arg His Lys Ile
            820                 825                 830

Ala Leu Gly Ile Ala Ala Gly Leu Glu Tyr Leu His Met Asp His Asn
        835                 840                 845

Pro Arg Ile Ile His Arg Asp Leu Lys Pro Ala Asn Val Leu Leu Asp

850                 855                 860

Asp Asp Met Glu Ala Arg Ile Ser Asp Phe Gly Leu Ala Lys Ala Met
865                 870                 875                 880

Pro Asp Ala Val Thr His Ile Thr Thr Ser His Val Ala Gly Thr Val
                885                 890                 895

Gly Tyr Ile Ala Pro Glu Phe Tyr Gln Thr His Lys Phe Thr Asp Lys
            900                 905                 910

Cys Asp Ile Tyr Ser Phe Gly Val Ile Leu Gly Ile Leu Val Ile Gly
        915                 920                 925

Lys Leu Pro Ser Asp Glu Phe Phe Gln His Thr Asp Glu Met Ser Leu
    930                 935                 940

Ile Lys Trp Met Arg Asn Ile Ile Thr Ser Glu Asn Pro Ser Leu Ala
945                 950                 955                 960

Ile Asp Pro Lys Leu Met Asp Gln Gly Phe Asp Glu Gln Met Leu Leu
                965                 970                 975

Val Leu Lys Ile Ala Cys Tyr Cys Thr Leu Asp Asp Pro Lys Gln Arg
            980                 985                 990

Pro Asn Ser Lys Asp Val Arg Thr  Met Leu Ser Gln Ile  Lys His
        995                 1000                1005

<210>  10
<211>  710
<212>  PRT
<213>  Artificial

<220>
<223>  EFRdeltakin-tmSOBIR1

<400>  10

Met Lys Leu Ser Phe Ser Leu Val Phe Asn Ala Leu Thr Leu Leu Leu
1               5                   10                  15

Gln Val Cys Ile Phe Ala Gln Ala Arg Phe Ser Asn Glu Thr Asp Met
            20                  25                  30

Gln Ala Leu Leu Glu Phe Lys Ser Gln Val Ser Glu Asn Asn Lys Arg
        35                  40                  45

Glu Val Leu Ala Ser Trp Asn His Ser Ser Pro Phe Cys Asn Trp Ile
    50                  55                  60

Gly Val Thr Cys Gly Arg Arg Arg Glu Arg Val Ile Ser Leu Asn Leu
65                  70                  75                  80

Gly Gly Phe Lys Leu Thr Gly Val Ile Ser Pro Ser Ile Gly Asn Leu
                85                  90                  95

Ser Phe Leu Arg Leu Leu Asn Leu Ala Asp Asn Ser Phe Gly Ser Thr
            100                 105                 110

Ile Pro Gln Lys Val Gly Arg Leu Phe Arg Leu Gln Tyr Leu Asn Met
        115                 120                 125

Ser Tyr Asn Leu Leu Glu Gly Arg Ile Pro Ser Ser Leu Ser Asn Cys
    130                 135                 140

Ser Arg Leu Ser Thr Val Asp Leu Ser Ser Asn His Leu Gly His Gly
145                 150                 155                 160

Val Pro Ser Glu Leu Gly Ser Leu Ser Lys Leu Ala Ile Leu Asp Leu
                165                 170                 175

Ser Lys Asn Asn Leu Thr Gly Asn Phe Pro Ala Ser Leu Gly Asn Leu
            180                 185                 190

Thr Ser Leu Gln Lys Leu Asp Phe Ala Tyr Asn Gln Met Arg Gly Glu
        195                 200                 205

Ile Pro Asp Glu Val Ala Arg Leu Thr Gln Met Val Phe Phe Gln Ile
    210                 215                 220

Ala Leu Asn Ser Phe Ser Gly Gly Phe Pro Pro Ala Leu Tyr Asn Ile
225                 230                 235                 240

Ser Ser Leu Glu Ser Leu Ser Leu Ala Asp Asn Ser Phe Ser Gly Asn
                245                 250                 255

Leu Arg Ala Asp Phe Gly Tyr Leu Leu Pro Asn Leu Arg Arg Leu Leu
            260                 265                 270

Leu Gly Thr Asn Gln Phe Thr Gly Ala Ile Pro Lys Thr Leu Ala Asn
        275                 280                 285

Ile Ser Ser Leu Glu Arg Phe Asp Ile Ser Ser Asn Tyr Leu Ser Gly
    290                 295                 300

Ser Ile Pro Leu Ser Phe Gly Lys Leu Arg Asn Leu Trp Trp Leu Gly

305                 310                 315                 320

Ile Arg Asn Asn Ser Leu Gly Asn Asn Ser Ser Ser Gly Leu Glu Phe
                325                 330                 335

Ile Gly Ala Val Ala Asn Cys Thr Gln Leu Glu Tyr Leu Asp Val Gly
            340                 345                 350

Tyr Asn Arg Leu Gly Gly Glu Leu Pro Ala Ser Ile Ala Asn Leu Ser
        355                 360                 365

Thr Thr Leu Thr Ser Leu Phe Leu Gly Gln Asn Leu Ile Ser Gly Thr
    370                 375                 380

Ile Pro His Asp Ile Gly Asn Leu Val Ser Leu Gln Glu Leu Ser Leu
385                 390                 395                 400

Glu Thr Asn Met Leu Ser Gly Glu Leu Pro Val Ser Phe Gly Lys Leu
                405                 410                 415

Leu Asn Leu Gln Val Val Asp Leu Tyr Ser Asn Ala Ile Ser Gly Glu
            420                 425                 430

Ile Pro Ser Tyr Phe Gly Asn Met Thr Arg Leu Gln Lys Leu His Leu
        435                 440                 445

Asn Ser Asn Ser Phe His Gly Arg Ile Pro Gln Ser Leu Gly Arg Cys
    450                 455                 460

Arg Tyr Leu Leu Asp Leu Trp Met Asp Thr Asn Arg Leu Asn Gly Thr
465                 470                 475                 480

Ile Pro Gln Glu Ile Leu Gln Ile Pro Ser Leu Ala Tyr Ile Asp Leu
                485                 490                 495

Ser Asn Asn Phe Leu Thr Gly His Phe Pro Glu Glu Val Gly Lys Leu
            500                 505                 510

Glu Leu Leu Val Gly Leu Gly Ala Ser Tyr Asn Lys Leu Ser Gly Lys
        515                 520                 525

Met Pro Gln Ala Ile Gly Gly Cys Leu Ser Met Glu Phe Leu Phe Met
    530                 535                 540

Gln Gly Asn Ser Phe Asp Gly Ala Ile Pro Asp Ile Ser Arg Leu Val
545                 550                 555                 560

Ser Leu Lys Asn Val Asp Phe Ser Asn Asn Asn Leu Ser Gly Arg Ile
                565                 570                 575

Pro Arg Tyr Leu Ala Ser Leu Pro Ser Leu Arg Asn Leu Asn Leu Ser
            580                 585                 590

Met Asn Lys Phe Glu Gly Arg Val Pro Thr Thr Gly Val Phe Arg Asn
        595                 600                 605

Ala Thr Ala Val Ser Val Phe Gly Asn Thr Asn Ile Cys Gly Gly Val
    610                 615                 620

Arg Glu Met Gln Leu Lys Pro Cys Ile Val Gln Ala Ser Pro Arg Lys
625                 630                 635                 640

Arg Lys Pro Leu Ser Val Arg Lys Lys Val Ala Ala Trp Ile Leu Gly
                645                 650                 655

Phe Val Val Gly Ala Ile Gly Gly Thr Ile Ser Gly Phe Val Phe Ser
            660                 665                 670

Val Leu Phe Met Lys Arg Lys Lys Lys Asn Asn Ala Ser Asp Gly Asn
        675                 680                 685

Pro Ser Asp Ser Thr Thr Leu Gly Met Phe His Glu Lys Val Ser Tyr
    690                 695                 700

Glu Glu Leu His Ser Ala
705                 710

<210>  11
<211>  3093
<212>  DNA
<213>  Arabidopsis thaliana

<400>  11
atgaagctgt ccttttcact tgttttcaat gctctcacgt tgcttcttca agtttgcatc      60

tttgctcaag ccaggttttc taatgagact gatatgcaag ctttgcttga gttcaagtct     120

caagtttctg aaaacaacaa gagagaggtc ttggcttcat ggaatcactc ctccccattt     180

tgtaattgga ttggagtcac atgtggccgc aggagagaaa gagttataag tttgaacctt     240

ggaggattca agttaaccgg tgtgatctca ccctccattg gtaatctctc ctttcttaga     300

ttacttaatc ttgcagacaa ctcttttgga agtaccatcc ctcaaaaggt gggaaggcta     360

tttaggcttc agtacttgaa catgagctat aatcttctcg aaggaaggat tccgtctagt     420

ctttctaact gctctagact gtcgaccgtt gatttatcgt caaaccatct tggacatggt     480

gttccttcag aactaggttc actttctaag cttgccattc tggatcttag caaaaacaac     540

cttactggaa attttcctgc atctttagga aacttgacgt cacttcagaa gcttgacttt 600

gcatataacc agatgagagg tgagattcca gacgaagtag ctagattgac tcaaatggtg 660

tttttccaaa tagcactgaa tagtttttca ggtggttttc ctcctgcatt gtacaacatc 720

tcctctcttg agtctctatc tctagctgac aatagctttt cgggtaatct tagggctgat 780

tttggttatc ttctaccaaa tctaagaaga cttcttttgg gaacaaatca gttcactgga 840

gctattccca aaacacttgc caatatctca agccttgaaa ggtttgatat ctcatctaat 900

tacctgtctg gtagtatccc tttgagcttt ggaaagttac gtaatctgtg gtggttaggg 960

attcgtaata actctcttgg aaataactcg tccagtggtc ttgaatttat tggggctgtg 1020

gcgaactgca ctcaattaga gtacttagat gttggttaca atagacttgg aggtgagctt 1080

cctgcttcta tagccaatct gtccactaca ttgactagtc tgttccttgg acaaaatctt 1140

atctctggaa ccattcctca tgacatcggg aatcttgtaa gcctgcaaga actcagctta 1200

gaaacaaata tgttgagtgg agaacttccc gtctctttcg ggaagctttt gaacttgcag 1260

gttgtggatc tgtattcaaa tgcaatatcg ggggaaatac catcttattt tggcaacatg 1320

actcggttgc agaagctcca tttgaatagc aatagtttcc acggaagaat ccctcagagt 1380

cttggacgtt gtcgatactt gctagacctg tggatggata caaataggtt gaatgggact 1440

atacctcagg aaatactgca aattccatcc ctcgcttaca tagatttgtc aaacaatttc 1500

ttgacaggcc attttccaga agaagttgga aagttagaac ttcttgttgg actaggtgct 1560

tcgtacaaca aattatcagg aaagatgcca caagctatag ggggttgtct ttcgatggaa 1620

tttctcttta tgcaaggaaa ttcgtttgac ggagccattc cagatataag ccggttggta 1680

agcctaaaga atgttgactt ctccaacaac aatctctctg gccggatacc tcgatatctg 1740

gccagtcttc cttcgctgcg aaatctgaat ctttctatga acaagtttga gggaagggtg 1800

ccaacaacag gagtgtttcg aaatgctaca gcagtttctg tttttggtaa cacaaatatt 1860

tgcggaggcg tccgagaaat gcaactaaag ccatgcattg tacaggcatc tccaaggaag 1920

agaaagcctc tgtcagttag aaagaaagtt gtcagtggta tttgtatagg tatagcttcg 1980

cttttgttaa tcataattgt ggcttctctg tgttggttca tgaagaggaa aaagaaaaac 2040

aatgccagtg atggtaaccc atctgattct actactttgg ggatgttcca tgagaaggta 2100

agttatgaag agcttcatag tgcaacaagt cgcttctctt caaccaattt gattggttca 2160

ggcaatttcg gtaatgtgtt taaaggattg cttggccctg agaataaact cgtcgcggtt 2220

aaagttttga acctcctaaa gcatggagcg acgaaaagct ttatggcgga atgtgaaacc 2280

ttcaagggta tacgacatcg taaccttgta aaactgataa cggtttgttc aagccttgat 2340

tccgagggaa atgatttcag agctctggtc tatgagttca tgccaaaagg aagtctggat 2400

atgtggctgc agctagaaga tctggaaagg gtaaacgatc actcgagatc tttaacaccc 2460

gcagagaaac tcaacatagc aatagatgtg gcttcagctt tggagtatct gcacgttcat 2520

tgtcatgacc ctgtagctca ctgtgatatt aagccaagca acattcttct agacgatgat 2580

ctgactgctc atgttagtga ctttggtttg gctcagctcc tctataaata cgatcgagaa 2640

tcctttctaa accagtttag ttctgctggt gtcagaggca ccattggcta tgccgcgcca 2700

gagtatggaa tgggaggcca accatcaata caaggagatg tgtacagctt cggaattcta 2760

cttttggaga tgtttagtgg aaagaaacca acagatgaat catttgcagg cgattataac 2820

ctccacagct acacaaagtc tatattatcg ggttgcacga gcagtggagg cagcaacgcc 2880

attgatgagg ggttgagact ggttttgcag gtggggataa agtgttctga agaatatccg 2940

agggatagga tgagaacgga tgaagcagta cgagaattaa tctcaataag atctaagttc 3000

ttcagttcca agacgactat tacagagagt cctcgagatg ctccgcaaag ttctcctcag 3060

gaatggatgt taaatacgga catgcatact atg 3093

<210> 12
<211> 2670
<212> DNA
<213> Arabidopsis thaliana

<400> 12
atgtcaaagg cgcttttgca tttgcatttt ctttctctgt ttttactctg ttgtgtctgc 60

cattcaagta tttttacttt aaattttcat tttactggta ttgtggcctg tcgtccccac 120

cagattcaag ccttcacgaa gttcacgaac gagtttgaca cccgcggttg caacaacagt 180

gacaccttta atggagtctg gtgcgataac tcgacgggtg cggtcgcagt gctacaacta 240

aggaagtgtc tcagtggaac tttgaagtcc aacagtagcc tcttcgggtt tcatcaactc 300

cgttacgttg atctccaaaa caacaactta acctcctctt cactcccttc cgggtttgga 360

aatctcaaaa gattagaggg cttgtttctt tcctctaatg gcttcctcgg ccaagttcct 420

tcctcattca gtaacctaac aatgcttgct caattagacc tttcttataa caagctcact 480

ggtagtttcc cacttgtccg gggtctaaga aagctcattg ttttagacct ttcatataat 540

cacttctccg gaactctgaa tccaaacagt agcctctttg agttgcacca gctacgttac 600

cttaatctcg cttttaacaa ctttagttcc tcactccctt ccaaatttgg caatctccat 660

agattagaga acttgattct ttcctctaat ggcttttctg gtcaagttcc ttccacaatt 720

agtaacctga cccggttaac taagttgtac cttgaccaaa acaagctcac tagtagtttc 780

ccacttgtac agaatctaac caacctctac gaattagacc tttcatataa taagttcttt 840

ggagtcatcc cttcttctct cctcactctg cctttcttag cacatcttgc tctacgtgaa 900

aacaatcttg ctggttctgt tgaagtttca aactcctcta cctcatctag gctcgaaatc 960

atgtatctcg ggtctaacca ttttgaagga caaatcctag agcctatctc aaagctcatc 1020

aacctcaagc atcttgacct ttctttccta aacacaagct acccaataga cttaaaactc 1080

ttctcctccc tcaaatcttt gcggtctctg gatctttctg gaaacagtat atcttcggcc 1140

agtttaagtt cagattcata catcccgttg accctggaaa tgttgacctt gaggcactgt 1200

gacatcaacg agttccccaa catcttaaag accctcaagg agttggtgta tatagacata 1260

tccaacaaca gaatgaaagg gaagatccct gagtggttat ggagcctacc tcttctacag 1320

tcagtaactc ttggaaataa ttatttcaca ggtttccaag gttcggcgga gattttagta 1380

aattcatcag tgctgctatt atatttggat tcaaacaatt ttgaaggagc acttcctgat 1440

ctaccactct ctatcaaagg cttcggtgtg gctagtaata gtttcacaag tgagatacct 1500

ctttcaatct gcaaccgaag ctctctcgct gctattgatc tatcctacaa caacttcact 1560

ggtcccattc ctccatgttt gcgtaatttg gaacttgtgt atctccggaa caacaacctg 1620

gaaggaagta tccctgacgc gctctgtgat ggtgcctctt tacggacact cgatgttagc 1680

cataaccgat taacagggaa gcttccaagg tcttttgtaa actgctcatc gctaaagttt 1740

ctaagcgtta taaacaacag aattgaagat acgtttccgt tctggctcaa agctttaccg 1800

aatttgcaag tccttactct tcgctcaaac agattttatg gtcctatatc tcctccgcat 1860

caaggtcctc tcgggtttcc agagctgcgg atatttgaaa tatctgacaa taagtttaca 1920

ggaagcttgc caccaaacta ctttgtgaac tggaaagcat catcacgcac gatgaatcaa 1980

gatgggggtt tatatatggt atacgaggag aagctgttcg acgaaggcgg ctatggttat 2040

acagatgctt tagatttgca atacaaaggt ctacacatgg agcaagctaa ggccctcact 2100

tcctacgccg ccattgattt ttctggaaat agactcgaag gacagattcc tgaatctatt 2160

ggtctcttga aggcactgat tgcagtcaat atatcaaaca acgccttcac aggccatatt 2220

cccctgtcta tggccaatct tgagaatctc gagtcactag acatgtcaag aaaccaactc 2280

tcggggacta ttcctaatgg actagggagc atttcgtttt tggcgtacat caacgtgtct 2340

cacaaccaac tcacgggtga aataccacaa ggaacacaga ttactgggca atctaaatct 2400

tccttcgaag ggaatgcagg gctttgtggt cttcctctca aggaaagttg cttcggcact 2460

ggtgcacctc cgatgtatca ccaaaagcaa gaagacaaag aagaagaaga agaagaagaa 2520

gaagaagaag aagaagtgtt gaacgggaga gcagtggcaa tagggtatgg atctggatta 2580

ttgcttggat tggcaatagc acaagttatt gcttcataca aaccggagtg gcttgtcaag 2640

ataattggtc tgaataaacg cagaaagcgt 2670

<210> 13
<211> 1923
<212> DNA
<213> Arabidopsis thaliana

<400> 13
atggctgttc ccacgggaag cgccaatctc tttctccggc ctctcatcct cgccgttctc 60

tcctttcttc ttctctcttc attcgtctcc tctgttgagt ggctcgatat cgattcctca 120

gatctcaaag ctcttcaggt catcgaaaca gagctcggag tcaacagtca acgttcttcg 180

gccagtgatg tcaacccttg cggtcgccga ggagttttct gcgagagacg gcactctgcc 240

accaccggag aatacgttct ccgtgtcacg aggctcgtct accgatccag gagcttgacg 300

gggactatct ctccggtcat cggaatgttg tcggagctca aggaacttac tttgtctaat 360

aaccagttgg tcaacgctgt tccggtggat atcttaagct gtaagcaact tgaagttctt 420

gatcttcgga aaaatagatt ttccggtcag attccgggaa acttttctag tttgagccgt 480

cttcgaatcc ttgatctctc ttcgaacaag ttatccggga acttgaattt cttgaagaat 540

ctacgcaatc tggagaatct ctctgttgca aacaatctct tctcaggcaa aatcccggaa 600

caaattgtgt cgttccacaa tctccggttc tttgatttct ccggaaaccg gtatttggaa 660

ggtccggctc cggttatgag cagtatcaag cttcagacat ctccacatca aacaagacac 720

attcttgctg aaactccaac ttcaagtccc acgaacaaac ccaacaacag cactacatca 780

aaagctccaa agggagctcc aaaaccaggg aagttgaaga agaagaagaa gaagagcaag 840

aagaagaaag tagcggcatg gatcttaggg tttgtcgttg gagccatagg aggaacaatc 900

tctgggtttg tcttctcggt gttgttcaaa ttgattattc aagcaataag aggatcagaa 960

aaaccaccag gtccttccat attcagccca ttgatcaaga aagctgaaga tttagctttc 1020

ttggagaatg aagaagcgtt agcttctctg gagatcatag gaagaggagg ttgtggagaa 1080

gttttcaaag ctgaactacc aggaagcaat gggaagatca tagctgtgaa gaaagtgatc 1140

caaccgccta aagacgccga tgaactaaca gatgaagatt ccaagtttct gaacaagaaa 1200

atgaggcaaa ttagatccga gatcaacaca gtcggccata tccggcaccg gaatcttctc 1260

ccattgttag cacacgtgtc aagacccgag tgccactacc tcgtttacga gtacatggaa 1320

aaggggagtt tgcaagatat attgactgac gtacaagctg gaaatcaaga actaatgtgg 1380

ccagcgaggc acaagattgc tttaggtata gctgcagggc ttgagtacct tcacatggat 1440

cataacccac gaatcattca cagagactta aagccagcca atgttcttct tgatgatgac 1500

atggaagcca gaatttcaga tttcggactg gcaaaggcaa tgccagatgc agtcacacat 1560

attacaacct cgcatgttgc aggtactgtg ggatacatag caccagagtt ttatcaaacc 1620

cacaagttca cagataaatg tgatatctat agttttggag tgattcttgg gattctggtg 1680

attgggaagc ttccttcaga tgagtttttt cagcatactg acgagatgag tctgataaag 1740

tggatgagga acataataac atcggaaaat cctagccttg cgattgatcc gaagttgatg 1800

gatcaaggat tcgatgaaca gatgcttctg gttctgaaga tcgcctgtta ctgtactttg 1860

gatgatccaa aacagaggcc gaacagtaaa gatgtcagga ccatgttgtc ccagatcaag 1920

cac 1923

<210> 14
<211> 3063
<212> DNA
<213> Arabidopsis thaliana

<400> 14
atgggcaaaa gaactaatcc aagacatttc cttgttactt ggtctttact gctcctagag 60

acagcttttg gattaacttc aagagaagtt aacaagaccc tttgtataga aaaggagaga 120

ggtgcccttc ttgagtttaa aagaggcctt aacgacgatt ttggtcgttt atctacctgg 180

ggtgatgaag aagaatgctg caattggaag ggtattgaat gtgacaaaag aacaggtcat 240

gttattgttc ttgatctcca cagtgaggtt acttgtccag gccatgcttg ttttgctcca 300

atattgacag gtaaagttag tccttctcta cttgagttgg agtatttgaa tttcttggac 360

ctcagtgtta atggatttga aaatagtgag ataccaagat tcataggctc ccttaagaga 420

ctggagtact taaacctttc atcttctgat ttttctggtg aaattccagc acagttccag 480

aatctaactt ctttgaggat tcttgatctc ggaaacaata atcttatagt aaaggacctt 540

gtgtggcttt ctcatctctc ctctctagaa ttcttgcgcc ttggtggtaa cgatttccaa 600

gcaagaaact ggtttcgaga gataactaag gtcccttcat tgaaagaatt ggacttgagt 660

gtttgtggac tctctaaatt cgttccgtct ccagctgatg tagctaattc atctttgatc 720

tctctttctg ttcttcattt atgttgtaat gagttttcta cttcatctga atatagctgg 780

ttattcaatt ttagcacaag cctaactagc atagaccttt ctcataatca actcagtcgt 840

caaattgatg atcgctttgg gagcttgatg tatcttgaac atcttaatct tgctaataat 900

tttggggctg aaggtggggt tccaagttct tttgggaatt tgacacgtct acattatctg 960

gacatgtcta acactcagac ataccaatgg cttcctgagt tgtttctcag gttatcaggt 1020

agtaggaaat cacttgaggt tttgggattg aacgacaact cgttgtttgg ttcaattgtt 1080

aatgtgccaa gattttcatc cttgaagaaa ttatacctgc agaagaatat gctgaatggt 1140

tttttcatgg aaagagtggg acaagtttcg agcctcgagt atctagactt gtctgataac 1200

caaatgagag ggccattacc agatttagca ctttttccat cattaagaga gttgcatctt 1260

ggctctaatc aatttcaagg gaggatacca caaggtattg gaaaactttc acagcttaga 1320

atttttgacg tctcgtccaa tagattagag ggtttaccag aaagtatggg gcaactatca 1380

aacctggaaa ggtttgatgc ttcttataat gtcctgaagg gtacaatcac agagtcccac 1440

ttttcaaacc tctccagttt agtagactta gacctatcct tcaacttgtt gtctttgaac 1500

acgagattcg attgggttcc tccttttcaa ctacaattta taaggcttcc atcttgcaat 1560

atgggacctt cttttccgaa atggctacaa actcagaata actacactct tcttgatatt 1620

tctcttgcga atatatcaga catgctacca agttggttct ccaatcttcc tcccgagctc 1680

aagattctga atctctctaa caaccacatc agtggcagag tttcggagtt catagtgagt 1740

aaacaagatt acatgattat agatttaagt tctaacaact tttcaggaca tttgccacta 1800

gttcctgcca atatacagat cttttacctt cataaaaatc acttctctgg atccatttct 1860

tccatttgta gaaatacaat aggagctgcc acttccatcg acttgtcacg caaccaattt 1920

tcaggagaag ttcctgattg ttggatgaat atgagtaatc tagctgttct aaatctagcc 1980

tacaacaatt tctccggaaa agttccacaa tcattaggct ccttgacaaa tttggaggcg 2040

ttatacatac gtcagaacag ctttagagga atgttgcctt ctttttcaca atgtcagctg 2100

ctgcaaatct tggatattgg agggaataag ttgactggaa gaatcccagc atggataggg 2160

actgacctac tccaattgcg cattctaagc ctacgttcca acaaattcga tggcagcatt 2220

ccatcactta tctgccagct tcaatttctt cagatactgg acctttcaga aaatggatta 2280

tctgggaaaa ttccacagtg cctcaacaac tttaccatat tgcgtcaaga aaatggctct 2340

ggtgagtcaa tggattttaa agtccgttat gactatattc caggatctta cttgtacata 2400

ggtgatttac tgattcaatg gaaaaaccag gagtccgagt acaagaatgc tttactatat 2460

ctgaagatca ttgatctttc aagtaataaa ttagttggag gtatccctaa agagatagct 2520

gaaatgagag gattgagatc attgaacctc tcgagaaatg atcttaatgg aactgtcgtt 2580

gaaggaatag gtcaaatgaa gttgttggag tccctcgact tgtcaagaaa ccaactctct 2640

ggcatgatcc ctcagggcct ttctaacttg actttcttta gtgtgttgga cttatcgaac 2700

aaccacttat caggaagaat tccatcaagc actcaactgc agagtttcga tagatcatcc 2760

tatagtggca atgctcaact ctgcggtcct cctcttgaag agtgtcctgg atatgctccc 2820

cctatcgatc gtggaagcaa caccaatcca caagaacatg atgatgatga tgagttctca 2880

tctctggagt tttatgtatc aatggtgcta ggtttcttcg tcacgttctg ggggaattta 2940

ggctgtttga ttgtcaaccg ttcgtggagg aatgcctact tcacattctt aacagacatg 3000

aagagttggc tccatatgac atcaagagtc tgctttgcaa gactgaaggg aaagctaagg 3060

aac 3063

<210> 15
<211> 2664
<212> DNA
<213> Artificial

<220>
<223> RLP23tmSOBIR1

<400> 15

atgtcaaagg cgcttttgca tttgcatttt ctttctctgt ttttactctg ttgtgtctgc 60

cattcaagta tttttacttt aaattttcat tttactggta ttgtggcctg tcgtccccac 120

cagattcaag ccttcacgaa gttcacgaac gagtttgaca cccgcggttg caacaacagt 180

gacaccttta atggagtctg gtgcgataac tcgacgggtg cggtcgcagt gctacaacta 240

aggaagtgtc tcagtggaac tttgaagtcc aacagtagcc tcttcgggtt tcatcaactc 300

cgttacgttg atctccaaaa caacaactta acctcctctt cactcccttc cgggtttgga 360

aatctcaaaa gattagaggg cttgtttctt tcctctaatg gcttcctcgg ccaagttcct 420

tcctcattca gtaacctaac aatgcttgct caattagacc tttcttataa caagctcact 480

ggtagtttcc cacttgtccg gggtctaaga aagctcattg ttttagacct ttcatataat 540

cacttctccg gaactctgaa tccaaacagt agcctctttg agttgcacca gctacgttac 600

cttaatctcg cttttaacaa ctttagttcc tcactccctt ccaaatttgg caatctccat 660

agattagaga acttgattct ttcctctaat ggcttttctg gtcaagttcc ttccacaatt 720

agtaacctga cccggttaac taagttgtac cttgaccaaa acaagctcac tagtagtttc 780

ccacttgtac agaatctaac caacctctac gaattagacc tttcatataa taagttcttt 840

ggagtcatcc cttcttctct cctcactctg cctttcttag cacatcttgc tctacgtgaa 900

aacaatcttg ctggttctgt tgaagtttca aactcctcta cctcatctag gctcgaaatc 960

atgtatctcg ggtctaacca ttttgaagga caaatcctag agcctatctc aaagctcatc 1020

aacctcaagc atcttgacct ttctttccta aacacaagct acccaataga cttaaaactc 1080

ttctcctccc tcaaatcttt gcggtctctg gatctttctg gaaacagtat atcttcggcc 1140

agtttaagtt cagattcata catcccgttg accctggaaa tgttgacctt gaggcactgt 1200

gacatcaacg agttccccaa catcttaaag accctcaagg agttggtgta tatagacata 1260

tccaacaaca gaatgaaagg gaagatccct gagtggttat ggagcctacc tcttctacag 1320

tcagtaactc ttggaaataa ttatttcaca ggtttccaag gttcggcgga gattttagta 1380

aattcatcag tgctgctatt atatttggat tcaaacaatt ttgaaggagc acttcctgat 1440

ctaccactct ctatcaaagg cttcggtgtg gctagtaata gtttcacaag tgagatacct 1500

ctttcaatct gcaaccgaag ctctctcgct gctattgatc tatcctacaa caacttcact 1560

ggtcccattc ctccatgttt gcgtaatttg gaacttgtgt atctccggaa caacaacctg 1620

gaaggaagta tccctgacgc gctctgtgat ggtgcctctt tacggacact cgatgttagc 1680

cataaccgat taacagggaa gcttccaagg tcttttgtaa actgctcatc gctaaagttt 1740

ctaagcgtta taaacaacag aattgaagat acgtttccgt tctggctcaa agctttaccg 1800

aatttgcaag tccttactct tcgctcaaac agattttatg gtcctatatc tcctccgcat 1860

caaggtcctc tcgggtttcc agagctgcgg atatttgaaa tatctgacaa taagtttaca 1920

ggaagcttgc caccaaacta ctttgtgaac tggaaagcat catcacgcac gatgaatcaa 1980

gatgggggtt tatatatggt atacgaggag aagctgttcg acgaaggcgg ctatggttat 2040

acagatgctt tagatttgca atacaaaggt ctacacatgg agcaagctaa ggccctcact 2100

tcctacgccg ccattgattt ttctggaaat agactcgaag gacagattcc tgaatctatt 2160

ggtctcttga aggcactgat tgcagtcaat atatcaaaca acgccttcac aggccatatt 2220

cccctgtcta tggccaatct tgagaatctc gagtcactag acatgtcaag aaaccaactc 2280

tcggggacta ttcctaatgg actagggagc atttcgtttt tggcgtacat caacgtgtct 2340

cacaaccaac tcacgggtga aataccacaa ggaacacaga ttactgggca atctaaatct 2400

tccttcgaag ggaatgcagg gctttgtggt cttcctctca aggaaagttg cttcggcact 2460

ggtgcacctc cgatgtatca ccaaaagcaa gaagacaaag aagaagaaga agaagaagaa 2520

gaagaagaag aagaagtagc ggcatggatc ttagggtttg tcgttggagc cataggagga 2580

acaatctctg ggtttgtctt ctcggtgttg ttcaaaccgg agtggcttgt caagataatt 2640

ggtctgaata aacgcagaaa gcgt 2664

<210> 16
<211> 3609
<212> DNA
<213> Artificial

<220>
<223> RLP23tmkSOBIR1

<400> 16
atgtcaaagg cgcttttgca tttgcatttt ctttctctgt tttactctgt ttgtgtctgc 60

cattcaagta tttttacttt aaattttcat tttactggta ttgtggcctg tcgtccccac 120

cagattcaag ccttcacgaa gttcacgaac gagtttgaca cccgcggttg caacaacagt 180

gacaccttta atggagtctg gtgcgataac tcgacgggtg cggtcgcagt gctacaacta 240

aggaagtgtc tcagtggaac tttgaagtcc aacagtagcc tcttcgggtt tcatcaactc 300

cgttacgttg atctccaaaa caacaactta acctcctctt cactcccttc cgggtttgga 360

aatctcaaaa gattagaggg cttgtttctt tcctctaatg gcttcctcgg ccaagttcct 420

tcctcattca gtaacctaac aatgcttgct caattagacc tttcttataa caagctcact 480

ggtagtttcc cacttgtccg gggtctaaga aagctcattg ttttagacct ttcatataat 540

cacttctccg gaactctgaa tccaaacagt agcctctttg agttgcacca gctacgttac 600

cttaatctcg cttttaacaa ctttagttcc tcactccctt ccaaatttgg caatctccat 660

agattagaga acttgattct ttcctctaat ggcttttctg gtcaagttcc ttccacaatt 720

agtaacctga cccggttaac taagttgtac cttgaccaaa acaagctcac tagtagtttc 780

ccacttgtac agaatctaac caacctctac gaattagacc tttcatataa taagttcttt 840

ggagtcatcc cttcttctct cctcactctg cctttcttag cacatcttgc tctacgtgaa 900

aacaatcttg ctggttctgt tgaagtttca aactcctcta cctcatctag gctcgaaatc 960

atgtatctcg ggtctaacca ttttgaagga caaatcctag agcctatctc aaagctcatc 1020

aacctcaagc atcttgacct ttctttccta aacacaagct acccaataga cttaaaactc 1080

ttctcctccc tcaaatcttt gcggtctctg gatctttctg gaaacagtat atcttcggcc 1140

agtttaagtt cagattcata catcccgttg accctggaaa tgttgacctt gaggcactgt 1200

gacatcaacg agttccccaa catcttaaag accctcaagg agttggtgta tatagacata 1260

tccaacaaca gaatgaaagg gaagatccct gagtggttat ggagcctacc tcttctacag 1320

tcagtaactc ttggaaataa ttatttcaca ggtttccaag gttcggcgga gattttagta 1380

aattcatcag tgctgctatt atatttggat tcaaacaatt ttgaaggagc acttcctgat 1440

ctaccactct ctatcaaagg cttcggtgtg gctagtaata gtttcacaag tgagatacct 1500

ctttcaatct gcaaccgaag ctctctcgct gctattgatc tatcctacaa caacttcact 1560

ggtcccattc ctccatgttt gcgtaatttg gaacttgtgt atctccggaa caacaacctg 1620

gaaggaagta tccctgacgc gctctgtgat ggtgcctctt tacggacact cgatgttagc 1680

cataaccgat taacagggaa gcttccaagg tcttttgtaa actgctcatc gctaaagttt 1740

ctaagcgtta taaacaacag aattgaagat acgtttccgt tctggctcaa agctttaccg 1800

aatttgcaag tccttactct tcgctcaaac agattttatg gtcctatatc tcctccgcat 1860

caaggtcctc tcgggtttcc agagctgcgg atatttgaaa tatctgacaa taagtttaca 1920

ggaagcttgc caccaaacta ctttgtgaac tggaaagcat catcacgcac gatgaatcaa 1980

gatgggggtt tatatatggt atacgaggag aagctgttcg acgaaggcgg ctatggttat 2040

acagatgctt tagatttgca atacaaaggt ctacacatgg agcaagctaa ggccctcact 2100

tcctacgccg ccattgattt ttctggaaat agactcgaag gacagattcc tgaatctatt 2160

ggtctcttga aggcactgat tgcagtcaat atatcaaaca acgccttcac aggccatatt 2220

cccctgtcta tggccaatct tgagaatctc gagtcactag acatgtcaag aaaccaactc 2280

tcggggacta ttcctaatgg actagggagc atttcgtttt tggcgtacat caacgtgtct 2340

cacaaccaac tcacgggtga aataccacaa ggaacacaga ttactgggca atctaaatct 2400

tccttcgaag ggaatgcagg gctttgtggt cttcctctca aggaaagttg cttcggcact 2460

ggtgcacctc cgatgtatca ccaaaagcaa gaagacaaag aagaagaaga agaagaagaa 2520

gaagaagaag aagaagtagc ggcatggatc ttagggtttg tcgttggagc cataggagga 2580

acaatctctg ggtttgtctt ctcggtgttg ttcaaattga ttattcaagc aataagagga 2640

tcagaaaaac caccaggtcc ttccatattc agcccattga tcaagaaagc tgaagattta 2700

gctttcttgg agaatgaaga agcgttagct tctctggaga tcataggaag aggaggttgt 2760

ggagaagttt tcaaagctga actaccagga agcaatggga agatcatagc tgtgaagaaa 2820

gtgatccaac cgcctaaaga cgccgatgaa ctaacagatg aagattccaa gtttctgaac 2880

aagaaaatga ggcaaattag atccgagatc aacacagtcg gccatatccg gcaccggaat 2940

cttctcccat tgttagcaca cgtgtcaaga cccgagtgcc actacctcgt ttacgagtac 3000

atggaaaagg ggagtttgca agatatattg actgacgtac aagctggaaa tcaagaacta 3060

atgtggccag cgaggcacaa gattgcttta ggtatagctg cagggcttga gtaccttcac 3120

atggatcata acccacgaat cattcacaga gacttaaagc cagccaatgt tcttcttgat 3180

gatgacatgg aagccagaat ttcagatttc ggactggcaa aggcaatgcc agatgcagtc 3240

acacatatta caacctcgca tgttgcaggt actgtgggat acatagcacc agagttttat 3300

caaacccaca agttcacaga taaatgtgat atctatagtt ttggagtgat tcttgggatt 3360

ctggtgattg ggaagcttcc ttcagatgag ttttttcagc atactgacga gatgagtctg 3420

ataaagtgga tgaggaacat aataacatcg gaaaatccta gccttgcgat tgatccgaag 3480

ttgatggatc aaggattcga tgaacagatg cttctggttc tgaagatcgc ctgttactgt 3540

actttggatg atccaaaaca gaggccgaac agtaaagatg tcaggaccat gttgtcccag 3600

atcaagcac 3609

<210> 17
<211> 3072
<212> DNA
<213> Artificial

<220>
<223> EIX2tmSOBIR1

<400> 17
atgggcaaaa gaactaatcc aagacatttc cttgttactt ggtctttact gctcctagag 60

acagcttttg gattaacttc aagagaagtt aacaagaccc tttgtataga aaaggagaga 120

ggtgcccttc ttgagtttaa aagaggcctt aacgacgatt ttggtcgttt atctacctgg 180

ggtgatgaag aagaatgctg caattggaag ggtattgaat gtgacaaaag aacaggtcat 240

gttattgttc ttgatctcca cagtgaggtt acttgtccag gccatgcttg ttttgctcca 300

atattgacag gtaaagttag tccttctcta cttgagttgg agtatttgaa tttcttggac 360

ctcagtgtta atggatttga aaatagtgag ataccaagat tcataggctc ccttaagaga 420

ctggagtact taaacctttc atcttctgat ttttctggtg aaattccagc acagttccag 480

aatctaactt ctttgaggat tcttgatctc ggaaacaata atcttatagt aaaggacctt 540

gtgtggcttt ctcatctctc ctctctagaa ttcttgcgcc ttggtggtaa cgatttccaa 600

gcaagaaact ggtttcgaga gataactaag gtcccttcat tgaaagaatt ggacttgagt 660
gtttgtggac tctctaaatt cgttccgtct ccagctgatg tagctaattc atctttgatc 720
tctctttctg ttcttcattt atgttgtaat gagttttcta cttcatctga atatagctgg 780
ttattcaatt ttagcacaag cctaactagc atagaccttt ctcataatca actcagtcgt 840
caaattgatg atcgctttgg gagcttgatg tatcttgaac atcttaatct tgctaataat 900
tttggggctg aaggtggggt tccaagttct tttgggaatt tgacacgtct acattatctg 960
gacatgtcta acactcagac ataccaatgg cttcctgagt tgtttctcag gttatcaggt 1020
agtaggaaat cacttgaggt tttgggattg aacgacaact cgttgtttgg ttcaattgtt 1080
aatgtgccaa gattttcatc cttgaagaaa ttatacctgc agaagaatat gctgaatggt 1140
tttttcatgg aaagagtggg acaagtttcg agcctcgagt atctagactt gtctgataac 1200
caaatgagag ggccattacc agatttagca ctttttccat cattaagaga gttgcatctt 1260
ggctctaatc aatttcaagg gaggatacca caaggtattg gaaaactttc acagcttaga 1320
atttttgacg tctcgtccaa tagattagag ggtttaccag aaagtatggg gcaactatca 1380
aacctggaaa ggtttgatgc ttcttataat gtcctgaagg gtacaatcac agagtcccac 1440
ttttcaaacc tctccagttt agtagactta gacctatcct tcaacttgtt gtctttgaac 1500
acgagattcg attgggttcc tccttttcaa ctacaattta taaggcttcc atcttgcaat 1560
atgggacctt cttttccgaa atggctacaa actcagaata actacactct tcttgatatt 1620
tctcttgcga atatatcaga catgctacca agttggttct ccaatcttcc tcccgagctc 1680
aagattctga atctctctaa caaccacatc agtggcagag tttcggagtt catagtgagt 1740
aaacaagatt acatgattat agatttaagt tctaacaact tttcaggaca tttgccacta 1800
gttcctgcca atatacagat cttttacctt cataaaaatc acttctctgg atccatttct 1860
tccatttgta gaaatacaat aggagctgcc acttccatcg acttgtcacg caaccaattt 1920
tcaggagaag ttcctgattg ttggatgaat atgagtaatc tagctgttct aaatctagcc 1980
tacaacaatt tctccggaaa agttccacaa tcattaggct ccttgacaaa tttggaggcg 2040
ttatacatac gtcagaacag ctttagagga atgttgcctt ctttttcaca atgtcagctg 2100
ctgcaaatct tggatattgg agggaataag ttgactggaa gaatcccagc atggataggg 2160
actgacctac tccaattgcg cattctaagc ctacgttcca acaaattcga tggcagcatt 2220
ccatcactta tctgccagct tcaatttctt cagatactgg acctttcaga aaatggatta 2280
tctgggaaaa ttccacagtg cctcaacaac tttaccatat tgcgtcaaga aaatggctct 2340
ggtgagtcaa tggattttaa agtccgttat gactatattc caggatctta cttgtacata 2400
ggtgatttac tgattcaatg gaaaaaccag gagtccgagt acaagaatgc tttactatat 2460
ctgaagatca ttgatctttc aagtaataaa ttagttggag gtatccctaa agagatagct 2520

gaaatgagag gattgagatc attgaacctc tcgagaaatg atcttaatgg aactgtcgtt 2580

gaaggaatag gtcaaatgaa gttgttggag tccctcgact tgtcaagaaa ccaactctct 2640

ggcatgatcc ctcagggcct ttctaacttg acttttctta gtgtgttgga cttatcgaac 2700

aaccacttat caggaagaat tccatcaagc actcaactgc agagtttcga tagatcatcc 2760

tatagtggca atgctcaact ctgcggtcct cctcttgaag agtgtcctgg atatgctccc 2820

cctatcgatc gtggaagcaa caccaatcca caagaacatg atgatgatga tgagttctca 2880

tctctggagg tagcggcatg gatcttaggg tttgtcgttg gagccatagg aggaacaatc 2940

tctgggtttg tcttctcggt gttgttccgt tcgtggagga atgcctactt cacattctta 3000

acagacatga agagttggct ccatatgaca tcaagagtct gctttgcaag actgaaggga 3060

aagctaagga ac 3072

<210> 18
<211> 3963
<212> DNA
<213> Artificial

<220>
<223> EIX2tmkSOBIR1

<400> 18
atgggcaaaa gaactaatcc aagacatttc cttgttactt ggtctttact gctcctagag 60

acagcttttg gattaacttc aagagaagtt aacaagaccc tttgtataga aaaggagaga 120

ggtgcccttc ttgagtttaa aagaggcctt aacgacgatt ttggtcgttt atctacctgg 180

ggtgatgaag aagaatgctg caattggaag ggtattgaat gtgacaaaag aacaggtcat 240

gttattgttc ttgatctcca cagtgaggtt acttgtccag gccatgcttg ttttgctcca 300

atattgacag gtaaagttag tccttctcta cttgagttgg agtatttgaa tttcttggac 360

ctcagtgtta atggatttga aaatagtgag ataccaagat tcataggctc ccttaagaga 420

ctggagtact taaacctttc atcttctgat ttttctggtg aaattccagc acagttccag 480

aatctaactt ctttgaggat tcttgatctc ggaaacaata atcttatagt aaaggacctt 540

gtgtggcttt ctcatctctc ctctctagaa ttcttgcgcc ttggtggtaa cgatttccaa 600

gcaagaaact ggtttcgaga gataactaag gtcccttcat tgaaagaatt ggacttgagt 660

gtttgtggac tctctaaatt cgttccgtct ccagctgatg tagctaattc atctttgatc 720

tctctttctg ttcttcattt atgttgtaat gagttttcta cttcatctga atatagctgg 780

ttattcaatt ttagcacaag cctaactagc atagaccttt ctcataatca actcagtcgt 840

caaattgatg atcgctttgg gagcttgatg tatcttgaac atcttaatct tgctaataat 900

tttggggctg aaggtggggt tccaagttct tttgggaatt tgacacgtct acattatctg 960

gacatgtcta acactcagac ataccaatgg cttcctgagt tgtttctcag gttatcaggt 1020
agtaggaaat cacttgaggt tttgggattg aacgacaact cgttgtttgg ttcaattgtt 1080
aatgtgccaa gattttcatc cttgaagaaa ttatacctgc agaagaatat gctgaatggt 1140
tttttcatgg aaagagtggg acaagtttcg agcctcgagt atctagactt gtctgataac 1200
caaatgagag ggccattacc agatttagca ctttttccat cattaagaga gttgcatctt 1260
ggctctaatc aatttcaagg gaggatacca caaggtattg gaaaactttc acagcttaga 1320
atttttgacg tctcgtccaa tagattagag ggtttaccag aaagtatggg gcaactatca 1380
aacctggaaa ggtttgatgc ttcttataat gtcctgaagg gtacaatcac agagtcccac 1440
ttttcaaacc tctccagttt agtagactta gacctatcct tcaacttgtt gtctttgaac 1500
acgagattcg attgggttcc tccttttcaa ctacaattta taaggcttcc atcttgcaat 1560
atgggacctt cttttccgaa atggctacaa actcagaata actacactct tcttgatatt 1620
tctcttgcga atatatcaga catgctacca agttggttct ccaatcttcc tcccgagctc 1680
aagattctga atctctctaa caaccacatc agtggcagag tttcggagtt catagtgagt 1740
aaacaagatt acatgattat agatttaagt tctaacaact tttcaggaca tttgccacta 1800
gttcctgcca atatacagat cttttacctt cataaaaatc acttctctgg atccatttct 1860
tccatttgta gaaatacaat aggagctgcc acttccatcg acttgtcacg caaccaattt 1920
tcaggagaag ttcctgattg ttggatgaat atgagtaatc tagctgttct aaatctagcc 1980
tacaacaatt tctccggaaa agttccacaa tcattaggct ccttgacaaa tttggaggcg 2040
ttatacatac gtcagaacag ctttagagga atgttgcctt ctttttcaca atgtcagctg 2100
ctgcaaatct tggatattgg agggaataag ttgactggaa gaatcccagc atggataggg 2160
actgacctac tccaattgcg cattctaagc ctacgttcca acaaattcga tggcagcatt 2220
ccatcactta tctgccagct tcaatttctt cagatactgg acctttcaga aaatggatta 2280
tctgggaaaa ttccacagtg cctcaacaac tttaccatat tgcgtcaaga aaatggctct 2340
ggtgagtcaa tggattttaa agtccgttat gactatattc caggatctta cttgtacata 2400
ggtgatttac tgattcaatg gaaaaaccag gagtccgagt acaagaatgc tttactatat 2460
ctgaagatca ttgatctttc aagtaataaa ttagttggag gtatccctaa agagatagct 2520
gaaatgagag gattgagatc attgaacctc tcgagaaatg atcttaatgg aactgtcgtt 2580
gaaggaatag gtcaaatgaa gttgttggag tccctcgact tgtcaagaaa ccaactctct 2640
ggcatgatcc ctcagggcct ttctaacttg actttcttta gtgtgttgga cttatcgaac 2700
aaccacttat caggaagaat tccatcaagc actcaactgc agagtttcga tagatcatcc 2760
tatagtggca atgctcaact ctgcggtcct cctcttgaag agtgtcctgg atatgctccc 2820
cctatcgatc gtggaagcaa caccaatcca caagaacatg atgatgatga tgagttctca 2880

tctctggagg tagcggcatg gatcttaggg tttgtcgttg gagccatagg aggaacaatc 2940

tctgggtttg tcttctcggt gttgttcaaa ttgattattc aagcaataag aggatcagaa 3000

aaaccaccag gtccttccat attcagccca ttgatcaaga aagctgaaga tttagctttc 3060

ttggagaatg aagaagcgtt agcttctctg gagatcatag gaagaggagg ttgtggagaa 3120

gttttcaaag ctgaactacc aggaagcaat gggaagatca tagctgtgaa gaaagtgatc 3180

caaccgccta aagacgccga tgaactaaca gatgaagatt ccaagtttct gaacaagaaa 3240

atgaggcaaa ttagatccga gatcaacaca gtcggccata tccggcaccg gaatcttctc 3300

ccattgttag cacacgtgtc aagacccgag tgccactacc tcgtttacga gtacatggaa 3360

aaggggagtt tgcaagatat attgactgac gtacaagctg gaaatcaaga actaatgtgg 3420

ccagcgaggc acaagattgc tttaggtata gctgcagggc ttgagtacct tcacatggat 3480

cataacccac gaatcattca cagagactta aagccagcca atgttcttct tgatgatgac 3540

atggaagcca gaatttcaga tttcggactg gcaaaggcaa tgccagatgc agtcacacat 3600

attacaacct cgcatgttgc aggtactgtg ggatacatag caccagagtt ttatcaaacc 3660

cacaagttca cagataaatg tgatatctat agttttggag tgattcttgg gattctggtg 3720

attgggaagc ttccttcaga tgagtttttt cagcatactg acgagatgag tctgataaag 3780

tggatgagga acataataac atcggaaaat cctagccttg cgattgatcc gaagttgatg 3840

gatcaaggat tcgatgaaca gatgcttctg gttctgaaga tcgcctgtta ctgtactttg 3900

gatgatccaa aacagaggcc gaacagtaaa gatgtcagga ccatgttgtc ccagatcaag 3960

cac 3963

<210> 19
<211> 3015
<212> DNA
<213> Artificial

<220>
<223> EFRtmkSOBIR1

<400> 19
atgaagctgt ccttttcact tgttttcaat gctctcacgt tgcttcttca agtttgcatc 60

tttgctcaag ccaggttttc taatgagact gatatgcaag ctttgcttga gttcaagtct 120

caagtttctg aaaacaacaa gagagaggtc ttggcttcat ggaatcactc ctccccattt 180

tgtaattgga ttggagtcac atgtggccgc aggagagaaa gagttataag tttgaacctt 240

ggaggattca agttaaccgg tgtgatctca ccctccattg gtaatctctc ctttcttaga 300

ttacttaatc ttgcagacaa ctcttttgga agtaccatcc ctcaaaaggt gggaaggcta 360

tttaggcttc agtacttgaa catgagctat aatcttctcg aaggaaggat tccgtctagt 420

ctttctaact gctctagact gtcgaccgtt gatttatcgt caaaccatct tggacatggt 480

gttccttcag aactaggttc actttctaag cttgccattc tggatcttag caaaaacaac 540

cttactggaa attttcctgc atctttagga aacttgacgt cacttcagaa gcttgacttt 600

gcatataacc agatgagagg tgagattcca gacgaagtag ctagattgac tcaaatggtg 660

tttttccaaa tagcactgaa tagtttttca ggtggttttc ctcctgcatt gtacaacatc 720

tcctctcttg agtctctatc tctagctgac aatagctttt cgggtaatct tagggctgat 780

tttggttatc ttctaccaaa tctaagaaga cttcttttgg gaacaaatca gttcactgga 840

gctattccca aaacacttgc caatatctca agccttgaaa ggtttgatat ctcatctaat 900

tacctgtctg gtagtatccc tttgagcttt ggaaagttac gtaatctgtg gtggttaggg 960

attcgtaata actctcttgg aaataactcg tccagtggtc ttgaatttat tggggctgtg 1020

gcgaactgca ctcaattaga gtacttagat gttggttaca atagacttgg aggtgagctt 1080

cctgcttcta tagccaatct gtccactaca ttgactagtc tgttccttgg acaaaatctt 1140

atctctggaa ccattcctca tgacatcggg aatcttgtaa gcctgcaaga actcagctta 1200

gaaacaaata tgttgagtgg agaacttccc gtctctttcg ggaagctttt gaacttgcag 1260

gttgtggatc tgtattcaaa tgcaatatcg ggggaaatac catcttattt tggcaacatg 1320

actcggttgc agaagctcca tttgaatagc aatagtttcc acggaagaat ccctcagagt 1380

cttggacgtt gtcgatactt gctagacctg tggatggata caaataggtt gaatgggact 1440

atacctcagg aaatactgca aattccatcc ctcgcttaca tagatttgtc aaacaatttc 1500

ttgacaggcc attttccaga agaagttgga aagttagaac ttcttgttgg actaggtgct 1560

tcgtacaaca aattatcagg aaagatgcca caagctatag ggggttgtct ttcgatggaa 1620

tttctcttta tgcaaggaaa ttcgtttgac ggagccattc cagatataag ccggttggta 1680

agcctaaaga atgttgactt ctccaacaac aatctctctg gccggatacc tcgatatctg 1740

gccagtcttc cttcgctgcg aaatctgaat ctttctatga acaagtttga gggaagggtg 1800

ccaacaacag gagtgtttcg aaatgctaca gcagtttctg tttttggtaa cacaaatatt 1860

tgcggaggcg tccgagaaat gcaactaaag ccatgcattg tacaggcatc tccaaggaag 1920

agaaagcctc tgtcagttag agtagcggca tggatcttag ggtttgtcgt tggagccata 1980

ggaggaacaa tctctgggtt tgtcttctcg gtgttgttca aattgattat tcaagcaata 2040

agaggatcag aaaaaccacc aggtccttcc atattcagcc cattgatcaa gaaagctgaa 2100

gatttagctt tcttggagaa tgaagaagcg ttagcttctc tggagatcat aggaagagga 2160

ggttgtggag aagttttcaa agctgaacta ccaggaagca atgggaagat catagctgtg 2220

aagaaagtga tccaaccgcc taaagacgcc gatgaactaa cagatgaaga ttccaagttt 2280

ctgaacaaga aaatgaggca aattagatcc gagatcaaca cagtcggcca tatccggcac 2340

cggaatcttc tcccattgtt agcacacgtg tcaagacccg agtgccacta cctcgtttac 2400

gagtacatgg aaaaggggag tttgcaagat atattgactg acgtacaagc tggaaatcaa 2460

gaactaatgt ggccagcgag gcacaagatt gctttaggta tagctgcagg gcttgagtac 2520

cttcacatgg atcataaccc acgaatcatt cacagagact taaagccagc caatgttctt 2580

cttgatgatg acatggaagc cagaatttca gatttcggac tggcaaaggc aatgccagat 2640

gcagtcacac atattacaac ctcgcatgtt gcaggtactg tgggatacat agcaccagag 2700

ttttatcaaa cccacaagtt cacagataaa tgtgatatct atagttttgg agtgattctt 2760

gggattctgg tgattgggaa gcttccttca gatgagtttt ttcagcatac tgacgagatg 2820

agtctgataa agtggatgag gaacataata acatcggaaa atcctagcct tgcgattgat 2880

ccgaagttga tggatcaagg attcgatgaa cagatgcttc tggttctgaa gatcgcctgt 2940

tactgtactt tggatgatcc aaaacagagg ccgaacagta aagatgtcag gaccatgttg 3000

tcccagatca agcac 3015

<210> 20
<211> 2121
<212> DNA
<213> Artificial

<220>
<223> EFRdeltakin-tmSOBIR1

<400> 20
atgaagctgt ccttttcact tgttttcaat gctctcacgt tgcttcttca agtttgcatc 60

tttgctcaag ccaggttttc taatgagact gatatgcaag ctttgcttga gttcaagtct 120

caagtttctg aaaacaacaa gagagaggtc ttggcttcat ggaatcactc ctccccattt 180

tgtaattgga ttggagtcac atgtggccgc aggagagaaa gagttataag tttgaacctt 240

ggaggattca agttaaccgg tgtgatctca ccctccattg gtaatctctc ctttcttaga 300

ttacttaatc ttgcagacaa ctcttttgga agtaccatcc ctcaaaaggt gggaaggcta 360

tttaggcttc agtacttgaa catgagctat aatcttctcg aaggaaggat tccgtctagt 420

ctttctaact gctctagact gtcgaccgtt gatttatcgt caaaccatct tggacatggt 480

gttccttcag aactaggttc actttctaag cttgccattc tggatcttag caaaaacaac 540

cttactggaa attttcctgc atctttagga aacttgacgt cacttcagaa gcttgacttt 600

gcatataacc agatgagagg tgagattcca gacgaagtag ctagattgac tcaaatggtg 660

tttttccaaa tagcactgaa tagtttttca ggtggttttc ctcctgcatt gtacaacatc 720

tcctctcttg agtctctatc tctagctgac aatagctttt cgggtaatct tagggctgat 780

tttggttatc ttctaccaaa tctaagaaga cttcttttgg gaacaaatca gttcactgga 840

gctattccca aaacacttgc caatatctca agccttgaaa ggtttgatat ctcatctaat 900

tacctgtctg gtagtatccc tttgagcttt ggaaagttac gtaatctgtg gtggttaggg 960

attcgtaata actctcttgg aaataactcg tccagtggtc ttgaatttat tggggctgtg 1020

gcgaactgca ctcaattaga gtacttagat gttggttaca atagacttgg aggtgagctt 1080

cctgcttcta tagccaatct gtccactaca ttgactagtc tgttccttgg acaaaatctt 1140

atctctggaa ccattcctca tgacatcggg aatcttgtaa gcctgcaaga actcagctta 1200

gaaacaaata tgttgagtgg agaacttccc gtctctttcg ggaagctttt gaacttgcag 1260

gttgtggatc tgtattcaaa tgcaatatcg ggggaaatac catcttattt tggcaacatg 1320

actcggttgc agaagctcca tttgaatagc aatagtttcc acggaagaat ccctcagagt 1380

cttggacgtt gtcgatactt gctagacctg tggatggata caaataggtt gaatgggact 1440

atacctcagg aaatactgca aattccatcc ctcgcttaca tagatttgtc aaacaatttc 1500

ttgacaggcc attttccaga agaagttgga aagttagaac ttcttgttgg actaggtgct 1560

tcgtacaaca aattatcagg aaagatgcca caagctatag ggggttgtct ttcgatggaa 1620

tttctcttta tgcaaggaaa ttcgtttgac ggagccattc cagatataag ccggttggta 1680

agcctaaaga atgttgactt ctccaacaac aatctctctg gccggatacc tcgatatctg 1740

gccagtcttc cttcgctgcg aaatctgaat ctttctatga acaagtttga gggaagggtg 1800

ccaacaacag gagtgtttcg aaatgctaca gcagtttctg tttttggtaa cacaaatatt 1860

tgcggaggcg tccgagaaat gcaactaaag ccatgcattg tacaggcatc tccaaggaag 1920

agaaagcctc tgtcagttag agtagcggca tggatcttag ggtttgtcgt tggagccata 1980

ggaggaacaa tctctgggtt tgtcttctcg gtgttgttca agaggaaaaa gaaaaacaat 2040

gccagtgatg gtaacccatc tgattctact actttgggga tgttccatga gaaggtaagt 2100

tatgaagagc ttcatagtgc a 2121

<210> 21
<211> 5
<212> PRT
<213> Artificial

<220>
<223> Motif


<220>
<221> misc_feature
<222> (2)..(4)
<223> Xaa can be any naturally occurring amino acid

<400> 21

Gly Xaa Xaa Xaa Gly
1               5

<210>  22
<211>  17
<212>  PRT
<213>  Artificial

<220>
<223>  Motif

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (10)..(12)
<223>  Xaa can be any naturally occurring amino acid

<220>
<221>  misc_feature
<222>  (14)..(16)
<223>  Xaa can be any naturally occurring amino acid

<400>  22

Gly Xaa Xaa Xaa Gly Xaa Xaa Xaa Gly Xaa Xaa Xaa Gly Xaa Xaa Xaa
1               5                   10                  15

Gly